(19) Europäisches Patentamt
European Patent Office
Office européen des brevets

(11) EP 2 621 523 B1

(12) **EUROPEAN PATENT SPECIFICATION**

(45) Date of publication and mention of the grant of the patent:
**09.08.2017 Bulletin 2017/32**

(21) Application number: **11766955.6**

(22) Date of filing: **29.09.2011**

(51) Int Cl.:
***A61K 39/00*** (2006.01) ***A61K 39/385*** (2006.01)
***G01N 33/543*** (2006.01) ***G01N 33/564*** (2006.01)
***A61K 47/62*** (2017.01) ***A61K 47/69*** (2017.01)

(86) International application number:
**PCT/EP2011/066994**

(87) International publication number:
**WO 2012/041968 (05.04.2012 Gazette 2012/14)**

(54) **METHODS FOR TREATING AUTOIMMUNE DISEASE USING BIOCOMPATIBLE BIOABSORBABLE NANOSPHERES**

VERFAHREN ZUR BEHANDLUNG EINER AUTOIMMUNERKRANKUNG ANHAND VON BIOVERTRÄGLICHEN BIOABSORBIERBAREN NANOKÜGELCHEN

MÉTHODES DE TRAITEMENT D'UNE MALADIE AUTO-IMMUNE À L'AIDE DE NANOSPHÈRES BIOABSORBABLES BIOCOMPATIBLES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.09.2010 US 387873 P**

(43) Date of publication of application:
**07.08.2013 Bulletin 2013/32**

(73) Proprietor: **UTI Limited Partnership**
**Calgary, AB T2L 2K7 (CA)**

(72) Inventor: **SANTAMARIA, Pedro**
**Calgary**
**Alberta T2L 2K7 (CA)**

(74) Representative: **Gibson, Mark**
**Sagittarius IP**
**Three Globeside**
**Fieldhouse Lane**
**Marlow, Buckinghamshire SL7 1HZ (GB)**

(56) References cited:
**EP-A1- 2 614 834 WO-A2-2008/109852**

- **TSAI SUE ET AL: "Reversal of Autoimmunity by Boosting Memory-like Autoregulatory T Cells", IMMUNITY, vol. 32, no. 4, April 2010 (2010-04), pages 568-580, XP002662908, ISSN: 1074-7613**
- **MOORE ANNA ET AL: "Tracking the recruitment of diabetogenic CD8+ T-cells to the pancreas in real time", DIABETES, AMERICAN DIABETES ASSOCIATION, US, vol. 53, no. 6, 1 June 2004 (2004-06-01), pages 1459-1466, XP002499320, ISSN: 0012-1797, DOI: 10.2337/DIABETES.53.6.1459**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

SEQUENCE LISTING

<110> UTI Limited Partnership

<120> METHODS FOR TREATING AUTOIMMUNE DISEASE USING BIOCOMPATIBLE BIOABSORBABLE NANOSPHERES

<130> UTI-C-P1347PCT (378701-0360)

<140> UNKNOWN
<141> 2011-09-29

<150> 61/387873
<151> 2010-09-29

<160> 66

<170> PatentIn version 3.5

<210> 1
<211> 9
<212> PRT
<213> Artificial Sequence

<220>
<223> Description of Artificial Sequence: Synthetic peptide

<400> 1

```
His Leu Val Glu Ala Leu Tyr Leu Val
1               5
```

<210> 2
<211> 9
<212> PRT
<213> Artificial Sequence

<220>
<223> Description of Artificial Sequence: Synthetic peptide

<400> 2

```
Leu Asn Ile Asp Leu Leu Trp Ser Val
1               5
```

<210> 3
<211> 9
<212> PRT
<213> Artificial Sequence

<220>
<223> Description of Artificial Sequence: Synthetic peptide

<400> 3

Val Leu Phe Gly Leu Gly Phe Ala Ile
1               5

<210> 4
<211> 9
<212> PRT
<213> Artificial Sequence

<220>
<223> Description of Artificial Sequence: Synthetic peptide

<400> 4

Val Tyr Leu Lys Thr Asn Val Phe Leu
1               5

<210> 5
<211> 9
<212> PRT
<213> Artificial Sequence

<220>
<223> Description of Artificial Sequence: Synthetic peptide

<400> 5

Val Tyr Leu Lys Thr Asn Leu Phe Leu
1               5

<210> 6
<211> 9
<212> PRT
<213> Artificial Sequence

<220>
<223> Description of Artificial Sequence: Synthetic peptide

<400> 6

Lys Tyr Asn Lys Ala Asn Ala Phe Leu
1               5

<210> 7
<211> 9
<212> PRT
<213> Artificial Sequence

<220>
<223> Description of Artificial Sequence: Synthetic peptide

<400> 7

Lys Tyr Asn Ile Ala Asn Val Phe Leu
1 5

<210> 8
<211> 9
<212> PRT
<213> Artificial Sequence

<220>
<223> Description of Artificial Sequence: Synthetic peptide

<400> 8

Lys Tyr Asn Lys Ala Asn Val Phe Leu
1 5

<210> 9
<211> 9
<212> PRT
<213> Artificial Sequence

<220>
<223> Description of Artificial Sequence: Synthetic peptide

<400> 9

Phe Gln Asp Glu Asn Tyr Leu Tyr Leu
1 5

<210> 10
<211> 9
<212> PRT
<213> Artificial Sequence

<220>
<223> Description of Artificial Sequence: Synthetic peptide

<400> 10

Leu Tyr Leu Val Cys Gly Glu Arg Gly
1 5

<210> 11
<211> 9
<212> PRT
<213> Artificial Sequence

<220>
<223> Description of Artificial Sequence: Synthetic peptide

<400> 11

Leu Tyr Leu Val Cys Gly Glu Arg Ile
1 5

<210> 12
<211> 9
<212> PRT

<213> Artificial Sequence

<220>
<223> Description of Artificial Sequence: Synthetic peptide

<400> 12

```
Lys Tyr Gln Ala Val Thr Thr Thr Leu
1               5
```

<210> 13
<211> 9
<212> PRT
<213> Artificial Sequence

<220>
<223> Description of Artificial Sequence: Synthetic peptide

<400> 13

```
Lys Tyr Cys Leu Ile Thr Ile Phe Leu
1               5
```

<210> 14
<211> 10
<212> PRT
<213> Artificial Sequence

<220>
<223> Description of Artificial Sequence: Synthetic peptide

<400> 14

```
Val Met Asn Ile Leu Leu Gln Tyr Val Val
1               5                   10
```

<210> 15
<211> 10
<212> PRT
<213> Artificial Sequence

<220>
<223> Description of Artificial Sequence: Synthetic peptide

<400> 15

```
Arg Met Met Glu Tyr Gly Thr Thr Met Val
1               5                   10
```

<210> 16
<211> 10
<212> PRT
<213> Artificial Sequence

<220>
<223> Description of Artificial Sequence: Synthetic peptide

<400> 16

Asn Leu Ala Gln Thr Asp Leu Ala Thr Val
1 5 10

<210> 17
<211> 9
<212> PRT
<213> Artificial Sequence

<220>
<223> Description of Artificial Sequence: Synthetic peptide

<400> 17

Gln Leu Ala Arg Gln Gln Val His Val
1 5

<210> 18
<211> 9
<212> PRT
<213> Artificial Sequence

<220>
<223> Description of Artificial Sequence: Synthetic peptide

<400> 18

Ser Leu Ser Pro Leu Gln Ala Glu Leu
1 5

<210> 19
<211> 9
<212> PRT
<213> Artificial Sequence

<220>
<223> Description of Artificial Sequence: Synthetic peptide

<400> 19

Ser Leu Ala Ala Gly Val Lys Leu Leu
1 5

<210> 20
<211> 9
<212> PRT
<213> Artificial Sequence

<220>
<223> Description of Artificial Sequence: Synthetic peptide

<400> 20

Val Ile Val Met Leu Thr Pro Leu Val
1 5

<210> 21
<211> 9
<212> PRT

<213> Artificial Sequence

<220>
<223> Description of Artificial Sequence: Synthetic peptide

<400> 21

Lys Leu Gln Val Phe Leu Ile Val Leu
1 5

<210> 22
<211> 9
<212> PRT
<213> Artificial Sequence

<220>
<223> Description of Artificial Sequence: Synthetic peptide

<400> 22

Phe Leu Ile Val Leu Ser Val Ala Leu
1 5

<210> 23
<211> 9
<212> PRT
<213> Artificial Sequence

<220>
<223> Description of Artificial Sequence: Synthetic peptide

<400> 23

Phe Leu Trp Ser Val Phe Met Leu Ile
1 5

<210> 24
<211> 9
<212> PRT
<213> Artificial Sequence

<220>
<223> Description of Artificial Sequence: Synthetic peptide

<400> 24

Asn Leu Phe Leu Phe Leu Phe Ala Val
1 5

<210> 25
<211> 9
<212> PRT
<213> Artificial Sequence

<220>
<223> Description of Artificial Sequence: Synthetic peptide

<400> 25

Phe Leu Phe Ala Val Gly Phe Tyr Leu
1 5

<210> 26
<211> 9
<212> PRT
<213> Artificial Sequence

<220>
<223> Description of Artificial Sequence: Synthetic peptide

<400> 26

Tyr Leu Leu Leu Arg Val Leu Asn Ile
1 5

<210> 27
<211> 9
<212> PRT
<213> Artificial Sequence

<220>
<223> Description of Artificial Sequence: Synthetic peptide

<400> 27

Arg Leu Leu Cys Ala Leu Thr Ser Leu
1 5

<210> 28
<211> 9
<212> PRT
<213> Artificial Sequence

<220>
<223> Description of Artificial Sequence: Synthetic peptide

<400> 28

Ala Leu Trp Met Arg Leu Leu Pro Leu
1 5

<210> 29
<211> 9
<212> PRT
<213> Artificial Sequence

<220>
<223> Description of Artificial Sequence: Synthetic peptide

<400> 29

Leu Trp Met Arg Leu Leu Pro Leu Leu
1 5

<210> 30
<211> 9
<212> PRT

<213> Artificial Sequence

<220>
<223> Description of Artificial Sequence: Synthetic peptide

<400> 30

```
Arg Leu Leu Pro Leu Leu Ala Leu Leu
1               5
```

<210> 31
<211> 10
<212> PRT
<213> Artificial Sequence

<220>
<223> Description of Artificial Sequence: Synthetic peptide

<400> 31

```
His Leu Cys Gly Ser His Leu Val Glu Ala
1               5                   10
```

<210> 32
<211> 9
<212> PRT
<213> Artificial Sequence

<220>
<223> Description of Artificial Sequence: Synthetic peptide

<400> 32

```
Ala Leu Tyr Leu Val Cys Gly Glu Arg
1               5
```

<210> 33
<211> 10
<212> PRT
<213> Artificial Sequence

<220>
<223> Description of Artificial Sequence: Synthetic peptide

<400> 33

```
Leu Tyr Leu Val Cys Gly Glu Arg Gly Phe
1               5                   10
```

<210> 34
<211> 9
<212> PRT
<213> Artificial Sequence

<220>
<223> Description of Artificial Sequence: Synthetic peptide

<400> 34

Leu Val Cys Gly Glu Arg Gly Phe Phe
1 5

<210> 35
<211> 10
<212> PRT
<213> Artificial Sequence

<220>
<223> Description of Artificial Sequence: Synthetic peptide

<400> 35

Val Cys Gly Glu Arg Gly Phe Phe Tyr Thr
1 5 10

<210> 36
<211> 8
<212> PRT
<213> Artificial Sequence

<220>
<223> Description of Artificial Sequence: Synthetic peptide

<400> 36

Gly Glu Arg Gly Phe Phe Tyr Thr
1 5

<210> 37
<211> 9
<212> PRT
<213> Artificial Sequence

<220>
<223> Description of Artificial Sequence: Synthetic peptide

<400> 37

Glu Arg Gly Phe Phe Tyr Thr Pro Lys
1 5

<210> 38
<211> 9
<212> PRT
<213> Artificial Sequence

<220>
<223> Description of Artificial Sequence: Synthetic peptide

<400> 38

Phe Tyr Thr Pro Lys Thr Arg Arg Glu
1 5

<210> 39
<211> 11
<212> PRT

<213> Artificial Sequence

<220>
<223> Description of Artificial Sequence: Synthetic peptide

<400> 39

```
Thr Pro Lys Thr Arg Arg Glu Ala Glu Asp Leu
1               5                   10
```

<210> 40
<211> 9
<212> PRT
<213> Artificial Sequence

<220>
<223> Description of Artificial Sequence: Synthetic peptide

<400> 40

```
Ser Leu Gln Pro Leu Ala Leu Glu Gly
1               5
```

<210> 41
<211> 9
<212> PRT
<213> Artificial Sequence

<220>
<223> Description of Artificial Sequence: Synthetic peptide

<400> 41

```
Ala Leu Glu Gly Ser Leu Gln Lys Arg
1               5
```

<210> 42
<211> 10
<212> PRT
<213> Artificial Sequence

<220>
<223> Description of Artificial Sequence: Synthetic peptide

<400> 42

```
Ser Leu Gln Lys Arg Gly Ile Val Glu Gln
1               5                   10
```

<210> 43
<211> 10
<212> PRT
<213> Artificial Sequence

<220>
<223> Description of Artificial Sequence: Synthetic peptide

<400> 43

Gly Ile Val Glu Gln Cys Cys Thr Ser Ile
1 5 10

<210> 44
<211> 9
<212> PRT
<213> Artificial Sequence

<220>
<223> Description of Artificial Sequence: Synthetic peptide

<400> 44

Ile Val Glu Gln Cys Cys Thr Ser Ile
1 5

<210> 45
<211> 9
<212> PRT
<213> Artificial Sequence

<220>
<223> Description of Artificial Sequence: Synthetic peptide

<400> 45

Ser Leu Tyr Gln Leu Glu Asn Tyr Cys
1 5

<210> 46
<211> 9
<212> PRT
<213> Artificial Sequence

<220>
<223> Description of Artificial Sequence: Synthetic peptide

<400> 46

Ser Leu Leu Leu Glu Leu Glu Glu Val
1 5

<210> 47
<211> 9
<212> PRT
<213> Artificial Sequence

<220>
<223> Description of Artificial Sequence: Synthetic peptide

<400> 47

Leu Met Trp Ala Lys Ile Gly Pro Val
1 5

<210> 48
<211> 9
<212> PRT

<213> Artificial Sequence

<220>
<223> Description of Artificial Sequence: Synthetic peptide

<400> 48

```
Val Leu Phe Ser Ser Asp Phe Arg Ile
1               5
```

<210> 49
<211> 9
<212> PRT
<213> Artificial Sequence

<220>
<223> Description of Artificial Sequence: Synthetic peptide

<400> 49

```
Ser Leu Ser Arg Phe Ser Trp Gly Ala
1               5
```

<210> 50
<211> 9
<212> PRT
<213> Artificial Sequence

<220>
<223> Description of Artificial Sequence: Synthetic peptide

<400> 50

```
Lys Val Glu Asp Pro Phe Tyr Trp Val
1               5
```

<210> 51
<211> 10
<212> PRT
<213> Artificial Sequence

<220>
<223> Description of Artificial Sequence: Synthetic peptide

<400> 51

```
Arg Thr Phe Asp Pro His Phe Leu Arg Val
1               5                   10
```

<210> 52
<211> 9
<212> PRT
<213> Artificial Sequence

<220>
<223> Description of Artificial Sequence: Synthetic peptide

<400> 52

Phe Leu Arg Val Pro Cys Trp Lys Ile
1 5

<210> 53
<211> 10
<212> PRT
<213> Artificial Sequence

<220>
<223> Description of Artificial Sequence: Synthetic peptide

<400> 53

Lys Ile Thr Leu Phe Val Ile Val Pro Val
1 5 10

<210> 54
<211> 9
<212> PRT
<213> Artificial Sequence

<220>
<223> Description of Artificial Sequence: Synthetic peptide

<400> 54

Val Leu Gly Pro Leu Val Ala Leu Ile
1 5

<210> 55
<211> 9
<212> PRT
<213> Artificial Sequence

<220>
<223> Description of Artificial Sequence: Synthetic peptide

<400> 55

Thr Leu Phe Val Ile Val Pro Val Leu
1 5

<210> 56
<211> 10
<212> PRT
<213> Artificial Sequence

<220>
<223> Description of Artificial Sequence: Synthetic peptide

<400> 56

Arg Leu Ala Gly Gln Phe Leu Glu Glu Leu
1 5 10

<210> 57
<211> 9
<212> PRT

<213> Artificial Sequence

<220>
<223> Description of Artificial Sequence: Synthetic peptide

<400> 57

Phe Leu Tyr Gly Ala Leu Leu Leu Ala
1 5

<210> 58
<211> 9
<212> PRT
<213> Artificial Sequence

<220>
<223> Description of Artificial Sequence: Synthetic peptide

<400> 58

Val Tyr Leu Ala Thr Asn Val Ala Leu
1 5

<210> 59
<211> 11
<212> PRT
<213> Artificial Sequence

<220>
<223> Description of Artificial Sequence: Synthetic peptide

<400> 59

Ala His His Pro Ile Trp Ala Arg Met Asp Ala
1 5

<210> 60
<211> 18
<212> PRT
<213> Artificial Sequence

<220>
<223> Description of Artificial Sequence: Synthetic peptide

<400> 60

Thr Ala Ala Leu Ser Tyr Thr Ile Ser Arg Met Glu Glu Ser Ser Val Thr Leu
1 5 10 15

<210> 61
<211> 19
<212> PRT
<213> Artificial Sequence

<220>
<223> Description of Artificial Sequence: Synthetic peptide

<400> 61

Leu His Arg Ser Gly Val Leu Ile Ile His His Leu Gln Glu Asp Tyr Arg Thr Tyr
1 5 10 15

<210> 62
<211> 20
<212> PRT
<213> Artificial Sequence

<220>
<223> Description of Artificial Sequence: Synthetic peptide

<400> 62

His Thr Pro Gly Val His Met Ala Ser Leu Ser Val Tyr Leu Lys Thr Asn Val Phe Leu
1 5 10 15 20

<210> 63
<211> 15
<212> PRT
<213> Artificial Sequence

<220>
<223> Description of Artificial Sequence: Synthetic peptide

<400> 63

Ser His Leu Val Glu Ala Leu Tyr Leu Val Ala Gly Glu Arg Gly
1 5 10 15

<210> 64
<211> 11
<212> PRT
<213> Artificial Sequence

<220>
<223> Description of Artificial Sequence: Synthetic peptide

<400> 64

Leu Ser Ile Ala Leu His Val Gly Phe Asp His
1 5 10

<210> 65
<211> 9
<212> PRT
<213> Artificial Sequence

<220>
<223> Description of Artificial Sequence: Synthetic peptide

<400> 65

Arg His Gly Leu Asp Asn Tyr Arg Gly
1 5

<210> 66

<211> 15
<212> PRT
<213> Artificial Sequence

<220>
<223> Description of Artificial Sequence: Synthetic peptide

<400> 65

```
Ala Met Lys Arg His Gly Leu Asp Asn Tyr Arg Gly Tyr Ser Leu
1               5                   10                  15
```

## Description

### Field of the Invention

This invention is directed to compositions and methods related to immunology and medicine. In particular, this invention is related to compositions for use in the treatment of autoimmune conditions, particularly diabetes.

### State of the Art

Antigen vaccination can be used for the induction of T-cell tolerance in autoimmunity. Administration of autoantigenic proteins or peptides in solution can blunt the initiation and/or progression of autoimmunity in experimental models of autoimmune disease (Wraith *et al.,* 1989; Metzler and Wraith, 1993; Liu and Wraith, 1995; Anderton and Wraith, 1998; Karin *et al.,* 1994). However, limited clinical trials in humans employing similar strategies have almost invariably met with failure (Weiner, 1993; Trentham *et al.,* 1993; McKown *et al.,* 1999; Pozzilli *et al.,* 2000; Group, D.P.T.-T.D.S. 2002; Kappos *et al.,* 2000; Bielekova *et al.,* 2000). This suggests that the principles guiding the choice and conditions of treatment are poorly defined and, as a result, inadequate for human application.

Spontaneous organ-specific autoimmune disorders result from complex responses against numerous epitopes in multiple antigens that arise spontaneously in a stochastic and often unpredictable sequence. This complexity is compounded by the fact that lymphocyte clones recognizing identical epitopes engage antigen/major histocompatibility complex (MHC) molecules within a broad range of avidities, the strength of which correlates with pathogenic potential (Amrani *et al.,* 2000; Santamaria, 2001; Liblau *et al.,* 2002). Consequently, the outcome of any immunization strategy for the prevention of autoimmunity is likely to be influenced by the choice of autoantigen(s), dose, periodicity of treatment, and route and form of administration.

Type 1 Diabetes (T1D) in mice is associated with autoreactive $CD8^+$ T-cells. Non- obese diabetic (NOD) mice develop a form of T1D, closely resembling human T1D, that results from selective destruction of pancreatic β cells by T-cells recognizing a growing list of autoantigens (Lieberman and DiLorenzo, 2003). Although initiation of T1D clearly requires the contribution of $CD4^+$ cells, there is compelling evidence that T1D is $CD8^+$ T-cell-dependent (Santamaria, 2001; Liblau *et al.,* 2002). It has been discovered that a significant fraction of islet-associated $CD8^+$ cells in NOD mice use CDR3-invariant $V\alpha17$-$J\alpha42^+$ TCRs, referred to as '8.3-TCR-like' (Santamaria *et al.,* 1995; Verdaguer *et al.,* 1996; Verdaguer *et al.,* 1997; DiLorenzo *et al.,* 1998). These cells, which recognize the mimotope NRP-A7 (defined using combinatorial peptide libraries) in the context of the MHC molecule $K^d$ (Anderson *et al.,* 1999), are already a significant component of the earliest NOD islet $CD8^+$ infiltrates (DiLorenzo *et al.,* 1998; Anderson *et al.,* 1999; Amrani *et al.,* 2001), are diabetogenic (Verdaguer *et al.,* 1996; Verdaguer *et al.,* 1997), and target a peptide from islet-specific glucose-6-phosphatase catalytic subunit-related protein (IGRP) (Lieberman *et al.,* 2003), a protein of unknown function (Arden *et al.,* 1999; Martin *et al.,* 2001). The $CD8^+$ cells that recognize this peptide ($IGRP_{206-214}$, similar to NRP-A7) are unusually frequent in the circulation (> 1/200 $CD8^+$ cells) (Lieberman *et al.,* 2003; Trudeau *et al.,* 2003). Notably, progression of insulitis to diabetes in NOD mice is invariably accompanied by cyclic expansion of the circulating $IGRP_{206-214}$-reactive $CD8^+$ pool (Trudeau *et al.,* 2003), and by avidity maturation of its islet-associated counterpart (Amrani *et al.,* 2000). More recently, it has been shown that islet-associated $CD8^+$ cells in NOD mice recognize multiple IGRP epitopes, indicating that IGRP is a dominant autoantigen for $CD8^+$ cells, at least in murine T1D (Han *et al.,* 2005). NOD islet-associated $CD8^+$ cells, particularly those found early on in the disease process also recognize an insulin epitope (Ins $B_{15-23}$ (Wong *et al.,* 1999)).

Association studies have suggested that certain HLA class I alleles (i.e., HLA-A*0201) afford susceptibility to human T1D (Fennessy *et al.,* 1994; Honeyman *et al.,* 1995; Tait *et al.,* 1995; Nejentsev *et al.,* 1997; Nakanishi *et al.,* 1999; Robles *et al.,* 2002). Pathology studies have shown that the insulitis lesions of newly diagnosed patients consist mostly of (HLA class I-restricted) $CD8^+$ T-cells (Bottazzo *et al.,* 1985; Atkinson and Maclaren, 1990; Castano and Eisenbarth, 1990; Hanninen *et al.,* 1992; Itoh *et al.,* 1993; Somoza *et al.,* 1994; Atkinson and Maclaren, 1994; Moriwaki *et al.,* 1999; Imagawa *et al.,* 2001), which are also the predominant cell population in patients treated by transplantation with pancreas isografts (from identical twins) or allografts (from related donors) (Sibley *et al.,* 1985; Santamaria *et al.,* 1992).

Insulin is a key target of the antibody and $CD4^+$ response in both human and murine T1D (Wong *et al.,* 1999; Palmer *et al.,* 1983; Chentoufi and Polychronakos, 2002; Toma *et al.,* 2005; Nakayama *et al.,* 2005; Kent *et al.,* 2005). The human insulin B chain epitope $hInsB_{10-18}$ is presented by HLA-A*0201 to autoreactive $CD8^+$ cells both in islet transplant recipients (Pinkse *et al.,* 2005) and in the course of spontaneous disease (Toma *et al.,* 2005). In addition, four additional peptides have been identified from mouse pre-proinsulin 1 or 2 that are recognized by islet-associated $CD8^+$ T-cells from HLA-A*0201-transgenic mice in the context of HLA-A*0201.

IGRP, which is encoded by a gene (located on chromosome 2q28-32 (Martin *et al.,* 2001)) that overlaps a T1D susceptibility locus, IDDM7 (2q31) (Pociot and McDermott, 2002; Owerbach, 2000), has also been recently identified as a beta-cell autoantigen of potential relevance in human T1D (Takaki *et al.,* 2006). Two HLA-A*0201-binding epitopes of human IGRP ($IGRP_{228-236}$ and $hIGRP_{265-273}$) are recognized by islet-associated $CD8^+$ cells from murine MHC class I-deficient NOD mice expressing an HLA-A*0201 transgene (Takaki *et al.,* 2006). Notably, the islet-associated $CD8^+$ T-cells of these 'humanized' HLA-A*0201-transgenic mice were cytotoxic to HLA-A*0201-positive human islets (Takaki *et al.,* 2006).

T1D in NOD mice can be prevented by expansion of low avidity autoreactive $CD8^+$ T-cells. Administration of soluble peptides (without adjuvant) is an effective way of inducing antigen-specific T-cell tolerance (Aichele *et al.,* 1994; Toes *et al.,* 1996). Previously, it was shown that treatment of pre-diabetic NOD mice with soluble NRP-A7 blunted avidity maturation of the $IGRP_{206-214}$-reactive $CD8^+$ subset by selectively deleting clonotypes expressing TCRs with the highest affinity for peptide/MHC (Amrani *et al.,* 2000). These observations raised the possibility that NRP-A7's anti-T1D activity was mediated also by fostering occupation of the 'high avidity clonotype niche' (emptied by NRP-A7 treatment) by 'low avidity' (and potentially anti-diabetogenic) clones. To test this hypothesis, altered peptide ligands (APLs) were identified with partial, full or super agonistic activity for $IGRP_{206-214}$-reactive $CD8^+$ T-cells and compared their anti-T1D activity over a wide dose-range.

Chronic treatment with moderate doses of an intermediate affinity APL (NRP-A7) or high doses of a low affinity APL (NRP-I4) afforded T1D protection. This was associated with local accumulation of low avidity $IGRP_{206-214}$-reactive $CD8^+$ cells at the expense of their high avidity counterparts, which were deleted. Unexpectedly, chronic treatment with high doses of a high affinity APL (NRP-V7) or the natural ligand ($IGRP_{206-214}$) only afforded marginal protection. Strikingly, the islets of these mice contained almost no $IGRP_{206-214}$-reactive $CD8^+$ cells, but increased populations of $CD8^+$ cells recognizing other IGRP epitopes. This led us to conclude that peptide therapy in autoimmunity may be most effective when it fosters occupation of the target organ lymphocyte niche by non-pathogenic, low avidity clones (Han *et al.,* 2005), a prediction supported by mathematical modeling (Maree *et al.,* 2006). Unfortunately, this outcome occurred only within a narrow range of APL dose and avidity (for target TCRs), suggesting that peptide therapy is ill-suited to prevent or cure T1D.

Tsai et al., Immunity (2010) 32, 568-680 describes that the progression to autoimmune diabetes results in the conversion of naive low-avidity autoreactive $CD8^+$ T cells into memory-like autoregulatory cells that can be expanded *in vivo* with nanoparticles coated with disease-relevant peptide-majorhistocompatibility complexes (pMHC-NP). Moore et al., Diabetes (2004) 53, 1459-1466 describes an antigen-specific magnetic label to selectively target a prevalent population of diabetogenic $CD8^+$ T cells that contributes to the progression of insulitis to overt diabetes in NOD mice.

Thus, there remains a need for additional compositions and related methods for the treatment of diabetes, as well as other autoimmune disorders.

## SUMMARY OF THE INVENTION

It would be difficult to treat a patient with peptides because, as is the case of IGRP, this would require several milligrams of peptides per dose. Delivery of antigen/MHC complexes, e.g., peptide/MHC/nanosphere complexes (without costimulatory molecules), on biocompatible, bioabsorbable nanospheres are provided herein. It is contemplated that these complexes will be more tolerogenic than peptides alone or non-bioabsorbable nanosphere complexes.

Aspects and embodiments of this application include the discovery of a new paradigm in the treatment of autoimmunity. Traditionally, vaccines have been used to expand T-cells capable of affording protection against pathogens or cancer, or to delete T-cells capable of causing autoimmunity. Aspects of the present invention relate to a novel type of 'vaccine' that selectively induces the expansion of autoreactive $CD8^+$ cells with anti-autoimmune properties and, at the same time, the deletion of autoreactive $CD8^+$ cells with pathogenic (autoimmune) properties, both according to the antigenic specificity of the T cells. The anti-autoimmune autoreactive $CD8^+$ T-cells (anti-pathogenic $CD8^+$ cells) suppress autoreactive T-cell responses in a tissue-specific (upon spontaneous recruitment to the target tissue) but antigen-non-specific manner (e.g., locally suppressing other autoreactive T-cell responses). As a result, treatment with this type of vaccine can both prevent and/or ameliorate T1D, as well as restore normoglycemia or reduce glucose levels in hyperglycemic NOD mice without causing generalized immunosuppression. This strategy can be applicable to the treatment of other T-cell mediated autoimmune diseases and may be able to prevent T1D recurrence upon islet transplantation. According to one aspect of the invention, there is provided a composition of high particle density nanoparticles comprising antigen-MHC complexes coupled at a ratio of from 0.1:1 to 500:1 to a biocompatible bioabsorbable nanosphere core having a diameter of 4 nm or from 5 to 15 nm for use in a method for preventing or treating an autoimmune disorder when administered to a subject. According to a further aspect of the invention, there is provided a composition of high particle density nanoparticles comprising antigen-MHC complexes coupled at a ratio of from 0.1:1 to 500:1 to a biocompatible bioabsorbable nanosphere core having a diameter of 4 nm or from 5 to 15 nm for use in a method for expanding and/or developing populations of anti-pathogenic autoreactive T-cells in a subject.

Certain embodiments of the present invention relate to compositions for use in methods of selectively reducing or expanding T cells according to the antigenic specificity of the T cells. Therefore, it is believed that the present invention can be used to reduce or eliminate T cells that recognize autoantigens, such as P cell specific T cells. As such, the present invention can be used to prevent, treat, or ameliorate autoimmune diseases such as IDDM. Furthermore, the present invention can be used to expand desirable T cells, such as T cells that recognize tumor antigens, to prevent, treat and/or ameliorate diseases battled by these T cells.

Embodiments of the invention are directed to compositions for use in methods of preventing, or treating an autoimmune disorder comprising administering an antigen/MHC/biocompatible, bioabsorbable nanosphere complex to a subject in an amount sufficient to expand anti-pathogenic autoreactive T cells. An antigen includes, but is not limited to all or part of a peptide, nucleic acid, carbohydrate, lipid or other molecule or compound that can modulate the activity of T cells or T cell populations, when in the context of a MHC or MHC like molecule coupled to a substrate. The bioabsorbability of the nanosphere complex is critical to the invention as it prevents long term accumulation of the nanospheres *in vivo* with any accompanying toxicity arising therefrom.

Embodiments of the invention include tolerogenic, nanospheres comprising a biocompatible, bioabsorbable nanosphere coupled to an antigen-MHC complex. The antigen-MHC complex may be coupled directly to such a nanosphere or via a linker. A preferred nanosphere can further comprise a biocompatible, bioabsorbable metal core and a biodegradable, bioabsorbable coating. The nanosphere may further comprise a covering or shell of other molecules that can be easily coupled to the antigen-MHC complex (e.g, streptavidin or avidin or other known molecules used to attach moieties to nanospheres). In certain aspects, a biocompatible, bioabsorbable nanosphere comprises a material selected from the group consisting of, for example, iron III oxide, tricalcium phosphate,chromium, gallium, as well as biocompatible, bioabsorbable polymers such as PGLA, PLLA, PGA, PDLLA, PCL, PDLGA, PLDLA, PLC (all of which are available from Zeus, 3737 Industrial Blvd, Orangeburg, SC, 29118 USA under the tradename Absorv™), hyaluronic acid, alginate, polyhydroxyalkanoates, and the like. In further aspects, a biocompatible bioabsorbable nanosphere is a metal or magnetizable or superparamagnetic nanosphere. The biocompatible, bioabsorbable nanosphere may further comprise a biodegradable coating formed from dextran; poly(ethylene glycol); poly(ethylene oxide); mannitol; poly(hydroxalkanoate)s of the PHB-PHV class; and other modified poly(saccharides) such as starch, cellulose and chitosan. Therefore, according to one aspect of the invention, there is provided an auto-antigen epitope-MHC- biocompatible bioabsorbable nanosphere complex comprising a biocompatible core and a biodegradable, bioabsorbable coating on the outer surface of said core and high density antigen-MHC complexes coupled at a ratio of from 0.1:1 to 500:1 to a biocompatible bioabsorbable nanosphere having a diameter of 4 nm or from 5 to 15 nm.

Certain aspects of the invention include methods and compositions concerning antigenic compositions including segments, fragments, or epitopes of polypeptides, peptides, nucleic acids, carbohydrates, lipids and other molecules that provoke or induce an antigenic or immune response, generally referred to as antigens. In particular aspects, the antigen is derived from, is a mimic of, or is an autoreactive antigen and/or complexes thereof

Peptide antigens include, but are not limited to $hInsB_{10-18}$ (HLVEALYLV (SEQ ID NO:1)), $hIGRP_{228-236}$ (LNIDLLWSV (SEQ ID NO:2)), $hIGRP_{265-273}$ (VLFGLGFAI (SEQ ID NO:3)), $IGRP_{206-214}$ (VYLKTNVFL (SEQ ID NO:4)), NRP-A7 (KYNKANAFL (SEQ ID NO:6)), NRP-14 (KYNIANVFL (SEQ ID NO:7)), NRP-V7 (KYNKANVFL (SEQ ID NO:8)), $YAI/D^b$ (FQDENYLYL (SEQ ID NO:9)) and/or INS $B_{15-23}$ (LYLVCGERG (SEQ ID NO:10)), as well as peptides and proteins disclosed in U.S. Publication 20050202032.

In certain aspects, a peptide antigen for treatment of T1D is $GAD65_{114-123}$, VMNILLQYVV (SEQ ID NO:14); $GAD65_{536-545}$, RMMEYGTTMV (SEQ ID NO:15); $GFAP_{143-151}$, NLAQTDLATV (SEQ ID NO:16); $GFAP_{214-222}$, QLARQQVHV (SEQ ID NO:17); IA-$2_{172-180}$, SLSPLQAEL (SEQ ID NO:18); IA-$2_{482-490}$, SLAAGVKLL (SEQ ID NO:19); IA-$2_{805-813}$, VIVMLTPLV (SEQ ID NO:20); $ppIAPP_{5-13}$, KLQVFLIVL (SEQ ID NO:21); $ppIAPP_{9-17}$, FLIVLSVAL (SEQ ID NO:22); $IGRP_{152-160}$, FLWSVFMLI (SEQ ID NO:23); $IGRP_{211-219}$, NLFLFLFAV (SEQ ID NO:24); $IGRP_{215-223}$, FLFAVGFYL (SEQ ID NO:25); $IGRP_{222-230}$, YLLLRVLNI (SEQ ID NO:26); $IGRP_{228-236}$, LNIDLLWSV (SEQ ID NO:2); $IGRP_{265-273}$, VLFGLGFAI (SEQ ID NO:3); $IGRP_{293-301}$, RLLCALTSL (SEQ ID NO:27); Pro-$insulin_{L2-10}$, ALWMRLLPL (SEQ ID NO:28); Pro-$insulin_{L3-11}$, LWMRLLPLL (SEQ ID NO:29); $Proinsulin_{L6-14}$, RLLPLLALL (SEQ ID NO:30); Pro-$insulin_{B5-14}$, HLCGSHLVEA (SEQ ID NO:31); Pro-$insulin_{B10-18}$, HLVEALYLV (SEQ ID NO:1); Pro-$insulin_{B14-22}$, ALYLVCGER (SEQ ID NO:32); Pro-$insulin_{B15-24}$, LYLVCGERGF (SEQ ID NO:33); Pro-$insulin_{B17-25}$, LVCGERGFF (SEQ ID NO:34); Pro-$insulin_{B18-27}$, VCGERGFFYT (SEQ ID NO:35); Pro-$insulin_{B20-27}$, GERGFFYT (SEQ ID NO:36); Pro-$insulin_{B21-29}$, ERGFFYTPK (SEQ ID NO:37); Pro-$insulin_{B25-C1}$, FYTPKTRRE (SEQ ID NO:38); Pro-$insulin_{B27-C5}$, TPKTRREAEDL (SEQ ID NO:39); $Proinsulin_{C20-28}$, SLQPLALEG (SEQ ID NO:40); Pro-$insulin_{C25-33}$, ALEGSLQKR (SEQ ID NO:41); Pro-$insulin_{C29-A5}$, SLQKRGIVEQ (SEQ ID NO:42); Pro-$insulin_{A1-10}$, GIVEQCCTSI (SEQ ID NO:43); Pro-$insulin_{A2-10}$, IVEQCCTSI (SEQ ID NO:44); Pro-$insulin_{A12-20}$, SLYQLENYC (SEQ ID NO:45) or combinations thereof.

In still further aspects peptide antigens associated with multiple sclerosis (MS) can be used and include: $MAG_{287-295}$, SLLLELEEV (SEQ ID NO:46); $MAG_{509-517}$, LMWAKIGPV (SEQ ID NO:47); $MAG_{556-564}$, VLFSSDFRI (SEQ ID NO:48); $MBPhl_{110-118}$, SLSRFSWGA (SEQ ID NO:49); $MOG_{114-122}$, KVEDPFYWV (SEQ ID NO:50); $MOG_{166-175}$, RTFDPHFLRV (SEQ ID NO:51); $MOG_{172-180}$, FLRVPCWKI (SEQ ID NO:52); $MOG_{179-188}$, KITLFVIVPV (SEQ ID NO:53); $MOG_{188-196}$, VLGPLVALI (SEQ ID NO:54); $MOG_{181-189}$, TLFVIVPVL (SEQ ID NO:55); $MOG_{205-214}$, RLAGQFLEEL (SEQ ID NO:56); $PLP_{80-88}$, FLYGALLLA (SEQ ID NO:57) or combinations thereof.

In certain aspects the antigen-MHC complex can be crosslinked (conjugated) to the nanospheres described herein. One non-limiting method of conjugating a nanosphere to an antigen-MHC complex includes (a) reacting an antigen-MHC complex with a conjugating agent, thereby forming an antigen-MHC- complex; and (b) reacting a biocompatible biodegradable nanosphere to the complex of step (a). In one embodiment, the method comprises concentrating the complex of step (a) before performing step (b). In another embodiment, the conjugating agent comprises a heterobifunctional agent. In yet another embodiment, the conjugating agent comprises DOTA-maleimide (4-maleimidobutyramidobenzyl-DOTA), SMPT (4-succinimidyloxycarbonyl-α-methyl-α-(2-pyridylditio)toluene-), sulfo-LC-SMPT (sulfosuccinimidyl-6-(α-methyl-α-(2-pyridylthio)toluamido) hexanoate, Traut's reagent (2-Iminothiolane-HCl), or any combination thereof. See U.S. Patent Publication 20070059775; U.S. Pat. Nos. 4,671,958, 4,659,839, 4,414,148, 4,699,784; 4,680,338; 4,569,789; 4,589,071; 7,186,814 and 5543391 European Patent Application No. 188,256 for a discussion of conjugating complexes to microparticles or nanoparticles.

An autoimmune disorder may include, but is not limited to, diabetes melitus, transplantation rejection, multiple sclerosis, premature ovarian failure, scleroderma, Sjogren's disease, lupus, vilelego, alopecia (baldness), polyglandular failure, Graves' disease, hypothyroidism, polymyositis, pemphigus, Crohn's disease, colititis, autoimmune hepatitis, hypopituitarism, myocarditis, Addison's disease, autoimmune skin diseases, uveititis, pernicious anemia, hypoparathyroidism, and/or rheumatoid arthritis. In certain aspects, a peptide component of an antigen/MHC/nanosphere complex is derived or designed from an autoantigen or an autoantigen epitope, or a mimic thereof, involved in the autoimmune response to be probed, modulated, or blunted by the treatment. In particular aspects, the autoantigen is a peptide, carbohydrate, or lipid. In certain aspects, an autoantigen is a fragment, epitope, or peptide of a protein, carbohydrate, or lipid expressed by certain cells of a subject, such as pancreatic beta cells, and includes, but is not limited to a fragment of IGRP, Insulin, GAD or IA-2 protein. Various such proteins or epitopes have been identified for a variety of autoimmune conditions. The autoantigen may be a peptide, carbohydrate, lipid or the like derived from a second endocrine or neurocrine component, such as peri-islet Schwann cell or the like.

In still further aspects of this invention, the MHC component of the antigen/MHC/nanosphere complex is a classical or non-classical MHC class I or MHC class II polypeptide component. The MHC class I component can comprise all or part of a HLA-A, HLA-B, HLA-C, HLA-E, HLA-F, HLA-G molecule, particularly all or part of a HLA-A molecule, such as a HLA-A*0201 MHC class I molecule. The non-classical MHC class I component can comprise CD1-like molecules. An MHC class II component may comprise all or part of a HLA-DR, HLA-DQ, or HLA-DP. In certain aspects, the antigen/MHC complex is covalently or non-covalently coupled or attached to a substrate (antigen/MHC/nanosphere complex). The substrate is typically a biocompatible absorbable nanosphere. In one embodiment, the nanosphere comprises a metal, such as iron or iron oxide. In another embodiment, the nanosphere comprises a biocompatible, bioabsorbable polymer. In either case, the nanosphere undergoes bioabsorption *in vivo* such that accumulation of the nanoparticles *in vivo* is limited. Peptides of the invention can be chemically coupled to a substrate and in particular coupled via a chemical or a peptide linker. CD1 molecules are an example of a non-classical MHC molecule. Non-classical MHC molecules are characterized as non-polymorphic, conserved among species and possessing narrow, deep, hydrophobic ligand binding pockets. These binding pockets are capable of presenting glycolipids and phospholipids to Natural Killer T (NKT) cells. NKT cells represent a unique lymphocyte population that co-express NK cell markers and a semi-invariant T cell receptor (TCR). They are implicated in the regulation of immune responses associated with a broad range of diseases.

In certain embodiments, the T cells expanded by the treatment have been pre-activated by the disease process and have a memory phenotype. In one aspect, T cells arise from autoreactive precursors recognizing the target epitope with low avidity. Avidity can be determined by a tetramer binding assay or the like. In a further aspect, the antigen/MHC/nanosphere complex is administered prior, after or both prior to and after the onset of clinical symptoms of the autoimmune disease of interest. In still a further aspect, the method may include a step that comprises assessing a biological parameter of an autoimmune condition, such as the subject's blood sugar levels before and/or after treatment. The methods of the invention may also include assessing a subject's autoimmune status, including the assessment of any autoreactive immune responses. In certain aspects, a T cell is a $CD4^+$ or $CD8^+$ T cell or a NK T (NKT) cell.

Further embodiments of the invention include compositions for use in methods of expanding anti-pathogenic autoreactive T cells comprising administering an antigen/MHC/nanosphere complex in an amount sufficient to stimulate expansion of a anti-pathogenic autoreactive T cell. In certain aspects the T cell is a $CD8^+$ or a $CD4^+$ T cell or a NKT cell.

Further disclosed are methods for protecting cells of a subject, such as a pancreatic islet cells, from an autoimmune response, particularly a pathogenic autoimmune response, comprising administering to a subject an antigen/MHC/nanosphere complex in an amount sufficient to inhibit the destruction of the cells or tissues comprising the cells, wherein the antigen or antigenic molecule from which it is derived is from an autoantigen associated with cells.

Further disclosed are methods for diagnosing autoimmunity comprising assessing treatment-induced expansion of anti-pathogenic $CD8^+$ or $CD4^+$ T cell responses as an indication of active autoimmunity.

Embodiments of the invention may include compositions for use in methods for preventing, ameliorating, or treating rejection of transplanted tissues by allogeneic or autoimmune responses by administering an antigen/MHC complex operatively coupled to a substrate (i.e., an antigen/MHC/nanosphere complex) to a subject in an amount sufficient to expand anti-pathogenic autoreactive T cells, or inducing expansion of anti-pathogenic cells recognizing alloantigens or autoantigens expressed by transplanted tissues or organs.

Further disclosed are methods of increasing or maintaining the number of functional cells, e.g., islet cells, of a predetermined type in a mammal by preventing or inhibiting cell death or killing. This method is used to treat an autoimmune disease where endogenous cell and/or tissue regeneration is desired. Such autoimmune diseases include, without limitation, diabetes melitus, multiple sclerosis, premature ovarian failure, scleroderm, Sjogren's disease, lupus, vitelego, alopecia (baldness), polyglandular failure, Graves' disease, hypothyroidism, polymyositis, pemphigus, Crohn's disease, colitits, autoimmune hepatitis, hypopituitarism, myocarditis, Addison's disease, autoimmune skin diseases, uveititis, pernicious anemia, hypoparathyroidism, rheumatoid arthritis and the like. One aspect of the invention provides a novel two-part therapeutic approach to ablate existing autoimmunity while re-educating the immune system.

In certain aspects, the antigen/MHC/nanosphere complex need not be administered with an adjuvant in order to induce an immune response, e.g., an antibody response. In particular embodiments, the antigen/MHC/nanosphere composition can be used in conjunction with well known polyclonal and monoclonal antibody techniques to produce an antibody using reduced or no adjuvant(s).

## DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.

**FIGS. 1A-1C. The low affinity autoreactive 17.6α/8.3β $CD8^+$ T cells are anti-diabetogenic**. FIG. 1A, Frequency of diabetes in 17.6α/8.3β-NOD (n=95) versus 17.4α/8.3β-NOD mice (n=598). FIG. 1B, Insulitis score in Tg mice (n=6 for 17.6α/8.3β-NOD, n=3 for 17.4α/8.3β-NOD). FIG. 1C, Frequency of diabetes in NOD (n=56) versus LCMV-NOD (n=10).

**FIGS. 2A-2B. Developmental biology of the 17.6α/8.3β TCR.** FIG. 2A, Developmental biology of 17.6α/8.3β versus 17.4α/8.3β TCR in Tg mice. Upper panels are representative CD4 versus CD8 dot plots of splenocytes. Lower panel is the comparison of $CD8^+$ T cell staining with NRP-V7/$K^d$ tetramer. FIG. 2B, Developmental biology of the 17.6α/8.30β versus 17.4α/8.3β TCRs in RAG-2-/- Tg mice. Upper panels are representative CD4 versus CD8 dot plots of splenocytes. Lower panel is the comparison of $CD8^+$ T cell staining with NRP-V7/$K^d$ tetramer.

**FIG**. **3**. **Frequency of diabetes in 17.6α/8.3β-NOD.RAG-2-/- (n=13) versus 17.4α/8.3β-NOD.RAG-2-/- mice (n=106).**

**FIGS**. **4A**-**4B**. **Developmental biology of 17.6α/8.3β versus 17.4α/8.3β TCR in TCRα-/- Tg mice**. FIG. 4A, Upper panels are representative CD4 versus CD8 dot plots of splenocytes. Lower panel is the comparison of $CD8^+$ T cell staining with NRP-V7/$K^d$ tetramer. FIG. 4B, Frequency of diabetes in 17.6α/8.3β-NOD.TCRa-/- (n=14) versus 17.4α/8.3β-NOD.TCRα-/- mice (n=28). Values in the dot plot FACS panels correspond to the percentages of the cells within each quadrant and values in the histogram panels correspond the percentages of the cells that stained positive (mean±SE).

**FIGS. 5A-5J. 17.6α/8.3β $CD8^+$ T cells spontaneously differentiate into memory T cells with regulatory function**. FIG. 5A, Representative FACS profiles of splenic $CD8^+$ T cells from 17.6α/8.3β-NOD.TCRa-/-versus 17.4α/8.3β-NOD.TCRα-/-mice. FIG. 5B, Percentage of $CD44^{hi}$ $CD122^+$ $CD8^+$ T cells within spleen (n=12 for 17.6α/8.3β-NOD.TCRα-/- and n=9 for 17.4α/8.30β-NOD.TCRα-/-), PLN (n=9 for 17.6α/8.3β-NOD.TCRα-/- and n=6 for 17.4α/8.3β-NOD.TCRα-/-) and BM (n=4 for 17.6α/8.3β-NOD.TCRα-/- and n=3 for 17.4α/8.3β-NOD.TCRα-/-) of TCRα-/- Tg mice (mean±SE). Mice were 9-18 weeks old. FIG. 5C, Representative FACS profile of splenic $CD8^+$ T cells from 17.6α/8.30β-NOD.TCRα-/- mice stained with NRP-V7/$K^d$ tetramer versus anti-CD 122 Ab. Values are mean±SE of five different experiments. FIG. 5D, Phenotypic analysis of naive versus memory splenic $CD8^+$ T cells from 17.6α/8.3β-NOD.TCRα-/- mice. Data are representative of at least two experiments for each marker. FIG. 5E, Comparison of CD122 staining in $CD8^+CD4^-$ thymocytes versus $CD8^+$ splenocytes from $TCR\alpha^{-/-}$Tg mice. Data are representative of four experiments. FIG. 5F, BrdU uptake by splenic $CD8^+$ T cell from $TCR\alpha^{-/-}$ Tg mice. FIG. 5G, Upper panel: representative FACS profile of the proliferation of splenic $CD8^+$ T cell from Tg mice in response to cytokines IL-2 and IL-15 (both at 100 ng/ml). Lower panel: Fold expansion of naive versus memory $CD8^+$ T cells from 17.6α/8.30β-NOD.$TCR\alpha^{-/-}$ mice in response to different concentration of IL-2 and IL-15. Data are representative of at least three experiments. FIG. 5H, Production of IFN-γ by splenic naive $CD8^+$ T cells from 17.4α/8.3β-

NOD.TCR$\alpha^{-/-}$ mice versus naive and memory CD8$^+$ T cells from 17.6$\alpha$/8.3$\beta$-NOD.TCR$\alpha^{-/-}$ mice in response to DCs pulsed with 1 $\mu$g/ml NRP-A7 after 24 and 48 hours. FIG. 5I, Intra-cellular IFN-$\gamma$ staining from splenic naive CD8$^+$ T cells from 17.4$\alpha$/8.3$\beta$-NOD.TCR$\alpha^{-/-}$ mice versus naive and memory CD8$^+$ T cells from 17.6$\alpha$/8.3$\beta$-NOD.TCR$\alpha^{-/-}$ mice in response to DCs pulsed with 1 $\mu$g/ml NRP-A7 after 6 hours. FIG. 5J, Production of IL-2 and proliferation in response to DCs pulsed with 1 $\mu$g/ml NRP-A7 at different time-points. Data in FIG. 5H and FIG. 5J are representative of four experiments and data in FIG. 5I are representative of three experiments.

**FIG. 6**. **Proliferation of CFSE-labeled 17.4$\alpha$/8.3$\beta$ CD8$^+$ T cells.** Proliferation of CFSE-labeled 17.4$\alpha$/8.3$\beta$ CD8$^+$ T cells in response to NRP-A7 pulsed DCs in the presence of naive versus memory CD8$^+$ T cells from 17.6$\alpha$/8.3$\beta$-NOD.TCR$\alpha^{-/-}$ mice (upper panel) or naive CD8$^+$ T cells from 17.4$\alpha$/8.3$\beta$-NOD versus LCMV-NOD mice (lower panel). Data are representative of at least five experiments.

**FIG. 7A-7B. Memory 17.6$\alpha$/8.3$\beta$ CD8$^+$ T cells kill antigen-pulsed APCs.** FIG. 7A, *In vitro* cytotoxicity of freshly isolated naive CD8$^+$ T cells from 17.4$\alpha$/8.30$\beta$-NOD.TCR$\alpha^{-/-}$ mice versus naive and memory CD8$^+$ T cells from 17.6$\alpha$/8.30$\beta$-NOD.TCR$\alpha^{-/-}$ mice against NRP-A7 and TUM-pulsed BM DCs. Data are representative of three experiments. Purified BM DCs were pulsed with 1 $\mu$g/ml NRP-A7 or TUM and labeled with [$^{51}$Cr]-sodium chromate. Effector:target ratio=8:1 (40000 effectors:5000 target cells). Supernatant was harvested after 8 hours. FIG. 7B, *In vivo* cytotoxicity assay: NRP-A7-pulsed (CFSE$^{lo}$) or TUM-pulsed (CFSE$^{hi}$) B-cell (upper panels) or freshly isolated splenic and LN DCs (lower panels) were injected into Tg hosts at 1:1 ratio. B cells or fresh DCs (from spleen and LNs) were isolated using anti-B220 or anti-CD11c MACS beads, pulsed with 10 $\mu$g/ml of peptides for 2 hours, washed, labeled with CFSE (TUM:3 $\mu$M CFSE, NRP-A7: 0.3 $\mu$M CFSE) for 3 mins at 37°C., washed 3 times and 4-5x10$^6$ cells from each population were injected into the hosts. After 18 hours mice were sacrificed and splenocytes were FACS analyzed.

**FIG 8** depicts a magnified image of pMHC conjugated PLGA (Poly(D,L-lactide-*co*-glycolide)) nanospheres.

**FIG 9** depicts the IFN$\gamma$ responses by CD8+ T-cells to IGRP/Kd-PLGA nanosphere complexes at the indicated nanoparticles/well concentration.

## DETAILED DESCRIPTION OF THE INVENTION

It is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

It must be noted that as used herein and in the appended claims, the singular forms "a", "an", and "the" include plural referents unless the context clearly dictates otherwise. Thus, for example, reference to "an excipient" includes a plurality of excipients.

## I. DEFINITIONS

Unless defined otherwise, all technical and scientific terms used herein have the same meaning as commonly understood by one of ordinary skill in the art to which this invention belongs. As used herein the following terms have the following meanings.

As used herein, the term "comprising" or "comprises" is intended to mean that the compositions and methods include the recited elements, but not excluding others. "Consisting essentially of" when used to define compositions and methods, shall mean excluding other elements of any essential significance to the combination for the stated purpose. Thus, a composition consisting essentially of the elements as defined herein would not exclude other materials or steps that do not materially affect the basic and novel characteristic(s) of the claimed invention. "Consisting of" shall mean excluding more than trace elements of other ingredients and substantial method steps. Embodiments defined by each of these transition terms are within the scope of this invention.

By "biocompatible", it is meant that the components of the delivery system will not cause tissue injury or injury to the human biological system. To impart biocompatibility, polymers and excipients that have had history of safe use in humans or with GRAS (Generally Accepted As Safe) status, will be used preferentially. By biocompatibility, it is meant that the ingredients and excipients used in the composition will ultimately be "bioabsorbed" or cleared by the body with no adverse effects to the body. For a composition to be biocompatible, and be regarded as non-toxic, it must not cause toxicity to cells. Similarly, the term "bioabsorbable" refers to nanoparticles made from materials which undergo bioabsorption *in vivo* over a period of time such that long term accumulation of the material in the patient is avoided. In a preferred embodiment, the biocompatible nanoparticle is bioabsorbed over a period of less than 2 years, preferably less than 1 year and even more preferably less than 6 months. The rate of bioabsorption is related to the size of the particle, the material used, and other factors well recognized by the skilled artisan. A mixture of bioabsorbable, biocompatible materials can be used to form the nanospheres used in this invention. In one embodiment, iron (III) oxide and a biocompatible, bioabsorbable polymer can be combined. For example, iron (III) oxide and PLGA can be combined to form a nanoparticle.

An antigen/MHC/nanosphere complex refers to presentation of a peptide, carbohydrate, lipid, or other antigenic segment, fragment, or epitope of an antigenic molecule or protein (i.e., self peptide or autoantigen) on a surface, such as a biocompatible biodegradable nanosphere. "Antigen" as used herein refers to all, part, fragment, or segment of a molecule that can induce an immune response in a subject or an expansion of anti-pathogenic cells.

The term "about" when used before a numerical designation, e.g., temperature, time, amount, and concentration, including range, indicates approximations which may vary by (+) or (-) 10 %, 5 %, or 1 %.

By "killing" or "kills" it is meant to cause cell death by apoptosis or necrosis. Apoptosis or necrosis can be mediated by any cell death pathway.

"Autoimmune cells" include, for example, adult splenocytes, T lymphocytes, B lymphocytes, and cells of bone marrow origin, such as defective antigen presenting cells of a mammal, that have activity towards the organism from which the autoimmune cell is derived.

A "mimic" is an analog of a given ligand or peptide, wherein the analog is substantially similar to the ligand. "Substantially similar" means that the analog has a binding profile similar to the ligand except the mimic has one or more functional groups or modifications that collectively accounts for less than about 50%, less than about 40%, less than about 30%, less than about 20%, less than about 10%, or less than about 5% of the molecular weight of the ligand.

An "effective amount" is an amount sufficient to achieve the intended purpose, e.g., modulation of T cell activity or T cell populations. As described herein in detail, the effective amount, or dosage, depends on the purpose and the antigen and can be determined according to the present disclosure.

An "auto-reactive T cell" is a T cell that recognizes an "auto-antigen", which is a molecule produced and contained by the same individual that contains the T cell. An auto-reactive T cell can be a CD8+ T cell or a CD4+ T cell. Furthermore, the CD4+ T cell can be classified as a TR1 (T Regulatory 1) cell.

A "pathogenic T cell" is a T cell that is harmful to a subject containing the T cell. Whereas, a non-pathogenic T cell is not substantially harmful to a subject, and an anti-pathogenic T cells reduces, ameliorates, inhibits, or negates the harm of a pathogenic T cell.

The terms "inhibiting," "reducing," or "prevention," or any variation of these terms, when used in the claims and/or the specification includes any measurable decrease or complete inhibition to achieve a desired result.

The use of the word "a" or "an" when used in conjunction with the term "comprising" in the claims and/or the specification may mean "one," but it is also consistent with the meaning of "one or more," "at least one," and "one or more than one."

The term "anti-pathogenic" refers to cells that have a protective effect against causing disease. For example, "anti-pathogenic autoreactive $CD8^+$ T cells" refers to cells that have a protective effect against T1D. "Anti-pathogenic autoreactive $CD8^+$ T cells" also refers to cells that have a protective effect against other autoimmune diseases such as those listed under subsection V. titled: DIAGNOSTIC AND THERAPEUTIC TARGETS.

By "nanosphere" herein is meant small discrete particles that are administrable to a subject. In certain embodiments, the nanospheres are substantially spherical in shape. The term "substantially spherical," as used herein, means that the shape of the particles does not deviate from a sphere by more than about 10%. Various known antigen or peptide complexes of the invention may be applied to the particles. The nanospheres of this invention range in size from about 10 nm to about 150 $\mu$m and, preferably, from about 10 nm to about 1 $\mu$m. Smaller nanosize particles can be obtained, for example, by fractionation whereby the larger particles are allowed to settle in an aqueous solution. The upper portion of the solution is then recovered. This upper portion is enriched in smaller size particles. The process can be repeated until a desired average size is generated.

Throughout this application, the term "about" is used to indicate that a value includes the standard deviation of error for the device or method being employed to determine the value.

The use of the term "or" in the claims is used to mean "and/or" unless explicitly indicated to refer to alternatives only or the alternatives are mutually exclusive, although the disclosure supports a definition that refers to only alternatives and "and/or."

As used herein and in the claims, the terms "antibody" or "immunoglobulin" are used interchangeably and refer to any of several classes of structurally related proteins that function as part of the immune response of an animal or recipient, which proteins include IgG, IgD, IgE, IgA, IgM and related proteins.

As used herein the terms "immunogenic agent" or "immunogen" or "antigen" are used interchangeably to describe a molecule capable of inducing an immunological response against itself on administration to a recipient, either alone, in conjunction with an adjuvant, or presented on a display vehicle.

As used herein, an "amino molecule" refers to any amino acid, amino acid derivative, or amino acid mimic known in the art. In certain embodiments, the residues of the proteinaceous molecule are sequential, without any non-amino molecule interrupting the sequence of amino molecule residues. In other embodiments, the sequence may comprise one or more non-amino molecule moieties. In particular embodiments, the sequence of residues of the proteinaceous molecule may be interrupted by one or more non-amino molecule moieties.

Accordingly, the term "proteinaceous composition" encompasses amino molecule sequences comprising at least one of the 20 common amino acids in naturally synthesized proteins, or at least one modified or unusual amino acid.

As used herein, the term "treatment" or "treating" means any treatment of a disease or condition in a patient, including:

- preventing or protecting against the disease or condition, that is, causing the clinical symptoms not to develop, for example, in a subject at risk of suffering from such a disease or condition, thereby substantially averting onset of the disease or condition;
- inhibiting the disease or condition, that is, arresting or suppressing the development of clinical symptoms; and/or
- relieving the disease or condition that is, causing the regression of clinical symptoms.

Other objects, features and advantages of the present invention will become apparent from the following detailed description.

Observations to date (Han *et al.,* 2005) suggested that, to be effective in autoimmunity, peptide therapy would have to target multiple epitope specificities. Soluble peptides can induce peptide-specific T-cell tolerance, but cannot blunt poly-specific autoimmune responses. The inventors reasoned that it would be highly impractical to accomplish this with peptides because, in the case of IGRP alone, this would require several milligrams of peptides per dose. Because peptides are much more tolerogenic (i.e., at lower amounts) when bound to MHC molecules on fixed APCs (Miller *et al.,* 1979), it was contemplated that systemic delivery of antigen/MHC complexes, e.g., peptide/MHC complexes (without costimulatory molecules) on nanospheres might be more tolerogenic than peptides alone. This thought evolved from the availability of a reagent initially conceived to image islet inflammation. The inventors sought to specifically deliver a probe amenable to magnetic resonance (MR) imaging to circulating 8.3-like $CD8^+$ T-cells (iron oxide nanospheres coated with NRP-V7/$K^d$ complexes) (Moore *et al.,* 2004). In particular, the inventors contemplated coating these nanospheres with several different antigen/MHC complexes as a way to induce the simultaneous deletion of multiple T-cell specificities below the threshold required for T1D development. Most surprisingly, it was found that pMHC complexes attached to a solid support function by expanding, in an epitope-specific manner, autoantigen-experienced autoreactive CD8+ cells that suppress the recruitment of other autoantigenic specificities. This therapeutic avenue exploits a new paradigm in the progression of chronic autoimmune responses that enables the rational design of disease-specific 'nanovaccines' capable of blunting autoimmunity without impairing systemic immunity, a long sought-after goal in the therapy of these disorders. According to the above paradigm, any *single* epitope (pMHC) specificity involved in the disease (among hundreds) can be used, when coated as a ligand on NPs, to blunt complex autoimmune responses. The particle/solid support component is essential, because multimeric soluble pMHCs cannot elicit the type of immune responses induced by their NP-coupled counterparts. The particle enables superior multimerization of pMHC and affords these pMHCs powerful receptor crosslinking properties. Therefore, this paradigm and the therapeutic approach that enabled its discovery provide a platform for a new class of therapeutics in autoimmunity.

It is contemplated that nanospheres coated with antigen/MHC complexes (antigen/MHC/nanosphere complex) will expand the type of low-avidity anti-pathogenic autoreactive $CD8^+$ cells that afforded T1D protection in APL-treated mice (Han *et al.,* 2005; Maree *et al.,* 2006). It is also contemplated that these nanospheres will expand pre-existing pools of memory autoreactive $CD8^+$ T-cells (i.e., they do not induce memory T cells *de novo).* It is believed that these pre-existing pools are predominantly (if not exclusively) comprised of low avidity (anti-pathogenic) autoreactive $CD8^+$ clonotypes. The high-avidity counterparts of these T-cells (with pathogenic activity) do not survive *in vivo* as memory cells, possibly, but not limiting the invention to any particular theory, because they undergo activation-induced cell death upon chronic exposure to their endogenous target beta cell autoantigen. It is believed that these nanospheres need not have to target a prevalent population of autoreactive $CD8^+$ T-cells to be effective: similar results were obtained with nanospheres coated with a subdominant peptide/MHC complex. In addition, it is believed that this technology does not require the design of APLs of defined avidity (unlike the case with peptides), and thus has the potential to accommodate any target antigen or peptide/MHC target. It is contemplated that this technology will restore normoglycemia in NOD mice with newly diagnosed T1D, at rates that are at least comparable, if not better, than those obtained with anti-CD3 mAb treatment, a non-antigen-specific approach that has shown some promise in clinical trials (Herold *et al.,* 2002; Keymeulen *et al.,* 2005).

It is believed that the compositions of the invention can be used to expand pre-existing pools of memory autoreactive $CD8^+$ T-cells (i.e., they do not appear to be able to induce memory T cells *de novo).* These pre-existing pools are predominantly (if not exclusively) comprised of low avidity (anti-pathogenic) autoreactive $CD8^+$ clonotypes. The high-avidity counterparts of these T-cells (with pathogenic activity) do not survive *in vivo* as memory cells and predominantly exist as naive T cells. It is further contemplated that naive T cells undergo cell death upon engaging autoantigen/MHC/nanosphere complexes in the absence of costimulation and so the invention may both delete naive pathogenic T cells and expand anti-diabetogenic memory T cells. The compositions described need not target a prevalent population of autoreactive $CD8^+$ T-cells to be effective. In certain embodiments, the compositions and methods can be used to induce autoreactive T cell tolerance.

It is contemplated that the biodegradable bioabsorbable nanospheres will have surprising and unexpected results compared to pMHC/antigen complexes coated on a non-biodegradable, non-bioabsorbable solid support. These unexpected results include decreased toxicity. Decreased toxicity can refer to a reduction in the accumulation of the nanosphere in organs and/or tissues throughout the body. Decreased toxicity may also refer to a decrease in an undesired biological response, such as a decrease in inflammation or a decrease in tissue damage of organs throughout the body. Inflammation and tissue damage are assessments that can readily be determined by the skilled artisan by known methods. It is also contemplated that the biodegradable, bioabsorbable nanospheres will be more tolerated by the body. This may lead to a reduction in therapeutic dose or an increase or more efficient therapeutic response. The therapeutic response as it relates to autoimmunity can be determined by assays described in the Examples.

In one embodiment of the invention, the biodegradable, bioabsorbable material is chosen by the skilled clinician based on the predicted *in vivo* half-life of the nanosphere. The half-life is one that can readily be determined based on physical properties of the nanosphere such as the material used and the size of the nanosphere. In a preferred embodiment, the rate of degradation corresponds to the time frame that the clinician determines to be one in which the maximum therapeutic effect is achieved. Therefore, it is contemplated that the biodegradable, bioabsorbable nanospheres will have improved and superior therapeutic potential due to selection of a material that will degrade within a predicted timeframe and have the least adverse side-effects.

## II. PHARMACEUTICAL COMPOSITIONS AND ADMINISTRATION

The present invention includes compositions for use in methods for preventing or ameliorating an autoreactive condition. As such, the invention contemplates "vaccines" or immune system modifiers for use in various embodiments. Compositions proposed to be suitable for use as a vaccine may be prepared from autoreactive molecules including autoreactive proteins and their fragments. The invention includes compositions that can be used to induce or modify an immune response against an autoreactive antigen, e.g., a polypeptide, a peptide, a carbohydrate, a lipid or other molecule or molecular fragment and against developing a condition or disease caused by such an autoimmune response.

Compositions of the invention may be conventionally administered parenterally, by injection, for example, intravenously, subcutaneously, or intramuscularly. Additional formulations which are suitable for other modes of administration include oral formulations. Oral formulations include such normally employed excipients as, for example, pharmaceutical grades of mannitol, lactose, starch, magnesium stearate, sodium saccharine, cellulose, magnesium carbonate and the like. These compositions take the form of solutions, suspensions, tablets, pills, capsules, sustained release formulations or powders and contain about 10% to about 95% of active ingredient, preferably about 25% to about 70%.

Typically, compositions of the invention are administered in a manner compatible with the dosage formulation, and in such amount as will be therapeutically effective and immune modifying. The quantity to be administered depends on the subject to be treated. Precise amounts of active ingredient required to be administered depend on the judgment of the practitioner. However, suitable dosage ranges are of the order of ten to several hundred nanograms or micrograms antigen/MHC/nanosphere complex per administration. Suitable regimes for initial administration and boosters are also variable, but are typified by an initial administration followed by subsequent administrations.

The manner of application may be varied widely. Any of the conventional methods for administration of a vaccine are applicable. These are believed to include oral application on a solid physiologically acceptable base or in a physiologically acceptable dispersion, parenterally, by injection and the like. The dosage of the antigen/MHC/nanosphere complex will depend on the route of administration and will vary according to the size and health of the subject.

In many instances, it will be desirable to have multiple administrations of a peptide/MHC/nanosphere complex, about, at most about or at least about 3, 4, 5, 6, 7, 8, 9, 10 or more. The administrations will normally range from 2 day to twelve week intervals, more usually from one to two week intervals. Periodic boosters at intervals of 0.5-5 years, usually two years, will be desirable to maintain the condition of the immune system. The course of the administrations may be followed by assays for autoreactive immune responses and T cell activity.

### A. Combination Therapy

The compositions and related methods of the present invention, particularly administration of a antigen/MHC/nanosphere complex, may also be used in combination with the administration of traditional therapies. These include, but are not limited to, the administration of immunosuppressive or modulating therapies or treatments.

In one aspect, it is contemplated that a antigen/MHC/nanosphere complex is used in conjunction with a cytokine treatment. Alternatively, antigen/MHC/nanosphere complex administration may precede or follow the other treatment by intervals ranging from minutes to weeks. In embodiments where the other agents and/or antigen/MHC/nanosphere complexes are administered separately, one would generally ensure that a significant period of time did not expire between the time of each delivery, such that the agent and antigen/MHC/nanosphere complex would still be able to exert an advantageously combined effect on the subject. In such instances, it is contemplated that one may administer both modalities within about 12-24 h of each other and, more preferably, within about 6-12 h of each other. In some situations, it may be desirable to extend the time period for administration significantly, however, where several days (2, 3, 4, 5, 6 or 7) to several weeks (1, 2, 3, 4, 5, 6, 7 or 8) lapse between the respective administrations.

Various combinations may be employed, for example antigen/MHC/nanosphere complex administration is "A" and the additional agent is "B":

A/B/A B/A/B B/B/A A/A/B A/B/B B/A/A A/B/B/B B/A/B/B
B/B/B/A B/B/A/B A/A/B/B A/B/A/B A/B/B/A/ BB/A/A
B/AB/A B/A/AB A/A/A/B B/A/A/A A/B/A/A A/A/B/A

Administration of the peptide-MHC complex compositions of the present invention to a patient/subject will follow general protocols for the administration of such compounds, taking into account the toxicity, if any. It is expected that the treatment cycles would be repeated as necessary. It also is contemplated that various standard therapies, such as hydration, may be applied in combination with the described therapy.

## B. Pharmaceutical Compositions

In some embodiments, pharmaceutical compositions are administered to a subject. Different aspects of the present invention involve administering an effective amount of a antigen/MHC/nanosphere complex composition to a subject. Additionally, such compositions can be administered in combination with modifiers of the immune system. Such compositions will generally be dissolved or dispersed in a pharmaceutically acceptable carrier or aqueous medium.

The phrases "pharmaceutically acceptable" or "pharmacologically acceptable" refer to molecular entities and compositions that do not produce an adverse, allergic, or other untoward reaction when administered to an animal, or human. As used herein, "pharmaceutically acceptable carrier" includes any and all solvents, dispersion media, coatings, antibacterial and antifungal agents, isotonic and absorption delaying agents, and the like. The use of such media and agents for pharmaceutical active substances is well known in the art. Except insofar as any conventional media or agent is incompatible with the active ingredients, its use in immunogenic and therapeutic compositions is contemplated.

The active compounds of the present invention can be formulated for parenteral administration, e.g., formulated for injection via the intravenous, intramuscular, sub-cutaneous, or even intraperitoneal routes. The preparation of an aqueous composition that contains a antigen/MHC/nanosphere complex that modifies the subject's immune condition will be known to those of skill in the art in light of the present disclosure. Typically, such compositions can be prepared as injectables, either as liquid solutions or suspensions; solid forms suitable for use to prepare solutions or suspensions upon the addition of a liquid prior to injection can also be prepared; and, the preparations can also be emulsified.

The pharmaceutical forms suitable for injectable use include sterile aqueous solutions or dispersions; formulations including sesame oil, peanut oil, or aqueous propylene glycol; and sterile powders for the extemporaneous preparation of sterile injectable solutions or dispersions. In all cases the form must be sterile and must be fluid to the extent that it may be easily injected. It also should be stable under the conditions of manufacture and storage and must be preserved against the contaminating action of microorganisms, such as bacteria and fungi.

The compositions may be formulated into a neutral or salt form. Pharmaceutically acceptable salts, include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier also can be a solvent or dispersion medium containing, for example, water, ethanol, polyol (for example, glycerol, propylene glycol, and liquid poly(ethylene glycol), and the like), suitable mixtures thereof, and vegetable oils. The proper fluidity can be maintained, for example, by the use of a coating, such as lecithin, by the maintenance of the required particle size in the case of dispersion, and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compounds in the required amount in the appropriate solvent with various of the other ingredients enumerated above, as required, followed by sterilization. Sterilization of the solution will be done in such a way as to not diminish the anti-pathogenic properties of the peptide/MHC/nanosphere. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques, which yield a powder of the active ingredient, plus any additional desired ingredient from a previously sterilized solution thereof. One such method of sterilization of the solution is sterile filtration, however, this invention is meant to include any method of sterilization that does not significantly decrease the anti-pathogenic properties of the peptide/MHC/nanosphere complexes. Methods of sterilization that involve intense heat and pressure, such as autoclaving, may compromise the tertiary structure of the complex, thus significantly decreasing the anti-pathogenic properties of the peptide/MHC/nanosphere complexes.

Administration of the compositions according to the present invention will typically be via any common route. This includes, but is not limited to orthotopic, intradermal, subcutaneous, intramuscular, intraperitoneal, intranasal, or intravenous injection. In certain embodiments, a vaccine composition may be inhaled (e.g., U.S. Pat. No. 6,651,655).

An effective amount of therapeutic or prophylactic composition is determined based on the intended goal. The term "unit dose" or "dosage" refers to physically discrete units suitable for use in a subject, each unit containing a predetermined quantity of the composition calculated to produce the desired responses discussed above in association with its administration, i.e., the appropriate route and regimen. The quantity to be administered, both according to number of treatments and unit dose, depends on the result and/or protection desired. Precise amounts of the composition also depend on the judgment of the practitioner and are peculiar to each individual. Factors affecting dose include physical and clinical state of the subject, route of administration, intended goal of treatment (alleviation of symptoms versus cure), and potency, stability, and toxicity of the particular composition. Upon formulation, solutions will be administered in a manner compatible with the dosage formulation and in such amount as is therapeutically or prophylactically effective. The formulations are easily administered in a variety of dosage forms, such as the type of injectable solutions described above.

### C. *In Vitro* or *Ex Vivo* Administration

As used herein, the term *in vitro* administration refers to manipulations performed on cells removed from or outside of a subject, including, but not limited to cells in culture. The term *ex vivo* administration refers to cells which have been manipulated *in vitro,* and are subsequently administered to a subject. The term *in vivo* administration includes all manipulations performed within a subject, including administrations.

In certain aspects of the present invention, the compositions may be administered either *in vitro, ex vivo,* or *in vivo.* In certain *in vitro* embodiments, autologous T cells are incubated with compositions of this invention. The cells can then be used for *in vitro* analysis, or alternatively for *ex vivo* administration.

## III. MHC COMPLEXES

Antigens, including segments, fragments and other molecules derived from an antigenic species, including but not limited to peptides, carbohydrates, lipids or other molecules presented by classical and non-classical MHC molecules of the invention are typically complexed or operatively coupled to a MHC molecule or derivative thereof. Antigen recognition by T lymphocytes is major histocompatibility complex (MHC)-restricted. A given T lymphocyte will recognize an antigen only when it is bound to a particular MHC molecule. In general, T lymphocytes are stimulated only in the presence of self MHC molecules, and antigen is recognized as fragments of the antigen bound to self MHC molecules. MHC restriction defines T lymphocyte specificity in terms of the antigen recognized and in terms of the MHC molecule that binds its antigenic fragment(s). In particular aspects certain antigens will be paired with certain MHC molecules or polypeptides derived there from.

The term "operatively coupled" or "coated" as used herein, refers to a situation where individual polypeptide (e.g., MHC) and antigenic (e.g., peptide) components are combined to form the active complex prior to binding at the target site, for example, an immune cell. This includes the situation where the individual polypeptide complex components are synthesized or recombinantly expressed and subsequently isolated and combined to form a complex, *in vitro,* prior to administration to a subject; the situation where a chimeric or fusion polypeptide (i.e., each discrete protein component of the complex is contained in a single polypeptide chain) is synthesized or recombinantly expressed as an intact complex. Typically, polypeptide complexes are added to the nanospheres to yield nanospheres with adsorbed or coupled polypeptide complexes having a ratio of number of molecules:number of nanosphere ratios from about, at least about or at most about 0.1, 0.5, 1, 10, 100, 500, 1000 or more to:1, more typically 0.1:1 to 50:1. The polypeptide content of the nanospheres can be determined using standard techniques.

**A.** MHC Molecules

Intracellular and extracellular antigens present quite different challenges to the immune system, both in terms of recognition and of appropriate response. Presentation of antigens to T cells is mediated by two distinct classes of molecules MHC class I (MHC-I) and MHC class II (MHC-II), which utilize distinct antigen processing pathways. Peptides derived from intracellular antigens are presented to $CD8^+$ T cells by MHC class I molecules, which are expressed on virtually all cells, while extracellular antigen-derived peptides are presented to $CD4^+$ T cells by MHC-II molecules. However, there are certain exceptions to this dichotomy. Several studies have shown that peptides generated from endocytosed particulate or soluble proteins are presented on MHC-I molecules in macrophages as well as in dendritic cells. In certain embodiments of the invention, a particular peptide derived from an autoantigen is identified and presented in the peptide/MHC/nanosphere complex in the context of an appropriate MHC class I or II polypeptide. In certain aspects, the genetic makeup of a subject may be assessed to determine which MHC polypeptide is to be used for a particular patient and a particular set of peptides.

Non-classical MHC molecules are also contemplated for use in MHC complexes of the invention. Non-classical MHC molecules are non-polymorphic, conserved among species, and possess narrow, deep, hydrophobic ligand binding pockets. These binding pockets are capable of presenting glycolipids and phospholipids to Natural Killer T (NKT) cells. NKT cells represent a unique lymphocyte population that co-express NK cell markers and a semi-invariant T cell receptor (TCR). They are implicated in the regulation of immune responses associated with a broad range of diseases.

**B. Antigenic Components**

Certain aspects of the invention include methods and compositions concerning antigenic compositions including segments, fragments, or epitopes of polypeptides, peptides, nucleic acids, carbohydrates, lipids and other molecules that provoke or induce an antigenic response, generally referred to as antigens. In particular, autoantigens, or antigenic segments or fragments of such autoantigens, which lead to the destruction of a cell via an autoimmune response, can be identified and used in making a MHC/nanosphere complex described herein. Such autoantigens can be presented on pancreatic islets or cell supporting pancreatic islet cells. Embodiments of the invention include compositions and methods for the modulation of an immune response against a particular cell or set of cells that carry out a particular physiologic function.

**1. Peptide Components and Proteinaceous Compositions**

Polypeptides and peptides of the invention may be modified by various amino acid deletions, insertions, and/or substitutions. In particular embodiments, modified polypeptides and/or peptides are capable of modulating an immune response in a subject. As used herein, a "protein" or "polypeptide" or "peptide" refers to a molecule comprising at least five amino acid residues. In some embodiments, a wild-type version of a protein or peptide is employed, however, in many embodiments of the invention, a modified protein or polypeptide is employed to generate a peptide/MHC/nanosphere complex. A peptide/MHC/nanosphere complex can be used to generate an immune response and/or to modify the T cell population of the immune system (i.e., re-educate the immune system). The terms described above may be used interchangeably herein. A "modified protein" or "modified polypeptide" or "modified peptide" refers to a protein or polypeptide whose chemical structure, particularly its amino acid sequence, is altered with respect to the wild-type protein or polypeptide. In some embodiments, a modified protein or polypeptide or peptide has at least one modified activity or function (recognizing that proteins or polypeptides or peptides may have multiple activities or functions). It is specifically contemplated that a modified protein or polypeptide or peptide may be altered with respect to one activity or function yet retains a wild-type activity or function in other respects, such as immunogenicity or ability to interact with other cells of the immune system when in the context of an MHC/nanosphere complex.

Peptides of the invention include any autoreactive peptide. Autoreactive peptides include, but are not limited to $hInsB_{10-18}$ (HLVEALYLV (SEQ ID NO:1)), $hIGRP_{228-236}$ (LNIDLLWSV (SEQ ID NO:2)), $hIGRP_{265-273}$ (VLFGLGFAI (SEQ ID NO:3)), $IGRP_{206-214}$ (VYLKTNVFL (SEQ ID NO:4)), $hIGRP_{206-214}$ (VYLKTNLFL (SEQ ID NO:5)), NRP-A7 (KYNKANAFL (SEQ ID NO:6)), NRP-I4 (KYNIANVFL (SEQ ID NO:7)), NRP-V7 (KYNKANVFL (SEQ ID NO:8)), YAI/$D^b$ (FQDENYLYL (SEQ ID NO:9)) and/or INS $B_{15-23}$ (LYLVCGERG (SEQ ID NO: 10)), as well as peptides and proteins disclosed in U.S. Publication 20050202032. Other peptides that may be used in conjunction with invention as autoreactive peptides or as control peptides include, but are not limited to INS-I9 (LYLVCGERI (SEQ ID NO:11)), TUM (KYQAVTTTL (SEQ ID NO:12)), and G6Pase (KYCLITIFL (SEQ ID NO:13)). In certain aspects, 1, 2, 3, 4, 5, 6 or more peptides can be used. Examples of peptides that can be used in conjunction with the present invention also include those provided in Table 1. These peptides may be associated with specific nanosphere/MHC molecules or multiple peptides may be associated with a common nanosphere and one or more MHC molecule. Administration of combinations of these peptides includes administering a population of nanospheres having multiple peptides attached and/or administering multiple nanosphere populations each having a specific peptide attached or a combination of such nanospheres that includes nanospheres with 1, 2, 3, 4, 5, 6, or more peptides attached to 1, 2, 3, 4, 5, 6, or more nanospheres.

TABLE 1A HLA class I-restricted epitopes for T1D

| Antigen | Epitope | HLA | Amino Acid Sequence | Comments | References |
|---|---|---|---|---|---|
| GAD65 | 114-123 | A2 | VMNILLQYVV (SEQ ID NO:14) | Reactivity detected in immunized HHD mice and T1D patients | Blancou *et. al.* 2007, Panina-Bordignon *et al.* 1995, Mallone *et al.* 2007 |
| | 536-545 | A2 | RMMEYGTTMV (SEQ IDNO:15) | Reactivity detected in plasmid-immunized HHD mice and T1D patients | Blancou *et. al.* 2007 |
| GFAP | 143-151 | A2 | NLAQTDLATV (SEQ ID NO:16) | Reactivity detected in T1D patients | Ouyang *et. al.* 2006 |
| | 214-222 | A2 | QLARQQVHV (SEQ ID NO:17) | Reactivity detected in T1D patients | Ouyang *et al.* 2006 |
| IA-2 | 172-180 | A2 | SLSPLQAEL (SEQ ID NO:18) | Reactivity detected in T1D patients | Ouyang *et al.* 2006 |
| | 482-490 | A2 | SLAAGVKLL (SEQ ID NO:19) | Reactivity detected in T1D patients | Ouyang *et al.* 2006 |
| | 805-813 | A2 | VIVMLTPLV (SEQ ID NO:20) | Reactivity detected in plasmid-immunized HHD mice and T1D patients | Blancou *et al.* 2007 |
| ppIAPP | 5-13 | A2 | KLQVFLIVL (SEQ ID NO:21) | Reactivity detected in T1D patients | Panagiotopoulos *et al.* 2003, Jarchum *et al.* 2008 |
| | 9-17 | A2 | FLIVLSVAL (SEQ ID NO:22) | Reactivity detected in T1D patients | Ouyang *et al.* 2006 |
| IGRP | 152-160 | A2 | FLWSVFMLI (SEQ ID NO:23) | Reactivity detected in T1D patients | Ouyang *et al.* 2006 |
| | 211-219 | A2 | NLFLFLFAV (SEQ ID NO:24) | Reactivity detected in T1D patients | Jarchum *et al.* 2008 |
| | 215-223 | A2 | FLFAVGFYL (SEQ ID NO:25) | Reactivity detected in T1D patients | Ouyang *et al.* 2006, Jarchum *et al.* 2008 |
| | 222-230 | A2 | YLLLRVLNI (SEQ ID NO:26) | Reactivity detected in T1D patients | Jarchum *et al.* 2008 |
| | 228-236 | A2 | LNIDLLWSV (SEQ ID NO:2) | Reactivity to the corresponding epitope from murine IGRP (differeing at 2 amino acids) detected in immunized HHD mice | Takaki *et al.* 2006 |
| | 265-273 | A2 | VLFGLGFAI (SEQ ID NO:3) | Reactivity detected in immunized HHD mice and recent-onset T1D patients | Takaki *et al.* 2006, Unger *et al.* 2007, Jarchum *et al.* 2008 |
| | 293-301 | A2 | RLLCALTSL (SEQ ID NO:27) | Reactivity detected in T1D patients | Ouyang *et al.* 2006 |
| Proinsulin | L2-10 | A2 | ALWMRLLPL (SEQ ID NO:28) | Reactivity detected in HHD mice and T1D patients | Mallone *et al.* 2007, Jarchum *et al.* 2007 |

(continued)

| Antigen | Epitope | HLA | Amino Acid Sequence | Comments | References |
|---|---|---|---|---|---|
| | L3-11 | A2 | LWMRLLPLL (SEQ ID NO:29) | Reactivity to the corresponding epitope from murine proinsulin 1 (differing at 5 amino acids) detected in HHD mice | Jarchum *et al.* 2007 |
| | L6-14 | A2 | RLLPLLALL (SEQ ID NO:30) | Reactivity detected in T1D patients | Mallone *et al.* 2007 |
| | B5-14 | A2 | HLCGSHLVEA (SEQ ID NO:31) | Reactivity to the corresponding mouse proinsulin 1 epitope (differing at one amino acid) detected in HHD mice | Jarchum *et al.* 2007 |
| | B10-18 | A2 | HLVEALYLV (SEQ ID NO:1) | Reactivity detected in immunized HHD mice and T1D patients | Toma *et al.* 2005, Hassainya *et al.* 2005, Pinkse *et al.* 2005 |
| | B 14-22 | A3, A11 | ALYLVCGER (SEQ ID NO:32) | Reactivity detected in T1D patients | Toma *et al.* 2005 |
| | B15-24 | A24 | LYLVCGERGF (SEQ ID NO:33) | Reactivity detected in T1D patients | Toma *et al.* 2005 |
| | B17-25 | A1, A3 | LVCGERGFF (SEQ ID NO:34) | Reactivity detected in T ID patients | Toma *et al.* 2005, Hassainya *et al.* 2005 |
| | B 18-27 | A1, A2, B8, B18 | VCGERGFFYT (SEQ ID NO:35) | Reactivity detected in T1D patients | Toma *et al.* 2005 |
| | B20-27 | A11, B8 | GERGFFYT (SEQ ID NO:36) | Reactivity detected in T1D patients | Toma *et al.* 2005 |
| | B21-29 | A3 | ERGFFYTPK (SEQ ID NO:37) | Reactivity detected in T1D patients | Toma *et al.* 2005 |
| | B25-C1 | B8 | FYTPKTRRE (SEQ ID NO:38) | Reactivity detected in T1D patients | Toma *et al.* 2005 |
| | B27-C5 | B8 | TPKTRREAEDL (SEQ ID NO:39) | Reactivity detected in T1D patients | Toma *et al.* 2005 |
| | C20-28 | A2 | SLQPLALEG (SEQ ID NO:40) | Reactivity detected in peptide-immunized HHD mice | Hassainya *et al.* 2005 |
| | C25-33 | A2 | ALEGSLQKR (SEQ ID NO:41) | Reactivity detected in peptide-immunized HHD mice | Hassainya *et al.* 2005 |
| | C29-A5 | A2 | SLQKRGIVEQ (SEQ ID NO:42) | Reactivity detected in peptide-immunized HHD mice | Hassainya *et al.* 2005 |
| | A1-10 | A2 | GIVEQCCTSI (SEQ ID NO:43) | Reactivity detected in peptide-immunized HHD mice | Hassainya *et al.* 2005 |
| | A2-10 | A2 | IVEQCCTSI (SEQ ID NO:44) | Reactivity to the corresponding mouse proinsulin 1 epitope (differing at one amino acid) detected in HHD mice | Jarchum *et al.* 2007 |
| | A12-20 | A2 | SLYQLENYC (SEQ ID NO:45) | Reactivity detected in peptide-immunized HHD mice | Hassainya *et al. 2005* |

GAD65: 65 kDa Glutamic acid decarboxylase, GFAP: glial fibrillary acidic protein, IA-2: insulinoma-associated antigen 2, ppIAPP: Islet amyloid polypeptide precursor protein, IGRP: Islet-specific glucose 6-phosphatase catalytic subunit-related protein

Table 1B HLA class I-restricted epitopes for MS

| Antigen | Epitope | HLA | Amino Acid Sequence | Comments | References |
|---|---|---|---|---|---|
| MAG | 287-295 | A2 | SLLLELEEV (SEQ ID NO:46) | Recognized by CD8+ T cell lines generated from MS patients and healthy individuals | Tsuchida *et al.* 1994 |
| MAG | 509-517 | A2 | LMWAKIGPV (SEQ ID NO:47) | Recognized by CD8+ T cell lines generated from MS patients and healthy individuals | Tsuchida *et al.* 1994 |
| MAG | 556-564 | A2 | VLFSSDFRI (SEQ ID NO:48) | Recognized by CD8+ T cell lines generated from MS patients and healthy individuals | Tsuchida *et al.* 1994 |
| MBP | 110-118 | A2 | SLSRFSWGA (SEQ ID NO:49) | Recognized by CD8+ T cell lines generated from MS patients and healthy individuals | Tsuchida *et al.* 1994, Jurewicz *et al.* 1998 |
| MOG | 114-122 | A2 | KVEDPFYWV (SEQ ID NO:50) | Reactivity detected in peptide-immunized HHD mice | Mars *et al.* 2007 |
| MOG | 166-175 | A2 | RTFDPHFLRV (SEQ ID NO:51) | Reactivity detected in peptide-immunized HHD mice | Mars *et al.* 2007 |
| MOG | 172-180 | A2 | FLRVPCWKI (SEQ ID NO:52) | Reactivity detected in peptide-immunized HHD mice | Mars *et al.* 2007 |
| MOG | 179-188 | A2 | KITLFVIVPV (SEQ ID NO:53) | Reactivity detected in peptide-immunized HHD mice | Mars *et al.* 2007 |
| MOG | 188-196 | A2 | VLGPLVALI (SEQ ID NO:54) | Reactivity detected in peptide-immunized HHD mice | Mars *et al.* 2007 |
| MOG | 181-189 | A2 | TLFVIVPVL (SEQ ID NO:55) | Reactivity detected in peptide-immunized HHD mice | Mars *et al.* 2007 |
| MOG | 205-214 | A2 | RLAGQFLEEL (SEQ ID NO:56) | Reactivity detected in peptide-immunized HHD mice | Mars *et al.* 2007 |
| PLP | 80-88 | A2 | FLYGALLLA (SEQ ID NO:57) | Recognized by CD8+ T cell lines generated from MS patients and healthy individuals | Tsuchida *et al.* 1994, Dressel *et al.* 1997 |

MBP: myelin basic protein, MAG: myelin-associated glycoprotein, MOG: myelin oligodendrocyte glycoprotein, PLP: proteolipid protein

In certain embodiments, the size of a protein or polypeptide (wild-type or modified), including any complex of a protein or peptide of interest and in particular a MHC/peptide fusion, may comprise, but is not limited to 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 120, 130, 140, 150, 160, 170, 180, 190, 200, 210, 220, 230, 240, 250, 275, 300, 325, 350, 375, 400, 425, 450, 475, 500, 525, 550, 575, 600, 625, 650, 675, 700, 725, 750, 775, 800, 825, 850, 875, 900, 925, 950, 975, 1000, 1100, 1200, 1300, 1400, 1500, 1750, 2000, 2250, 2500 amino molecules or greater, including any range or value derivable therein, or derivative thereof. In certain aspects, 5, 6, 7, 8, 9, 10 or more contiguous amino acids, including derivatives thereof, and fragments of an autoantigen, such as those amino acid sequences disclosed and referenced herein, can be used as antigens. It is contemplated that polypeptides may be mutated by truncation, rendering them shorter than their corresponding wild-type form, but also they might be altered by fusing or conjugating a heterologous protein sequence with a particular function (e.g., for presentation as a protein complex, for enhanced immunogenicity, etc.).

Proteinaceous compositions may be made by any technique known to those of skill in the art, including (i) the expression of proteins, polypeptides, or peptides through standard molecular biological techniques, (ii) the isolation of proteinaceous compounds from natural sources, or (iii) the chemical synthesis of proteinaceous materials. The nucleotide as well as the protein, polypeptide, and peptide sequences for various genes have been previously disclosed, and may be found in the recognized computerized databases. One such database is the National Center for Biotechnology Information's GenBank and GenPept databases (on the World Wide Web at ncbi.nlm.nih.gov/). The all or part of the coding regions for these genes may be amplified and/or expressed using the techniques disclosed herein or as would be known to those of ordinary skill in the art.

Amino acid sequence variants of autoantigenic epitopes and other polypeptides of these compositions can be substitutional, insertional, or deletion variants. A modification in a polypeptide of the invention may affect 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500 or more non-contiguous or contiguous amino acids of a peptide or polypeptide, as compared to wild-type. A peptide or polypeptide that results in an autoimmune response and in particular a pathologic autoimmune response is contemplated for use in methods of the invention.

Deletion variants typically lack one or more residues of the native or wild-type amino acid sequence. Individual residues can be deleted or a number of contiguous amino acids can be deleted. A stop codon may be introduced (by substitution or insertion) into an encoding nucleic acid sequence to generate a truncated protein. Insertional mutants typically involve the addition of material at a non-terminal point in the polypeptide. This may include the insertion of one or more residues. Terminal additions, called fusion proteins, may also be generated.

Substitutional variants typically contain the exchange of one amino acid for another at one or more sites within the protein, and may be designed to modulate one or more properties of the polypeptide, with or without the loss of other functions or properties. Substitutions may be conservative, that is, one amino acid is replaced with one of similar shape and charge. Conservative substitutions are well known in the art and include, for example, the changes of: alanine to serine; arginine to lysine; asparagine to glutamine or histidine; aspartate to glutamate; cysteine to serine; glutamine to asparagine; glutamate to aspartate; glycine to proline; histidine to asparagine or glutamine; isoleucine to leucine or valine; leucine to valine or isoleucine; lysine to arginine; methionine to leucine or isoleucine; phenylalanine to tyrosine, leucine or methionine; serine to threonine; threonine to serine; tryptophan to tyrosine; tyrosine to tryptophan or phenylalanine; and valine to isoleucine or leucine. Alternatively, substitutions may be non-conservative such that a function or activity of a polypeptide or peptide is affected, such as avidity or affinity for a cellular receptor(s). Non-conservative changes typically involve substituting a residue with one that is chemically dissimilar, such as a polar or charged amino acid for a nonpolar or uncharged amino acid, and vice versa.

Proteins of the invention may be recombinant, or synthesized *in vitro.* Alternatively, a recombinant protein may be isolated from bacteria or other host cell.

The term "functionally equivalent codon" is used herein to refer to codons that encode the same amino acid, such as the six codons for arginine or serine, and also refers to codons that encode biologically equivalent amino acids (see Table 2, below).

TABLE 2 Codon Table

| Amino Acids | | | Codons |
|---|---|---|---|
| Alanine | Ala | A | GCA GCC GCG GCU |
| Cysteine | Cys | C | UGC UGU |
| Aspartic acid | Asp | D | GAC GAU |
| Glutamic acid | Glu | E | GAA GAG |
| Phenylalanine | Phe | F | UUC UUU |
| Glycine | Gly | G | GGA GGC GGG GGU |
| Histidine | His | H | CAC CAU |
| Isoleucine | Ile | I | AUA AUC AUU |
| Lysine | Lys | K | AAA AAG |
| Leucine | Leu | L | UUA UUG CUA CUC CUG CUU |
| Methionine | Met | M | AUG |
| Asparagine | Asn | N | AAC AAU |
| Proline | Pro | P | CCA CCC CCG CCU |
| Glutamine | Gln | Q | CAA CAG |
| Arginine | Arg | R | AGA AGG CGA CGC CGG CGU |
| Serine | Ser | S | AGC AGU UCA UCC UCG UCU |
| Threonine | Thr | T | ACA ACC ACG ACI |
| Valine | Val | V | GUA GUC GUG GUU |
| Tryptophan | Trp | W | UGG |
| Tyrosine | Tyr | Y | UAC UAU |

It also will be understood that amino acid and nucleic acid sequences may include additional residues, such as additional N- or C-terminal amino acids, or 5’ or 3’ nucleic acid sequences, respectively, and yet still be essentially as set forth in one of the sequences disclosed herein, so long as the sequence meets the criteria set forth above, including the maintenance of biological protein activity (e.g., immunogenicity). The addition of terminal sequences particularly applies to nucleic acid sequences that may, for example, include various non-coding sequences flanking either of the 5’ or 3’ portions of the coding region.

It is contemplated that in compositions of the invention, there is between about 0.001 mg and about 10 mg of total protein per ml. Thus, the concentration of protein in a composition can be about, at least about or at most about 0.001, 0.010, 0.050, 0.1, 0.2, 0.3, 0.4, 0.5, 0.6, 0.7, 0.8, 0.9, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10.0, 50, 100 μg/ml or mg/ml or more (or any range derivable therein). Of this, about, at least about, or at most about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100% may be peptide/MHC/nanosphere complex.

The present invention contemplates the administration of a peptide/MHC/nanosphere complex to effect a treatment or preventative therapy against the development of a disease or condition associated with autoimmune responses.

In addition, U.S. Pat. No. 4,554,101 (Hopp) teaches the identification and preparation of epitopes from primary amino acid sequences on the basis of hydrophilicity. Through the methods disclosed in Hopp, one of skill in the art would be able to identify potential epitopes from within an amino acid sequence and confirm their immunogenicity. Numerous scientific publications have also been devoted to the prediction of secondary structure and to the identification of epitopes, from analyses of amino acid sequences (Chou & Fasman, 1974a,b; 1978a,b; 1979). Any of these may be used, if desired, to supplement the teachings of Hopp in U.S. Pat. No. 4,554,101.

### 2. Other Antigenic Components

Molecules other than peptides can be used as antigens or antigenic fragments in complex with MHC molecules, such molecules include, but are not limited to carbohydrates, lipids, small molecules, and the like. Carbohydrates are major components of the outer surface of a variety of cells. Certain carbohydrates are characteristic of different stages of differentiation and very often these carbohydrates are recognized by specific antibodies. Expression of distinct carbohydrates can be restricted to specific cell types. Autoantibody responses to endometrial and serum antigens have been shown to be a common feature of endometriosis. There has been described a serum autoantibody response in endometriosis to a number of previously identified antigens, including 2-Heremans Schmidt glycoprotein and carbonic anhydrase, that is specific for a carbohydrate epitope (Yeaman *et al.,* 2002).

### C. Substrates/Nanospheres

In a certain aspect, antigen/MHC complexes are operatively coupled to a substrate. A substrate can be in the form of a nanoparticle comprising a biocompatible, bioabsorbable material. A substrate can also be in the form of a nanoparticle such as those described previously in US Publication 20090155292. Nanoparticles can have a structure of variable dimension and known variously as a nanosphere or biocompatible biodegradable nanosphere. Such particulate formulations containing an antigen/MHC complex can be formed by covalent or non-covalent coupling of the complex to the nanosphere.

The nanospheres typically consist of a substantially spherical core and optionally one or more layers. The core may vary in size and composition. In addition to the core, the nanosphere may have one or more layers to provide functionalities appropriate for the applications of interest. The thicknesses of layers, if present, may vary depending on the needs of the specific applications. For example, layers may impart useful optical properties.

Layers may also impart chemical or biological functionalities, referred to herein as chemically active or biologically active layers, and for these functionalities the layer or layers may typically range in thickness from about 0.001 micrometers (1 nanometer) to about 10 micrometers or more (depending on the desired nanosphere diameter), these layers typically being applied on the outer surface of the nanosphere.

Preferably, the compositions of the core and layers may vary provided that the nanospheres are biocompatible and bioabsorbable. The core could be of homogeneous composition, or a composite of two or more classes of material depending on the properties desired. In certain aspects, metal nanosperes will be used. These metal nanospheres can be formed from Fe, Ca, Ga and the like

As previously stated, the nanosphere may, in addition to the core, include one or more layers. The nanosphere may include a layer consisting of a biodegradable sugar or other polymer. Examples of biodegradable layers include but are not limited to dextran; poly(ethylene glycol); poly(ethylene oxide); mannitol; poly(esters) based on polylactide (PLA), polyglycolide (PGA), polycaprolactone (PCL); poly(hydroxalkanoate)s of the PHB-PHV class; and other modified poly(saccharides) such as starch, cellulose and chitosan. Additionally, the nanosphere may include a layer with suitable surfaces for attaching chemical functionalities for chemical binding or coupling sites.

Layers can be produced on the nanospheres in a variety of ways known to those skilled in the art. Examples include sol-gel chemistry techniques such as described in Iler (1979); Brinker and Scherer (1990). Additional approaches to producing layers on nanospheres include surface chemistry and encapsulation techniques such as described in Partch and Brown (1998); Pekarek et al. (1994); Hanprasopwattana (1996); Davies (1998); and references therein. Vapor deposition techniques may also be used; see for example Golman and Shinohara (2000); and U.S. Pat. No. 6,387,498. Still other approaches include layer-by-layer self-assembly techniques such as described in Sukhorukov et al. (1998); Caruso et al. (1998); Caruso et al. (1999); U.S. Pat. No. 6,103,379 and references cited therein.

Nanospheres may be formed by contacting an aqueous phase containing the antigen/MHC complex and a polymer and a nonaqueous phase followed by evaporation of the nonaqueous phase to cause the coalescence of particles from the aqueous phase as taught in U.S. Pat. No. 4,589,330 or 4,818,542. Preferred polymers for such preparations are natural or synthetic copolymers or polymers selected from the group consisting of gelatin agar, starch, arabinogalactan, albumin, collagen, polyglycolic acid, polylactic acid, glycolide-L(-) lactide, poly(epsilon-caprolactone), poly(epsilon-caprolactone-CO-lactic acid), poly(epsilon-caprolactone-CO-glycolic acid), poly(β-hydroxy butyric acid), poly(ethylene oxide), polyethylene, poly(alkyl-2-cyanoacrylate), poly(hydroxyethyl methacrylate), polyamides, poly(amino acids), poly(2-hydroxyethyl DL-aspartamide), poly(ester urea), poly(L-phenylalanine/ethylene glycol/1,6-diisocyanatohexane) and poly(methyl methacrylate). Particularly preferred polymers are polyesters, such as polyglycolic acid, polylactic acid, glycolide-L(-) lactide, poly(epsilon-caprolactone), poly(epsilon-caprolactone-CO-lactic acid), and poly(epsilon-caprolactone-CO-glycolic acid). Solvents useful for dissolving the polymer include: water, hexafluoroisopropanol, methylenechloride, tetrahydrofuran, hexane, benzene, or hexafluoroacetone sesquihydrate.

**D. Coupling Antigen-MHC Complex with Nanosphere**

In order to couple the substrate or nanospheres to the antigen-MHC complexes the following techniques can be applied.

The binding can be generated by chemically modifying the substrate or nanosphere which typically involves the generation of "functional groups" on the surface, said functional groups being capable of binding to an antigen-MHC complex, and/or linking the optionally chemically modified surface of the substrate or nanoparticle with covalently or non-covalently bonded so-called "linking molecules," followed by reacting the antigen-MHC complex with the nanospheres obtained.

The term "linking molecule" means a substance capable of linking with the substrate or nanosphere and also capable of linking to an antigen-MHC complex.

The term "functional groups" as used hereinbefore is not restricted to reactive chemical groups forming covalent bonds, but also includes chemical groups leading to an ionic interaction or hydrogen bonds with the antigen-MHC complex. Moreover, it should be noted that a strict distinction between "functional groups" generated at the surface and linking molecules bearing "functional groups" is not possible, since sometimes the modification of the surface requires the reaction of smaller linking molecules such as ethylene glycol with the nanosphere surface.

The functional groups or the linking molecules bearing them may be selected from amino groups, carbonic acid groups, thiols, thioethers, disulfides, guanidino, hydroxyl groups, amine groups, vicinal dioles, aldehydes, alpha-haloacetyl groups, mercury organyles, ester groups, acid halide, acid thioester, acid anhydride, isocyanates, isothiocyanates, sulfonic acid halides, imidoesters, diazoacetates, diazonium salts, 1,2-diketones, phosphonic acids, phosphoric acid esters, sulfonic acids, azolides, imidazoles, indoles, N-maleimides, alpha-beta-unsaturated carbonyl compounds, arylhalogenides or their derivatives.

Non-limiting examples for other linking molecules with higher molecular weights are nucleic acid molecules, polymers, copolymers, polymerizable coupling agents, silica, proteins, and chain-like molecules having a surface with the opposed polarity with respect to the substrate or nanosphere. Nucleic acids can provide a link to affinity molecules containing themselves nucleic acid molecules, though with a complementary sequence with respect to the linking molecule.

As examples for polymerizable coupling agents, diacetylene, styrene butadiene, vinylacetate, acrylate, acrylamide, vinyl compounds, styrene, silicone oxide, boron oxide, phosphorous oxide, borates, pyrrole, polypyrrole and phosphates can be cited.

The surface of the substrate or nanosphere can be chemically modified, for instance by the binding of phosphonic acid derivatives having functional reactive groups. One example of these phosphonic acid or phosphonic acid ester derivates is imino-bis(methylenphosphono) carbonic acid which can be synthesized according to the "Mannich-Moedritzer" reaction. This binding reaction can be performed with substrate or nanosphere as directly obtained from the preparation process or after a pre-treatment (for instance with trimethylsilyl bromide). In the first case the phosphonic acid (ester) derivative may for instance displace components of the reaction medium which are still bound to the surface. This displacement can be enhanced at higher temperatures. Trimethylsilyl bromide, on the other hand, is believed to dealkylate alkyl group-containing phosphorous-based complexing agents, thereby creating new binding sites for the phosphonic acid (ester) derivative. The phosphonic acid (ester) derivative, or linking molecules bound thereto, may display the same functional groups as given above. A further example of the surface treatment of the substrate or nanosphere involves heating in a diole such as ethylene glycol. It should be noted that this treatment may be redundant if the synthesis already proceeded in a diole. Under these circumstances the synthesis product directly obtained is likely to show the necessary functional groups. This treatment is however applicable to substrate or nanosphere that were produced in N- or P-containing complexing agents. If such substrate or particle is subjected to an after-treatment with ethylene glycol, ingredients of the reaction medium (e.g. complexing agent) still binding to the surface can be replaced by the diole and/or can be dealkylated.

It is also possible to replace N-containing complexing agents still bound to the particle surface by primary amine derivatives having a second functional group. The surface of the substrate or nanosphere can also be coated with silica. Silica allows a relatively simple chemical conjugation of organic molecules since silica easily reacts with organic linkers, such as triethoxysilane or chlorosilane. The nanosphere surface may also be coated by homo- or copolymers. Examples for polymerizable coupling agents are. N-(3-aminopropyl)-3-mercaptobenzamidine, 3-(trimethoxysilyl)propylhydrazide and 3-trimethoxysilyl)propylmaleimide. Other non-limiting examples of polymerizable coupling agents are mentioned herein. These coupling agents can be used singly or in combination depending on the type of copolymer to be generated as a coating.

Another surface modification technique that can be used with substrates or nanospheres containing oxidic transition metal compounds is conversion of the oxidic transition metal compounds by chlorine gas or organic chlorination agents to the corresponding oxychlorides. These oxychlorides are capable of reacting with nucleophiles, such as hydroxy or amino groups as often found in biomolecules. This technique allows generating a direct conjugation with proteins, for instance-via the amino group of lysine side chains. The conjugation with proteins after surface modification with oxychlorides can also be effected by using a bi-functional linker, such as maleimidopropionic acid hydrazide.

For non-covalent linking techniques, chain-type molecules having a polarity or charge opposite to that of the substrate or nanosphere surface are particularly suitable. Examples for linking molecules which can be non-covalently linked to core/shell nanospheres involve anionic, cationic or zwitter-ionic surfactants, acid or basic proteins, polyamines, polyamides, polysulfone or polycarboxylic acid. The hydrophobic interaction between substrate or nanosphere and amphiphilic reagent having a functional reactive group can generate the necessary link. In particular, chain-type molecules with amphiphilic character, such as phospholipids or derivatised polysaccharides, which can be crosslinked with each other, are useful. The absorption of these molecules on the surface can be achieved by coincubation. The binding between affinity molecule and substrate or nanosphere can also be based on non-covalent, self-organising bonds. One example thereof involves simple detection probes with biotin as linking molecule and avidin- or streptavidin-coupled molecules.

Protocols for coupling reactions of functional groups to biological molecules can be found in the literature, for instance in "Bioconjugate Techniques" (Greg T. Hermanson, Academic Press 1996). The biological molecule (e.g., MHC molecule or derivative thereof) can be coupled to the linking molecule, covalently or non-covalently, in line with standard procedures of organic chemistry such as oxidation, halogenation, alkylation, acylation, addition, substitution or amidation. These methods for coupling the covalently or non-covalently bound linking molecule can be applied prior to the coupling of the linking molecule to the substrate or nanosphere or thereafter. Further, it is possible, by means of incubation, to effect a direct binding of molecules to correspondingly pre-treated substrate or nanospheres (for instance by trimethylsilyl bromide), which display a modified surface due to this pre-treatment (for instance a higher charge or polar surface).

### E. Protein Production

The present invention describes polypeptides, peptides, and proteins for use in various embodiments of the present invention. For example, specific peptides and their complexes are assayed for their abilities to elicit or modulate an immune response. In specific embodiments, all or part of the peptides or proteins of the invention can also be synthesized in solution or on a solid support in accordance with conventional techniques. Various automatic synthesizers are commercially available and can be used in accordance with known protocols. See, for example, Stewart and Young (1984); Tam *et al.* (1983); Merrifield (1986); and Barany and Merrifield (1979). Alternatively, recombinant DNA technology may be employed wherein a nucleotide sequence which encodes a peptide of the invention is inserted into an expression vector, transformed or transfected into an appropriate host cell and cultivated under conditions suitable for expression.

One embodiment of the invention includes the use of gene transfer to cells, including microorganisms, for the production of proteins. The gene for the protein of interest may be transferred into appropriate host cells followed by culture of cells under the appropriate conditions. A nucleic acid encoding virtually any polypeptide may be employed. The generation of recombinant expression vectors, and the elements included therein, are known to one skilled in the art and are briefly discussed herein. Examples of mammalian host cell lines include, but are not limited to Vero and HeLa cells, other B- and T-cell lines, such as CEM, 721.221, H9, Jurkat, Raji, as well as cell lines of Chinese hamster ovary, W138, BHK, COS-7, 293, HepG2, 3T3, RIN and MDCK cells. In addition, a host cell strain may be chosen that modulates the expression of the inserted sequences, or that modifies and processes the gene product in the manner desired. Such modifications (e.g., glycosylation) and processing (e.g., cleavage) of protein products may be important for the function of the protein. Different host cells have characteristic and specific mechanisms for the post-translational processing and modification of proteins. Appropriate cell lines or host systems can be chosen to ensure the correct modification and processing of the foreign protein expressed.

A number of selection systems may be used including, but not limited to HSV thymidine kinase, hypoxanthine-guanine phosphoribosyltransferase, and adenine phosphoribosyltransferase genes, in tk-, hgprt- or aprt-cells, respectively. Also, anti-metabolite resistance can be used as the basis of selection: for dhfr, which confers resistance to trimethoprim and methotrexate; gpt, which confers resistance to mycophenolic acid; neo, which confers resistance to the aminoglycoside G418; and hygro, which confers resistance to hygromycin.

### F. Nucleic Acids

The present invention may include recombinant polynucleotides encoding the proteins, polypeptides, peptides of the invention. The nucleic acid sequences for autoantigens and MHC molecules for presenting the autoantigens, are included and can be used to prepare a peptide/MHC complex.

As used in this application, the term "polynucleotide" refers to a nucleic acid molecule that either is recombinant or has been isolated free of total genomic nucleic acid. Included within the term "polynucleotide" are oligonucleotides (nucleic acids 100 residues or less in length), recombinant vectors, including, for example, plasmids, cosmids, phage, viruses, and the like. Polynucleotides include, in certain aspects, regulatory sequences, isolated substantially away from their naturally occurring genes or protein encoding sequences. Polynucleotides may be RNA, DNA, analogs thereof, or a combination thereof.

In this respect, the term "gene," "polynucleotide," or "nucleic acid" is used to refer to a nucleic acid that encodes a protein, polypeptide, or peptide (including any sequences required for proper transcription, post-translational modification, or localization). As will be understood by those in the art, this term encompasses genomic sequences, expression cassettes, cDNA sequences, and smaller engineered nucleic acid segments that express, or may be adapted to express, proteins, polypeptides, domains, peptides, fusion proteins, and mutants. A nucleic acid encoding all or part of a polypeptide may contain a contiguous nucleic acid sequence encoding all or a portion of such a polypeptide of the following lengths: 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 441, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000, 1010, 1020, 1030, 1040, 1050, 1060, 1070, 1080, 1090, 1095, 1100, 1500, 2000, 2500, 3000, 3500, 4000, 4500, 5000, 5500, 6000, 6500, 7000, 7500, 8000, 9000, 10000, or more nucleotides, nucleosides, or base pairs. It also is contemplated that a particular polypeptide from a given species may be encoded by nucleic acids containing natural variations that having slightly different nucleic acid sequences but, nonetheless, encode the same or substantially similar protein, polypeptide, or peptide.

In particular embodiments, the invention concerns isolated nucleic acid segments and recombinant vectors incorporating nucleic acid sequences that encode an autoantigen and/or a MHC molecule. The term "recombinant" may be used in conjunction with a polypeptide or the name of a specific polypeptide, and this generally refers to a polypeptide produced from a nucleic acid molecule that has been manipulated *in vitro* or that is a replication product of such a molecule.

The nucleic acid segments used in the present invention, regardless of the length of the coding sequence itself, may be combined with other nucleic acid sequences, such as promoters, polyadenylation signals, additional restriction enzyme sites, multiple cloning sites, other coding segments, and the like, such that their overall length may vary considerably. It is therefore contemplated that a nucleic acid fragment of almost any length may be employed, with the total length preferably being limited by the ease of preparation and use in the intended recombinant nucleic acid protocol. In some cases, a nucleic acid sequence may encode a polypeptide sequence with additional heterologous coding sequences, for example to allow for purification of the polypeptide, transport, secretion, post-translational modification, or for therapeutic benefits such as targeting or efficacy. A tag or other heterologous polypeptide may be added to the modified polypeptide-encoding sequence, wherein "heterologous" refers to a polypeptide that is not the same as the modified polypeptide.

## IV. COMPOSITIONS FOR USE IN THERAPEUTIC METHODS

### A. Immune Response and Assays

As discussed above, the invention concerns evoking or modifying an immune response in a subject against an autoantigen. In one embodiment, the resulting immune response or condition can protect against or treat a subject having, suspected of having, or at risk of developing a disease or symptoms related an autoimmune response.

#### 1. Immunoassays

The present invention includes the implementation of serological assays to evaluate whether and to what extent an immune response is present, induced, evoked, or modified by a peptide/MHC/nanosphere complex. There are many types of immunoassays that can be implemented. Immunoassays encompassed by the present invention include, but are not limited to, those described in U.S. Pat. No. 4,367,110 (double monoclonal antibody sandwich assay) and U.S. Pat. No. 4,452,901 (western blot). Other assays include immunoprecipitation of labeled ligands and immunocytochemistry, both *in vitro* and *in vivo.*

One method for quantifying the number of circulating antigen-specific $CD8^+$ T cells is the tetramer assay. In this assay, a specific epitope is bound to synthetic tetrameric forms of fluorescently labeled MHC Class I molecules. Since $CD8^+$ T cells recognize antigen in the form of short peptides bound to Class I molecules, cells with the appropriate T cell receptor will bind to the labeled tetramers and can be quantified by flow cytometry. Although this method is less time-consuming than an ELISPOT assay, the tetramer assay measures only binding, not function. Not all cells that bind a particular antigen necessarily become activated. However, correlation between ELISPOT, tetramer, and cytotoxicity assays has been demonstrated (Goulder *et al.*, 2000).

Immunoassays generally are binding assays. Certain preferred immunoassays are the various types of enzyme linked immunosorbent assays (ELISAs), radioimmunoassays (RIA) or bead based assays, such as Luminex.RTM. technology, are known in the art. Immunohistochemical detection using tissue sections is also particularly useful.

In one example of ELISA, the antibodies or antigens are immobilized on a selected surface, such as a well in a polystyrene microtiter plate, dipstick, or column support. Then, a test composition suspected of containing the desired antigen or antibody, such as a clinical sample, is added to the wells. After binding and washing to remove non specifically bound immune complexes, the bound antigen or antibody may be detected. Detection is generally achieved by the addition of another antibody, specific for the desired antigen or antibody, that is linked to a detectable label. This type of ELISA is known as a "sandwich ELISA". Detection also may be achieved by the addition of a second antibody specific for the desired antigen, followed by the addition of a third antibody that has binding affinity for the second antibody, with the third antibody being linked to a detectable label. Variations on ELISA techniques are known to those of skill in the art.

Competition ELISAs are also possible in which test samples compete for binding with known amounts of labeled antigens or antibodies. The amount of reactive species in the unknown sample is determined by mixing the sample with the known labeled species before or during incubation with coated wells. The presence of reactive species in the sample acts to reduce the amount of labeled species available for binding to the well and thus reduces the ultimate signal.

Irrespective of the format employed, ELISAs have certain features in common, such as coating, incubating or binding, washing to remove non specifically bound species, and detecting the bound immune complexes.

Antigen or antibodies may also be linked to a solid support, such as in the form of plate, beads, dipstick, membrane, or column matrix, and the sample to be analyzed is applied to the immobilized antigen or antibody. In coating a plate with either antigen or antibody, one will generally incubate the wells of the plate with a solution of the antigen or antibody, either overnight or for a specified period. The wells of the plate will then be washed to remove incompletely-adsorbed material. Any remaining available surfaces of the wells are then "coated" with a nonspecific protein that is antigenically neutral with regard to the test antisera. These include bovine serum albumin (BSA), casein, and solutions of milk powder. The coating allows for blocking of nonspecific adsorption sites on the immobilizing surface and thus reduces the background caused by nonspecific binding of antisera onto the surface.

In ELISAs, it is more customary to use a secondary or tertiary detection means rather than a direct procedure. Thus, after binding of the antigen or antibody to the well, coating with a non reactive material to reduce background, and washing to remove unbound material, the immobilizing surface is contacted with the clinical or biological sample to be tested under conditions effective to allow immune complex (antigen/antibody) formation. Detection of the immune complex then requires a labeled secondary binding ligand or antibody, or a secondary binding ligand or antibody in conjunction with a labeled tertiary antibody or third binding ligand.

## B. Assessing an Autoimmune Response or Condition

In addition to the use of proteins, polypeptides, and/or peptides to treat or prevent an autoimmune condition, the present disclosure contemplates the use of these polypeptides, proteins, and/or peptides in a variety of ways, including the detection of the presence of autoantigens or an autoimmune condition to diagnose the presence of certain autoreactive cell populations or conditions. A method of detecting the presence of autoreactivity involves the steps of obtaining a sample from an individual, for example, from one's blood, saliva, tissues, bone, muscle, cartilage, or skin. Following isolation of the sample, diagnostic assays utilizing the polypeptides, proteins, and/or peptides of the present invention may be carried out to detect the presence of autoreactivity, and such assay techniques for determining such in a sample are well known to those skilled in the art and include methods such as tetramer assays, immunoassays, western blot analysis, and/or ELISA assays.

As used herein the phrase "immune response" or its equivalent "immunological response" refers to the development of a cellular response (mediated by antigen-specific T cells or their secretion products) directed against an autoantigen or an related epitope of an autoantigen. A cellular immune response is elicited by the presentation of polypeptide epitopes in association with Class I or Class II MHC molecules, to activate antigen-specific $CD4^+$ T helper cells and/or CD8+ cytotoxic T cells. The response may also involve activation of other components.

For purposes of this specification and the accompanying claims the terms "epitope" and "antigenic determinant" are used interchangeably to refer to a site on an antigen to which B and/or T cells respond or recognize. B-cell epitopes can be formed both from contiguous amino acids or noncontiguous amino acids juxtaposed by tertiary folding of a protein. Epitopes formed from contiguous amino acids are typically retained on exposure to denaturing solvents whereas epitopes formed by tertiary folding are typically lost on treatment with denaturing solvents. An epitope typically includes at least 3, and more usually, at least 5 or 8-10 amino acids in a unique spatial conformation. Methods of determining spatial conformation of epitopes include, for example, x-ray crystallography and 2-dimensional nuclear magnetic resonance. See, e.g., Epitope Mapping Protocols (1996). T-cells recognize continuous epitopes of about nine amino acids for CD8 cells or about 13-15 amino acids for CD4 cells. T cells that recognize the epitope can be identified by in vitro assays that measure antigen-dependent proliferation, as determined by $^3$H-thymidine incorporation by primed T cells in response to an epitope (Burke et al., 1994), by antigen-dependent killing (cytotoxic T lymphocyte assay, Tigges et al., 1996) or by cytokine secretion. The presence of a cell-mediated immunological response can be determined by proliferation assays ($CD4^+$ T cells) or CTL (cytotoxic T lymphocyte) assays.

As used herein and in the claims, the terms "antibody" or "immunoglobulin" are used interchangeably and refer to any of several classes of structurally related proteins that function as part of the immune response of an animal or recipient, which proteins include IgG, IgD, IgE, IgA, IgM and related proteins.

Optionally, an autoantigen or preferably an epitope of an autoantigen, can be chemically conjugated to, or expressed as, a fusion protein with other proteins, such as MHC and MHC related proteins.

As used herein the terms "immunogenic agent" or "immunogen" or "antigen" are used interchangeably to describe a molecule capable of inducing an immunological response against itself on administration to a recipient, either alone, in conjunction with an adjuvant, or presented on a display vehicle.

## V. DIAGNOSTIC AND THERAPEUTIC TARGETS

Embodiments of the invention can be used to treat or ameliorate a number of immune-mediated or autoimmune disease, e.g., diabetes, graft rejection, etc. "Autoimmune disease" includes diseases or disorders arising from and directed against an individual's own tissues or organs or manifestation thereof or a condition resulting there from. In one embodiment, it refers to a condition that results from, or is aggravated by, the production by T cells that are reactive with normal body tissues and antigens. Examples of autoimmune diseases or disorders include, but are not limited to arthritis (rheumatoid arthritis such as acute arthritis, chronic rheumatoid arthritis, gout or gouty arthritis, acute gouty arthritis, acute immunological arthritis, chronic inflammatory arthritis, degenerative arthritis, type II collagen-induced arthritis, infectious arthritis, Lyme arthritis, proliferative arthritis, psoriatic arthritis, Still's disease, vertebral arthritis, and juvenile-onset rheumatoid arthritis, osteoarthritis, arthritis chronica progrediente, arthritis deformans, polyarthritis chronica primaria, reactive arthritis, and ankylosing spondylitis), inflammatory hyperproliferative skin diseases, psoriasis such as plaque psoriasis, gutatte psoriasis, pustular psoriasis, and psoriasis of the nails, atopy including atopic diseases such as hay fever and Job's syndrome, dermatitis including contact dermatitis, chronic contact dermatitis, exfoliative dermatitis, allergic dermatitis, allergic contact dermatitis, dermatitis herpetiformis, nummular dermatitis, seborrheic dermatitis, non-specific dermatitis, primary irritant contact dermatitis, and atopic dermatitis, x-linked hyper IgM syndrome, allergic intraocular inflammatory diseases, urticaria such as chronic allergic urticaria and chronic idiopathic urticaria, including chronic autoimmune urticaria, myositis, polymyositis/dermatomyositis, juvenile dermatomyositis, toxic epidermal necrolysis, scleroderma (including systemic scleroderma), sclerosis such as systemic sclerosis, multiple sclerosis (MS) such as spino-optical MS, primary progressive MS (PPMS), and relapsing remitting MS (RRMS), progressive systemic sclerosis, atherosclerosis, arteriosclerosis, sclerosis disseminata, ataxic sclerosis, neuromyelitis optica (NMO), inflammatory bowel disease (IBD) (for example, Crohn's disease, autoimmune-mediated gastrointestinal diseases, colitis such as ulcerative colitis, colitis ulcerosa, microscopic colitis, collagenous colitis, colitis polyposa, necrotizing enterocolitis, and transmural colitis, and autoimmune inflammatory bowel disease), bowel inflammation, pyoderma gangrenosum, erythema nodosum, primary sclerosing cholangitis, respiratory distress syndrome, including adult or acute respiratory distress syndrome (ARDS), meningitis, inflammation of all or part of the uvea, iritis, choroiditis, an autoimmune hematological disorder, rheumatoid spondylitis, rheumatoid synovitis, hereditary angioedema, cranial nerve damage as in meningitis, herpes gestationis, pemphigoid gestationis, pruritis scroti, autoimmune premature ovarian failure, sudden hearing loss due to an autoimmune condition, IgE-mediated diseases such as anaphylaxis and allergic and atopic rhinitis, encephalitis such as Rasmussen's encephalitis and limbic and/or brainstem encephalitis, uveitis, such as anterior uveitis, acute anterior uveitis, granulomatous uveitis, nongranulomatous uveitis, phacoantigenic uveitis, posterior uveitis, or autoimmune uveitis, glomerulonephritis (GN) with and without nephrotic syndrome such as chronic or acute glomerulonephritis such as primary GN, immune-mediated GN, membranous GN (membranous nephropathy), idiopathic membranous GN or idiopathic membranous nephropathy, membrano- or membranous proliferative GN (MPGN), including Type I and Type II, and rapidly progressive GN, proliferative nephritis, autoimmune polyglandular endocrine failure, balanitis including balanitis circumscripta plasmacellularis, balanoposthitis, erythema annulare centrifugum, erythema dyschromicum perstans, eythema multiform, granuloma annulare, lichen nitidus, lichen sclerosus et atrophicus, lichen simplex chronicus, lichen spinulosus, lichen planus, lamellar ichthyosis, epidermolytic hyperkeratosis, premalignant keratosis, pyoderma gangrenosum, allergic conditions and responses, allergic reaction, eczema including allergic or atopic eczema, asteatotic eczema, dyshidrotic eczema, and vesicular palmoplantar eczema, asthma such as asthma bronchiale, bronchial asthma, and auto-immune asthma, conditions involving infiltration of T cells and chronic inflammatory responses, immune reactions against foreign antigens such as fetal A-B-O blood groups during pregnancy, chronic pulmonary inflammatory disease, autoimmune myocarditis, leukocyte adhesion deficiency, lupus, including lupus nephritis, lupus cerebritis, pediatric lupus, non-renal lupus, extra-renal lupus, discoid lupus and discoid lupus erythematosus, alopecia lupus, systemic lupus erythematosus (SLE) such as cutaneous SLE or subacute cutaneous SLE, neonatal lupus syndrome (NLE), and lupus erythematosus disseminatus, juvenile onset (Type I) diabetes mellitus, including pediatric insulin-dependent diabetes mellitus (IDDM), and adult onset diabetes mellitus (Type II diabetes). Also contemplated are immune responses associated with acute and delayed hypersensitivity mediated by cytokines and T-lymphocytes, sarcoidosis, granulomatosis including lymphomatoid granulomatosis, Wegener's granulomatosis, agranulocytosis, vasculitides, including vasculitis, large-vessel vasculitis (including polymyalgia rheumatica and giant cell (Takayasu's) arteritis), medium-vessel vasculitis (including Kawasaki's disease and polyarteritis nodosa/periarteritis nodosa), microscopic polyarteritis, immunovasculitis, CNS vasculitis, cutaneous vasculitis, hypersensitivity vasculitis, necrotizing vasculitis such as systemic necrotizing vasculitis, and ANCA-associated vasculitis, such as Churg-Strauss vasculitis or syndrome (CSS) and ANCA-associated small-vessel vasculitis, temporal arteritis, aplastic anemia, autoimmune aplastic anemia, Coombs positive anemia, Diamond Blackfan anemia, hemolytic anemia or immune hemolytic anemia including autoimmune hemolytic anemia (AIHA), Addison's disease, autoimmune neutropenia, pancytopenia, leukopenia, diseases involving leukocyte diapedesis, CNS inflammatory disorders, Alzheimer's disease, Parkinson's disease, multiple organ injury syndrome such as those secondary to septicemia, trauma or hemorrhage, antigen-antibody complex-mediated diseases, anti-glomerular basement membrane disease, anti-phospholipid antibody syndrome, allergic neuritis, Behcet's disease/syndrome, Castleman's syndrome, Goodpasture's syndrome, Reynaud's syndrome, Sjogren's syndrome, Stevens-Johnson syndrome, pemphigoid such as pemphigoid bullous and skin pemphigoid, pemphigus (including pemphigus vulgaris, pemphigus foliaceus, pemphigus mucus-membrane pemphigoid, and pemphigus erythematosus), autoimmune polyendocrinopathies, Reiter's disease or syndrome, thermal injury, preeclampsia, an immune complex disorder such as immune complex nephritis, antibody-mediated nephritis, polyneuropathies, chronic neuropathy such as IgM polyneuropathies or IgM-mediated neuropathy, autoimmune or immune-mediated thrombocytopenia such as idiopathic thrombocytopenic purpura (ITP) including chronic or acute ITP, scleritis such as idiopathic cerato-scleritis, episcleritis, autoimmune disease of the testis and ovary including autoimmune orchitis and oophoritis, primary hypothyroidism, hypoparathyroidism, autoimmune endocrine diseases including thyroiditis such as autoimmune thyroiditis, Hashimoto's disease, chronic thyroiditis (Hashimoto's thyroiditis), or subacute thyroiditis, autoimmune thyroid disease, idiopathic hypothyroidism, Graves' disease, polyglandular syndromes such as autoimmune polyglandular syndromes (or polyglandular endocrinopathy syndromes), paraneoplastic syndromes, including neurologic paraneoplastic syndromes such as Lambert-Eaton myasthenic syndrome or Eaton-Lambert syndrome, stiff-man or stiff-person syndrome, encephalomyelitis such as allergic encephalomyelitis or encephalomyelitis allergica and experimental allergic encephalomyelitis (EAE), myasthenia gravis such as thymoma-associated myasthenia gravis, cerebellar degeneration, neuromyotonia, opsoclonus or opsoclonus myoclonus syndrome (OMS), and sensory neuropathy, multifocal motor neuropathy, Sheehan's syndrome, autoimmune hepatitis, chronic hepatitis, lupoid hepatitis, giant cell hepatitis, chronic active hepatitis or autoimmune chronic active hepatitis, lymphoid interstitial pneumonitis (LIP), bronchiolitis obliterans (non-transplant) vs NSIP, Guillain-Barre syndrome, Berger's disease (IgA nephropathy), idiopathic IgA nephropathy, linear IgA dermatosis, acute febrile neutrophilic dermatosis, subcorneal pustular dermatosis, transient acantholytic dermatosis, cirrhosis such as primary biliary cirrhosis and pneumonocirrhosis, autoimmune enteropathy syndrome, Celiac or Coeliac disease, celiac sprue (gluten enteropathy), refractory sprue, idiopathic sprue, cryoglobulinemia, amylotrophic lateral sclerosis (ALS; Lou Gehrig's disease), coronary artery disease, autoimmune ear disease such as autoimmune inner ear disease (AIED), autoimmune hearing loss, polychondritis such as refractory or relapsed or relapsing polychondritis, pulmonary alveolar proteinosis, Cogan's syndrome/nonsyphilitic interstitial keratitis, Bell's palsy, Sweet's disease/syndrome, rosacea autoimmune, zoster-associated pain, amyloidosis, a non-cancerous lymphocytosis, a primary lymphocytosis, which includes monoclonal B cell lymphocytosis (e.g., benign monoclonal gammopathy and monoclonal gammopathy of undetermined significance, MGUS), peripheral neuropathy, paraneoplastic syndrome, channelopathies such as epilepsy, migraine, arrhythmia, muscular disorders, deafness, blindness, periodic paralysis, and channelopathies of the CNS, autism, inflammatory myopathy, focal or segmental or focal segmental glomerulosclerosis (FSGS), endocrine opthalmopathy, uveoretinitis, chorioretinitis, autoimmune hepatological disorder, fibromyalgia, multiple endocrine failure, Schmidt's syndrome, adrenalitis, gastric atrophy, presenile dementia, demyelinating diseases such as autoimmune demyelinating diseases and chronic inflammatory demyelinating polyneuropathy, Dressler's syndrome, alopecia greata, alopecia totalis, CREST syndrome (calcinosis, Raynaud's phenomenon, esophageal dysmotility, sclerodactyl), and telangiectasia), male and female autoimmune infertility, e.g., due to anti-spermatozoan antibodies, mixed connective tissue disease, Chagas' disease, rheumatic fever, recurrent abortion, farmer's lung, erythema multiforme, post-cardiotomy syndrome, Cushing's syndrome, bird-fancier's lung, allergic granulomatous angiitis, benign lymphocytic angiitis, Alport's syndrome, alveolitis such as allergic alveolitis and fibrosing alveolitis, interstitial lung disease, transfusion reaction, leprosy, malaria, parasitic diseases such as leishmaniasis, kypanosomiasis, schistosomiasis, ascariasis, aspergillosis, Sampter's syndrome, Caplan's syndrome, dengue, endocarditis, endomyocardial fibrosis, diffuse interstitial pulmonary fibrosis, interstitial lung fibrosis, pulmonary fibrosis, idiopathic pulmonary fibrosis, cystic fibrosis, endophthalmitis, erythema elevatum et diutinum, erythroblastosis fetalis, eosinophilic faciitis, Shulman's syndrome, Felty's syndrome, flariasis, cyclitis such as chronic cyclitis, heterochronic cyclitis, iridocyclitis (acute or chronic), or Fuch's cyclitis, Henoch-Schonlein purpura, human immunodeficiency virus (HIV) infection, SCID, acquired immune deficiency syndrome (AIDS), echovirus infection, sepsis, endotoxemia, pancreatitis, thyroxicosis, parvovirus infection, rubella virus infection, post-vaccination syndromes, congenital rubella infection, Epstein-Barr virus infection, mumps, Evan's syndrome, autoimmune gonadal failure, Sydenham's chorea, post-streptococcal nephritis, thromboangitis ubiterans, thyrotoxicosis, tabes dorsalis, chorioiditis, giant cell polymyalgia, chronic hypersensitivity pneumonitis, keratoconjunctivitis sicca, epidemic keratoconjunctivitis, idiopathic nephritic syndrome, minimal change nephropathy, benign familial and ischemia-reperfusion injury, transplant organ reperfusion, retinal autoimmunity, joint inflammation, bronchitis, chronic obstructive airway/pulmonary disease, silicosis, aphthae, aphthous stomatitis, arteriosclerotic disorders, asperniogenese, autoimmune hemolysis, Boeck's disease, cryoglobulinemia, Dupuytren's contracture, endophthalmia phacoanaphylactica, enteritis allergica, erythema nodosum leprosum, idiopathic facial paralysis, chronic fatigue syndrome, febris rheumatica, Hamman-Rich's disease, sensoneural hearing loss, haemoglobinuria paroxysmatica, hypogonadism, ileitis regionalis, leucopenia, mononucleosis infectiosa, traverse myelitis, primary idiopathic myxedema, nephrosis, ophthalmia symphatica, orchitis granulomatosa, pancreatitis, polyradiculitis acuta, pyoderma gangrenosum, Quervain's thyreoiditis, acquired spenic atrophy, non-malignant thymoma, vitiligo, toxic-shock syndrome, food poisoning, conditions involving infiltration of T cells, leukocyte-adhesion deficiency, immune responses associated with acute and delayed hypersensitivity mediated by cytokines and T-lymphocytes, diseases involving leukocyte diapedesis, multiple organ injury syndrome, antigen-antibody complex-mediated diseases, antiglomerular basement membrane disease, allergic neuritis, autoimmune polyendocrinopathies, oophoritis, primary myxedema, autoimmune atrophic gastritis, sympathetic ophthalmia, rheumatic diseases, mixed connective tissue disease, nephrotic syndrome, insulitis, polyendocrine failure, autoimmune polyglandular syndrome type I, adult-onset idiopathic hypoparathyroidism (AOIH), cardiomyopathy such as dilated cardiomyopathy, epidermolisis bullosa acquisita (EBA), hemochromatosis, myocarditis, nephrotic syndrome, primary sclerosing cholangitis, purulent or nonpurulent sinusitis, acute or chronic sinusitis, ethmoid, frontal, maxillary, or sphenoid sinusitis, an eosinophil-related disorder such as eosinophilia, pulmonary infiltration eosinophilia, eosinophilia-myalgia syndrome, Loffler's syndrome, chronic eosinophilic pneumonia, tropical pulmonary eosinophilia, bronchopneumonic aspergillosis, aspergilloma, or granulomas containing eosinophils, anaphylaxis, seronegative spondyloarthritides, polyendocrine autoimmune disease, sclerosing cholangitis, sclera, episclera, chronic mucocutaneous candidiasis, Bruton's syndrome, transient hypogammaglobulinemia of infancy, Wiskott-Aldrich syndrome, ataxia telangiectasia syndrome, angiectasis, autoimmune disorders associated with collagen disease, rheumatism, neurological disease, lymphadenitis, reduction in blood pressure response, vascular dysfunction, tissue injury, cardiovascular ischemia, hyperalgesia, renal ischemia, cerebral ischemia, and disease accompanying vascularization, allergic hypersensitivity disorders, glomerulonephritides, reperfusion injury, ischemic re-perfusion disorder, reperfusion injury of myocardial or other tissues, lymphomatous tracheobronchitis, inflammatory dermatoses, dermatoses with acute inflammatory components, multiple organ failure, bullous diseases, renal cortical necrosis, acute purulent meningitis or other central nervous system inflammatory disorders, ocular and orbital inflammatory disorders, granulocyte transfusion-associated syndromes, cytokine-induced toxicity, narcolepsy, acute serious inflammation, chronic intractable inflammation, pyelitis, endarterial hyperplasia, peptic ulcer, valvulitis, and endometriosis.

## VI. EXAMPLES

The following examples are given for the purpose of illustrating various embodiments of the invention and are not meant to limit the present invention in any fashion.

### Example 1

### T1D Protection by Treatment with Peptide/MHC-Coated Nanospheres

Diabetes protection by treatment with super-paramagnetic nanospheres coated with NRP-V7/$K^d$ monomers. To study whether NRP-V7/$K^d$-coated nanospheres are tolerogenic *in vivo*, 8.3-TCR-transgenic NOD mice will be treated (also referred to as 8.3-NOD or V$\alpha$17.4+TCR-TG mice further below) with several i.v. injections of a small volume of nanospheres (5 $\mu$l, carrying 0.6 $\mu$g of NRP-V7, once every 3 days). It is contemplated that the transgenic high-avidity $IGRP_{206-214}$-reactive splenic CD8+ T-cell pools of these mice will be significantly depleted. It is also contemplated that the non deleted $CD8^+$T cells will be anergized by the treatment.

To study the effectiveness of 'multiplexing', nanospheres will be coated with 6 different peptide/MHC monomers. Cohorts of wild-type NOD mice will be treated with a pool of these nanospheres, with nanospheres coated with a control peptide (TUM)/$K^d$, or with nanospheres coated with NRP-V7/$K^d$. It is contemplated that NOD mice treated with NRP-V7/$K^d$-coated nanospheres (once every 2-3 wk) will be highly protected from T1D while mice treated with uncoated nanospheres, avidin biotin coated nanospheres, TUM/ $K^d$ coated nanospheres or NRP V7 peptide alone will likely not be protected from T1D.

Systemic expansion of low-avidity clonotypes by treatment with nanospheres coated with NRP-V7/$K^d$ monomers. Studies will be done employing radioactively-labeled nanospheres to determine their tissue distribution. It is contemplated that there will be systemic tissue distribution at all ages of the mice examined. It is further contemplated that treatment of the mice with the nanospheres will not lead to increased serum levels of cytokines resulting from stimulation of diverse immune cell types, including NRP-V7-reactive CD8+ T-cells.

It is believed that mice treated with NRP-V7/$K^d$-coated nanospheres will have significantly increased pools of circulating and intra-islet NRP-V7/$K^d$ tetramer$^+$ $CD8^+$ cells at the end of the follow-up period (32 wk), as compared to those of age-matched non-diabetic animals treated with control nanospheres. It is further contemplated that the intra-islet $CD8^+$ T-cells of the NRP-V7/$K^d$-nanosphere-treated mice will bind NRP-V7/$K^d$ tetramers with significantly lower avidity (higher $K^d$) than those found in the islets of control mice, suggesting that the nanosphere treatment will foster the expansion of anti-pathogenic low-avidity clonotypes at the expense of their pathogenic high-avidity counterparts in a dosage-dependent manner.

To investigate the recognition and uptake of NRP V7/K/$K^d$-coated nanospheres, the inventors will assess the presence of green fluorescence (bound to the avidin molecule of the peptide-MHC np complex) in different splenocyte subpopulations of NOD mice expressing a transgenic NRP-V7-reactive TCR as well as in wild-type, non-transgenic NOD mice. It is contemplated that NRP-V7/$K^d$ nanosphere injection, green fluorescence will only be detected in the $CD8^+$ T-cell subset of TCR-transgenic mice and, to a much lesser extent, in the $CD8^+$ T-cell subset of non-transgenic mice. It is further believed that there will be no detection of accumulation of green fluorescence in the splenic $CD4^+$ T, B, $CD11b^+$, or $CD11c^+$ cell subsets of either type of mice.

Anti-diabetogenic properties of nanospheres coated with a subdominant autoantigenic peptide/MHC ($DMK_{138\text{-}146}$/$D^b$) complex. The inventors will investigate whether the protective effects of the above therapeutic avenue were a peculiarity of NRP-V7-reactive $CD8^+$ T-cells (a prevalent autoreactive T-cell subset in NOD mice), or a phenomenon applicable to other, less dominant autoantigenic specificities. To this end, mice will be treated with beads coated with a peptide that is derived from another autoantigen that is presented by $D^b$ and is targeted by a much smaller pool of diabetogenic autoreactive $CD8^+$ T-cells (residues 138-146 of Dystrophia Myotonica Kinase; DMK; herein referred to as "$DMK_{138\text{-}146}$/$D^b$") (Lieberman et al., 2004). It is contemplated that treatment of NOD mice with $DMK_{138\text{-}146}$/$D^b$-coated nanospheres will cause significant expansions of circulating, splenic and intra-islet $DMK_{138\text{-}146}D^b$-reactive CD8+ T cells and afford significant diabetes protection. It is further contemplated that T cell expansion in vivo will be antigen-specific because $DMK_{138\text{-}146}$/$D^b$-coated nanospheres will likely not expand NRP-V7-reactive $CD8^+$ T cells and NRP-V7/$K^d$-coated nanospheres will likely not expand $DMK_{138\text{-}146}$/$D^b$-reactive T cells.

Impaired recruitment of other IGRP-autoreactive $CD8^+$ T-cell specificities to islets in mice treated with NRP-V7/$K^d$- or $DMK_{138\text{-}146}$/$D^b$-coated nanospheres. The inventors will investigate whether recruitment of nanosphere-expanded low-avidity NRP-V7- and/or $DMK_{138\text{-}146}$/$D^b$-reactive CD8+ T cells impair the recruitment of other beta cell autoreactive T cell specificities to islets. This will be done by comparing responsiveness of islet-associated $CD8^+$ T cells of mice treated with control, NRP-V7/$K^d$- or $DMK_{138\text{-}146}$/$D^b$-coated nanospheres to a panel of 76 different IGRP epitopes as well as $DMK_{138\text{-}146}$. It is expected that the islet-associated $CD8^+$ T cells of mice treated with NRP-V7/$K^d$-coated and $DMK_{138\text{-}146}$/$D^b$-coated nanospheres will produced significantly more IFN-$\gamma$ in response to NRP-V7 and $DMK_{138\text{-}146}$, respectively, than those isolated from control mice. It is contemplated that there will be significant reductions in the number of epitopes capable of eliciting significant IFN-$\gamma$ responses by the islet-associated $CD8^+$ T cells of mice treated with NRP-V7/$K^d$ or $DMK_{138\text{-}14d}D^b$-coated nanospheres, as compared to those from mice treated with control nanospheres, suggesting impaired recruitment and/or accumulation.

**It is believed that NRP-V7/$K^d$- and $DMK_{138\text{-}146/}D^b$-coated nanospheres will induce high rates of diabetes remission in newly diabetic NOD mice.** Studies to investigate the ability of nanosphere therapy to restore normoglycemia in newly diagnosed diabetic mice will be performed. Cohorts of mice will be monitored twice a week for blood glucose levels and considered hyperglycemic at ≥10.5 mM blood glucose. Mice will be randomized into mice receiving TUM/$K^d$-coated nanospheres or NRP-V7/$K^d$-coated nanospheres (two weekly injections). Half a unit of subcutaneous insulin will also be given once daily to mice displaying glycosuria, to reduce beta cell stress and foster beta cell regeneration. Additional cohorts of mice will receive $DMK_{138\text{-}14d}D^b$-coated nanospheres. Treatment with monoclonal anti-CD3 antibody (20 $\mu$g/d for 5 days), which has been shown to induce stable remission in a variable percentage of animals in different studies, will be used as positive control. It is contemplated that the majority of mice treated with NRP-V7/$K^d$-coated nanospheres will become normoglycemic within 5-12 weeks of treatment. Likewise, it is further contemplated that the majority of mice treated with $DMK_{138\text{-}146}$/$D^b$-coated nanospheres will become normoglycemic. It is expected that the majority of mice receiving control TUM/$K^d$-coated nanospheres will progress to overt hyperglycemia.

To investigate whether the effects of treatment in diabetic mice are long-lasting, treatment will be withdrawn after 4 consecutive weeks of normoglycemia and mice will be monitored for diabetes recurrence. The effects of treatment on the size of the circulating tetramer-positive pool will be assessed at treatment withdrawal, 4 weeks later and at the time of recurrent hyperglycemia. It is contemplated that there will be a decline in the size of the circulating tetramer-reactive T cell pool 4 weeks after cessation of treatment. In this case, reactivation of the expanded low-avidity autoreactive T-cell pool, either by endogenous autoantigen (i.e., in pre-diabetic animals) or by booster injections of nanospheres (i.e., in diabetic animals with a severely reduced beta cell mass) may be required for long-term protection.

It is contemplated that intraperitoneal glucose tolerance tests (IPGTTs) in cured versus diabetic and non-diabetic untreated mice will show that the former have glucose tolerance curves nearly identical to those displayed by non-diabetic untreated animals and significantly better than those corresponding to diabetic mice. Furthermore, it is believed that the cured animals will have postprandial serum insulin levels that are statistically comparable to those seen in non-diabetic untreated mice and significantly higher than those corresponding to diabetic untreated animals.

**Investigation of whether Peptide/MHC-coated nanospheres can effectively 'discriminate' between high- and low-avidity autoreactive CD8⁺ T-cells.** Most $IGRP_{206-214}$-reactive $CD8^+$ cells employ CDR3-invariant Vα17-Jα42 chains but heterogeneous VDJβ chains. 'Avidity maturation' of this T cell subset during diabetogenesis is associated with changes in usage of 3 different Vα17 elements. That these 3 different Vα elements afford differences in ligand-binding avidity (Vα17.5>Vα17.4>Vα17.6) was confirmed in studies of TCRαβ-transfectants expressing the 3 different CDR3-invariant Vα17-Jα42 chains in the context of a single TCRβ chain (Han *et al*., 2005). To investigate whether peptide/MHC-coated nanospheres can in fact differentially target T cells recognizing ligand with different avidity, the ability of NRP-V7/$K^d$-coated nanoshperes to induce 'capping' of CD8 molecules on these transfectants will be assessed. It is contemplated that more Vα17.5⁺ cells than Vα17.4⁺ or Vα17.6⁺ cells, will form caps by 5 min of incubation with NRP-V7/$K^d$-coated nanospheres. This result would indicate that NRP-V7/$K^d$-coated nanospheres can in fact discriminate between high and low-avidity T cells and provide an explanation as to why these nanospheres preferentially delete naive high avidity clonotypes.

**$CD8^+$ cells expressing the low-affinity Vα17.6/8.3β TCR are anti-diabetogenic.** To investigate whether low-avidity autoreactive $CD8^+$ T-cells have anti-diabetogenic properties *in vivo*, the low affinity $IGR-P_{206-214}$-reactive Vα17.6/8.3β TCR were transgenically expressed in NOD mice (referred herein to as 'Vα17.6⁺'; which has ~10-food lower affinity than the 8.3-TCR (Vα17.4); Teyton and Santamaria, unpublished data). It has been shown that this TCR fosters positive selection of $CD8^+$ cells, but clearly less than the 8.3-TCR (Vα17.$^{4+}$) (Han *et al*., 2005). As a result, Vα17.6⁺ TCR-TG mice contain fewer NRP-V7 tetramer-reactive $CD8^+$ thymocytes and splenocytes than Vα17.4⁺ TCR-TG mice. Furthermore, the tetramer+ (high and low) $CD8^+$ cells from Vα17.6⁺ TCR-TG mice secrete less IFN-γ (and IL-2) than those derived from Vα17.4⁺ TCR-TG mice upon peptide stimulation *in vitro*, and are inefficient killers of NRP-V7-pulsed RMA-$SK^d$ targets, compatible with their low avidity for ligand (Han *et al*., 2005; and data not shown). Most importantly, these mice are almost completely protected from diabetes (only 2 of 70 females have developed T1D) and insulitis [scores of <0.4 vs >3 (out of a maximum of 4) in Vα17.6⁺ vs. Vα17.4⁺ TCR-TG mice, respectively ($P<0.012$)] (FIG. 1A and FIG. 1B).

This is in stark contrast to what occurs in NOD mice expressing an irrelevant non-autoreactive TCR that recognizes a LCMV epitope (LCMV TCR-TG NOD mice). As reported previously by Serreze *et al.* (2001), and confirmed by use herein, these mice develop T1D essentially like wild-type NOD mice and recruit endogenous $IGRP_{206-214}$-reactive CD8+ cells to islets (completely absent in the islets of Vα17.6⁺ TCR-TG mice) (FIG. 1C). Thus, unlike the Vα17.4⁺ and LCMV TCRs (pro-diabetogenic and neutral, respectively), the Vα17.6⁺ TCR appears to have anti-diabetogenic properties.

Notwithstanding the fact that most TG T cells of these Vα17.6⁺ TCR-TG mice bind tetramers weakly or not at all, a fraction of the cells that exit the thymus binds tetramer with apparent high avidity (i.e., with high mfi) (FIG. 2A). The inventors suspected that the tetramer-low (lo) and tetramer-negative $CD8^+$ T cells of these mice originate from $CD4^+CD8^+$ thymocytes that express the TG TCR but undergo positive selection on endogenous TCRs (i.e., TCRα chains). The tetramer-hi cells, on the other hand, would originate from $CD4^+$ $CD8^+$ thymocytes that only express the TG TCRαβ chains and, because of their low affinity for peptide/MHC, can only undergo positive selection if they express higher levels of the TG TCR than normal. This interpretation is supported by the observation that, in mice expressing the Vα17.6⁺ TCR in a RAG-$2^{-/-}$ background, the only cells that mature are those binding tetramer with high avidity (FIG. 2B). Importantly, these two types of RAG-$2^{-/-}$ TCR-TG mice develop diabetes with similar incidence (FIG. 3). Thus, the inventors suspect that the tetramer-lo and tetramer-$CD8^+$ T cells that mature in RAG-2+Vα17.6 TCR-TG mice inhibit the diabetogenic potential of their tetramer-hi counterparts (which cause diabetes in $RAG^{-/-}$ TCR-TG mice). These results were reproduced in stocks of Vα17.6 TCR-TG mice carrying an endogenous TCR-Cα deficiency that recludes expression of endogenous (i.e., non-transgenic) TCRα chains (FIGS. 4A and 4B).

**Vα17.6+(but not Vα17.4+) TCR-TG mice spontaneously generate a pool of memory $CD8^+$ cells with immunosuppressive activities.** Cytofluorometric studies of the tetramer-positive $CD8^+$ T-cells contained in the different lymphoid organs of Vα17.6⁺ TCR-TG mice and TCR-Cα-deficient Vα17.6 TCR-TG mice revealed the presence of enlarged pools of CD44hi and CD44hi$CD122^+$ CD8+ cells as compared to Vα17.4⁺ TCR-TG mice in the spleen, lymph nodes and, especially, the bone marrow, a known reservoir of memory T-cells (FIGS. 5A and 5B). Importantly, this occurs primarily within the tetramer-low, but not in the tetramer-high subset, which does not contain $CD122^+$ cells (FIG. 5C). These cells express markers described on both central and effector memory lymphocytes (FIG. 5D), and are predominantly found in the peripheral lymphoid organs but not thymus, suggesting a peripheral origin (FIG. 5E). Furthermore, BrdU incorporation assays suggested that they proliferate *in vivo* (FIG. 5F). Functionally, these memory-like cells clearly behave as 'memory' T-cells, as purified splenic Vα17.6⁺(but not Vα17.4⁺ TCR-TG $CD8^+$ cells proliferate vigorously in response to IL-2 or IL-15 in the absence of APCs and antigen (FIG. 5G). Furthermore, they rapidly produce IFN-γ upon stimulation with antigen *in vitro* (FIGS. 5H and 5I). However, they neither proliferate nor produce interleukin-2 upon antigenic stimulation *in vitro* (FIG. 5J). This functional profile is highly reminiscent of that of the regulatory (suppressive) $CD4^+CD25^+$ T cell subset. Altogether, these data suggest that Vα17.6⁺(but not Vα17.4⁺ TCR-TG $CD8^+$

cells have an increased ability to become long-lived memory T cells (upon one or more antigen encounters), presumably capable of surviving indefinitely in response to homeostatic cues, even in the absence of antigen.

These observations led the inventors to suspect that the superior homeostatic 'fitness' of these memory low-avidity T cells, otherwise unable to kill beta cells, contributes to their anti-diabetogenic activity (i.e., by affording a competitive advantage over their higher-avidity, but mostly naive, beta cell killer clonotypes, and/or by inhibiting their activation). To assess the latter, the ability of purified $CD122^+$ and CD122-Vα17.6$^+$ TCR-TG $CD8^+$ T cells were assessed for their ability to inhibit the proliferation of CFSE-labeled splenic $CD8^+$ T cells from Vα17.4$^+$ TCR-TG NOD mice. As shown in FIG. 6, $CD122^+$ (but not $CD122^-$) Vα17.6$^+$ TCR-TG CD8+ T cells almost completely inhibited the proliferation of their higher avidity naive T cell counterparts.

Consistent with the idea that the spontaneously expanded pool of memory ($CD122^+$) low-avidity autoreactive CD8+ T cells in Vα17.6$^+$ TCR-TG NOD mice is anti-diabetogenic, systemic (i.v.) treatment of Vα17.6$^+$ and Vα17.4$^+$ TCR-TG mice with NRP-V7-pulsed DCs, an agonistic mAb against CD40, or an agonistic mAb against 4-1BB (to enhance $CD8^+$ T cell activation/survival), induced rapid onset of diabetes in Vα17.4$^+$ TCR-TG NOD mice, but were unable to elicit disease in Vα17.6$^+$ TCR-TG mice (Table 3).

TABLE 3 Treatments that promote memory T-cell development and expansion precipitate acute onset of diabetes in 17.4α/8.3β-TG NOD mice, but not in 17.6α/8.3β-TG NOD mice.

| Treatment | Host | Diabetes Incidence | Diabetes onset Day (s.e.) |
|---|---|---|---|
| Agonistic Anti-CD40 mAB | 17.4 NOD<br>17.6 NOD | 4/4<br>0/3 | 10.5(4.6)<br>-- |
| Agonistic Anti-4-IBB mAB | 17.4 NOD<br>17.6 NOD | 3/3<br>0/2 | 2.3 (1.5)<br>-- |
| NRP-V7 pulsed Dendritic cells | 17.4 NOD<br>17.6 NOD | N.A.<br>0.3 | --<br>-- |
| 3 injections of Anti-CD40 mAb or Anti-4-1BB mAb 100 μg i.p. with 3-4 days intervals 2 injections of 106 LPS-activated bone marrow-derived DCs pulsed with 100 μg/ml NRP-V7 Mice were followed for diabetes at least 8 weeks after the last injection | | | |

The ability of $CD122^+$ Vα17.6$^+$ TCR-TG $CD8^+$ T cells to suppress cognate and non-cognate diabetogenic T cell responses (i.e., directed against autoantigenic peptides other than the target autoantigenic peptide of these suppressive T-cells-$IGRP_{206-214}$-), led the inventors to suspect that they might effect their suppressive activity by targeting antigen-presenting cells (APCs). Cytotoxicity ($^{51}$Chromium-release) assays employing peptide-pulsed DCs as target cells and $CD122^+$ or CD122-Vα17.6$^+$ and CD122-Vα17.4$^+$ TCR-TG $CD8^+$ T cells as effectors indicated that the former, but not the latter were able to specifically lyse NRP-V7-pulsed DCs in vitro (FIG. 7A). The inventors confirmed that this was also true in vivo. They transfused equal numbers of NRP-V7-pulsed and TUM-pulsed B-cells (labeled with low or high concentrations of the dye CFSE, respectively) into Vα17.6$^+$ TCR-TG and Vα17.4$^+$ TCR-TG mice and a day later sacrificed the hosts to investigate which cells had survived the transfer. As shown in FIG. 7B, whereas NRP-V7-pulsed B-cells only survived in Vα17.4$^+$ TCR-TG mice, B-cells pulsed with the negative control peptide TUM survived in both TCR-TG strains. Virtually identical results were obtained when DCsm rather than B-cells, were used as APCs (FIG. 7B). These data suggest that low-avidity $CD122^+$ Vα17.6$^+$ TCR-TG $CD8^+$ T cells suppress cognate and non-cognate diabetogenic T cell responses by killing autoantigen-loaded APCs.

**Investigate whether NRP-V7/K$^d$-coated nanospheres induce the expansion of low avidity (tetramer-intermediate) memory autoreactive $CD8^+$ cells in wild-type NOD mice.** It is contemplated that treating NOD mice with peptide/MHC-coated nanospheres will result in the disappearance of high avidity clonotypes, and expansion and recruitment of low-avidity $CD8^+$ T cells, impaired recruitment of other IGRP epitope-reactive specificities to islets, and protection from diabetes.

To assess whether the bead-expanded CD8+ T cells in wild-type NOD mice are long-lived low avidity memory T cells, the inventors will analyze the presence of memory markers (CD44 and CD 122) in the NRP-V7/K$^d$ tetramer-positive $CD8^+$ T-cells contained in the spleen bone marrow of mice treated with NRP-V7/K$^d$-coated nanospheres. It is contemplated that the expanded populations of tetramer-positive cells contained in the spleen and marrow of these mice will contain an increased percentages of CD44hi and CD44hi$CD122^+$ $CD8^+$ T-cells, suggesting that NRP-V7/K$^d$ nanosphere treatment increases the size of the tetramer$^+$CD44hi and tetramer$^+$CD44hiCD $122^+$ T cell pools.

It is contemplated that the memory-like T-cells that are expanded by *in vivo* therapy with NRP-V7/K$^d$-coated nanospheres will behave like the memory $CD122^+$ Vα17.6$^+$ TCR-TG $CD8^+$ T cells that spontaneously accumulate in Vα17.6$^+$ TCR-TG mice: they neither produce IL-2 nor proliferate, yet produce high levels of IFN-γ in response to antigenic stimulation *in vitro.* It is further contemplated that, upon activation with anti-CD3 mAb and IL-2, these memory-like T-cells will efficiently suppress the proliferation of CFSE-labeled responder $V\alpha17.4^+$ TCR-TG $CD8^+$ T-cells *in vitro.*

**Peptide/MHC-coated nanospheres expand pre-existing low-avidity memory T cells.** It is believed that peptide/MHC-coated nanospheres will not generate memory T-cells *de novo*, but rather expand pre-existing pools of memory T cells. It is contemplated that treatment of NOD mice with TUM/$K^d$-coated nanospheres will not induce systemic expansion of TUM reactive $CD8^+$T cells. It is further contemplated that treatment of B 10.H2g7 mice with NRP-V7/$K^d$-coated nanospheres will not induce a significant expansion of the NRP-V7/$K^d$ tetramer$^+$$CD8^+$ T-cell subset in all lymphoid organs and systemic expansion of tetramer-reactive $CD8^+$ T-cells in nanosphere-treated NOD mice will be significantly more effective when initiated at diabetes onset than in the pre-diabetic stage. It is also believed that, unlike naive $CD8^+$ T cells, which tend to undergo apoptosis upon TCR ligation in the absence of costimulation, memory $CD8^+$ T cells are costimulation-independent for growth.

To investigate the above hypothesis formally, the inventors will determine whether $IGRP_{206-214}$/$K^d$-coated nanospheres can expand $IGRP_{206-214}$/$K^d$-reactive $CD8^+$ T-cells in a gene-targeted NOD strain that expresses a mutant form of IGRP in which the two TCR-contact residues of $IGRP^{206-214}$ have been replaced with alanines (K209A and F213A). The targeted alleles (herein referred to as FLEX1 or NOD.IGR-$P_{K209A/F213A}{}^{KI/KI}$) are backcrossed onto the NOD background (from 129) using the speed-congenic approach, to ensure homozygosity for NOD alleles at all Idd loci. Because the $CD8^+$ T-cells that mature in these gene-targeted mice are never exposed to $IGRP_{206-214}$ in vivo, these mice cannot spontaneously generate memory $IGRP_{206-214}$/$K^d$-reactive $CD8^+$ T-cells. Despite the fact that these mice develop both diabetes and insulitis (not shown), their islet-associated $CD8^+$ T-cells recognize epitopes in IGRP, but are completely devoid of IGR-$P_{206-214}$-reactive $CD8^+$ clonotypes. It is contemplated that FLEX1-homozygous NOD mice treated with optimal doses of $IGRP_{206-214}$/$K^d$-coated nanospheres will not contain expanded pools of $IGRP_{206-214}$/$K^d$-reactive $CD8^+$ T-cells in their lymphoid organs. This will suggest that peptide/MHC-coated nanosphers expand pre-existing pools of memory T-cells with suppressive properties and cannot generate memory T-cells de novo.

Since low-avidity clonotypes (i.e., in $V\alpha17.6^+$ TCR-TG mice) appear to be more efficient at generating memory T-cell progeny than their high-avidity counterparts (i.e., in $V\alpha17.4^+$ TCR-TG mice) during diabetogenesis, it is believed that peptide/MHC-coated nanospheres work by inducing the deletion of naive high-avidity clonotypes and the expansion of small pools of pre-existing memory low-avidity clonotypes.

**Example 2**

**Testing the Ability of Iron Oxide Nanospheres Coated with Human Type 1 Diabetes-Relevant Peptide/HLA Complexes to Restore Normoglycemia**

**"Humanized" mice expressing HLA transgenes and peptide/HLA complexes available for the proposed studies.** As mentioned above, peptides derived from insulin and IGRP are primary targets of $CD8^+$ T cells in wild-type NOD mice. Assessment of human MHC molecules (Human Leukocyte Antigens, HLA) presented peptides derived from these two autoantigens during diabetogenesis is being investigated in 'humanized' HLA-transgenic NOD mice. Studies focused initially on HLAA*0201, a MHC molecule that is expressed by nearly 50% of certain ethnic groups. This study employs the strain designated NOD.$\beta2$ $m^{null}$.HHD, which lacks the murine $\beta2$ macroglobulin gene and expresses the chimeric monochain construct HHD (Pascolo *et al.*, 1997). This construct encodes human $\beta2m$ covalently linked to the $\alpha1$ and $\alpha2$ domains of human HLA-A*0201, and the $\alpha3$, transmembrane, and cytoplasmic domains of murine H-$2D^b$. Though the strain expresses only HLA-A*0201, and not endogenous murine class I MHC molecules, it is diabetes-susceptible, with 55% of females affected by 30 weeks of age (Takaki *et al.*, 2006). Two epitopes of human IGRP ($hIGRP_{228-236}$ and $IGRP_{265-273}$) that bind to HLA-A*0201 are recognized by islet-associated $CD8^+$ T cells of these mice and $CD8^+$ T cells isolated from the islets of NOD.$\beta2$ $m^{null}$.HHD mice are cytotoxic to human HLA-A*0201-positive islets (Takaki *et al.*, 2006). Peptide/HLA-A*0201 tetramers were made using one of these peptides. To facilitate binding of these tetramers by murine CD8 molecules, the $\alpha3$ (CD8-binding) domain of the HLA-A*0201 complex was replaced with that of the murine H-2 $K^b$ molecule. Results from these studies have established the utility of these mice for the identification of HLA-A*0201-restricted T cells and beta cell autoantigens of potential relevance to human T1D (Takaki *et al.* 2006). Based on the current disclosure, one can identify the human peptides that are targeted by HLA-A*0201-restricted T cells from T1D patients. In addition, the inventors have generated NOD mice expressing HLA-A*1101, HLA-B*0702, or HLA-Cw*0304. These mice also have murine $\beta2m$ replaced with human $\beta2m$ by crossing them with the NOD.$\beta2$ $m^{null}$.h$\beta2m$ strain (Hamilton-Williams *et al.*, 2001). All three HLA transgenes express well, and all three of the HLA-transgenic strains are diabetes-susceptible. Taken together HLAs from these "humanized" animals are representative of the four different HLA supertypes HLA-A2, HLA-A3, HLA-B7, and HLA-C1, respectively (Sidney *et al.*, 1996; Doytchinova *et al.*, 2004). The gene frequencies of HLA-A*1101, HLA-B*0702, or HLA-Cw*0304 alleles can be as high as 23%, 11%, or 10%, respectively, depending on the ethnic group examined (Cao *et al.*, 2001). Coverage of the population can be over 90%, depending on the ethnic group considered, when all four supertypes are targeted (Sidney *et al.*, 1996;

Doytchinova *et al.*, 2004; Cao *et al.*, 2001). This consideration is significant in regard to translation of these studies to humans. These animals as well as the previously described NOD.$\beta 2m^{null}$.HHD strain are available for further studies.

In this Example the inventors propose a design on how to translate these observations in wild-type NOD mice to 'humanized' HLA-transgenic NOD mice. The objective is to investigate if treatment with nanospheres coated with several different peptide/HLA complexes targeting pools of autoreactive $CD8^+$ T cells relevant to human T1D can protect the mice from diabetes as well as restore normoglycemia in their newly-diagnosed counterparts. Here the inventors present a translational approach to identify T1D-relevant peptide/HLA combinations for use in human T1D. Specifically, the inventors contemplate that nanospheres coated with different T1D-relevant autoantigenic peptide/HLA-A*0201 complexes will afford diabetes protection and cure T1D in NOD.$\beta 2m^{null}$.HHD mice (expressing HLA-A*0201). One of skill in the art can use this disclosure for use with other epitopes related to other autoimmune diseases, using compositions and methods similar to those used with insulin and/or IGRP epitopes presented by other HLA molecules in 'humanized' HLA-transgenic mice to islet-associated $CD8^+$ T cells, to include other compositions and methods. One of skill will be able to identify the minimal treatment conditions and the type of peptide/HLA complexes that will afford maximum therapeutic benefits, as well as the requirement for pre-therapeutic existence of memory low-avidity $CD8^+$ T cells for therapeutic success and identification of additional peptide/HLA combinations covering as many individuals in different ethnic groups as possible.

**Nanosphere synthesis.** Nanospheres are synthesized and characterized at the physical and chemical levels essentially as described previously (Moore et al., 2004), but using biotinylated peptide/HLA-A*0201 monomers. The MHC molecule of the complex is composed of human $\beta 2$ microglobulin and a chimeric form of human HLA-A*0201 in which its $\alpha 1$ and $\alpha 2$ domains are fused to the $\alpha 3$ domain of murine H-2 $K^b$ (to facilitate recognition by the murine CD8 molecule). As autoantigenic peptides several different insulin and IGRP derivatives (such as, for example, $hIns_{B10\text{-}18}$, $hIGRP_{228\text{-}236}$ and $hIGRP_{265\text{-}273}$) are used that have been shown to be recognized by islet-associated $CD8^+$ T cells in the context of HLA-A*0201. Biotinylated peptide/HLA-A*0201 monomers will be added at a molar ratio of 4 moles of biotin per mole of avidin. Biotinylated proteins will be added in multiple portions (about 0.4 moles biotin per avidin) over a period of 24 hours at 4°C with slow stirring (10 rpm). The resultant probes are purified on a magnetic separation column (Miltenyi Biotec). A monomer consisted of an unrelated HLA-A*0201-binding peptide complexed with HLA-A*0201 molecules are used for the synthesis of negative control probe. Nanosphere size, relaxivity (change in relaxation rate per mM), number of biotin binding sites, and iron and protein content are measured.

**Administration of nanospheres.** Cohorts of 10-15 female NOD.$\beta 2m^{null}$.HHD mice will be treated with nanospheres coated with each of the different peptide/HLA complexes referred to above or a negative control peptide (influenza)/HLA complex (0.01, 0.05, 0.1, 0.5 and 1 $\mu$g peptide equivalents, one dose every 3 wk from 4 to 30 wk of age, or two doses/week starting at 10 weeks of age for 5 consecutive weeks). Peripheral expansion of antigen-specific $CD8^+$ T cells will be documented by staining blood mononuclear cells with anti-CD8 mAb and peptide/MHC tetramers (before initiation of treatment and at treatment withdrawal). Mice will be killed at the onset of hyperglycemia or at the end of the study. Individual mice will be studied by multicolor flow cytometry for presence of central and/or effector memory ($CD69^-$, $CD44^{hi}$, $CD62L^{hi}$ or $CD62L^{lo}$, $CD122^+$, $Ly6C^+$) $tetramer^+$ $CD8^+$ T cells in different lymphoid organs (spleen, lymph nodes), bone marrow (a known reservoir of memory T cells), liver, lung and islets. Tetramer-binding avidity will be measured as described (Han *et al.*, 2005; Amrani *et al.*, 2000). The inventors contemplate that treatment induces systemic expansion of low-avidity central and effector memory $tetramer^+$ $CD8^+$ cells and preferential (but not exclusive) accumulation of these T cells in marrow, pancreatic lymph nodes (PLNs) and islets.

**Administration of multiple doses of peptide/MHC complex.** In another study, cohorts of mice will be treated with one, two, three or four injections of an effective dose, which the inventors contemplate to be similar for all those complexes exhibiting therapeutic efficacy in other studies (at 4, 7, 10 and 13 wk). It is expected that protection will require one or more than one dose (to expand the memory low-avidity T cell pool above the protective threshold) and that the expanded $tetramer^+$ $CD8^+$ memory T cell population progressively disappears from the circulation to accumulate in marrow, PLNs, and islets.

**Administration of peptide/MHC complexes at the onset of hyperglycemia.** Mice will be treated at the onset of hyperglycemia (>10.5 mM/1) with a more aggressive nanosphere treatment protocol (1-5 $\mu$g peptide equivalents twice a wk for 5 wk). Negative and positive controls will receive nanospheres coated with an irrelevant peptide/HLA complex or anti-CD3 mAb (a daily i.v. injection of 20 $\mu$g for 5 days (Haller, 2005)), respectively. Mice will be bled immediately before the initiation of treatment to assess baseline percentages of tetramer-positive $CD8^+$ T cells in the circulation. Reversal of T1D will be considered when blood glucose values stabilize at <10 mM/l for at least 4 weeks at which time treatment with be withdrawn. Mice are bled again to confirm presence of significantly expanded pools of circulating tetramer-positive $CD8^+$ T cells. The animals are followed for at least an additional 8-12 weeks to ensure stable remission. Mice are sacrificed at the end of the observation period to establish the long-term persistence of the expanded pools of memory tetramer-positive $CD8^+$ T cells in different lymphoid and non-lymphoid organs. Pancreas tissue will also be harvested for histological analysis. It is expected that long-term remission will be associated with the presence of numerous small islets devoid of mononuclear cell infiltration. That is, unlike the situation in pre-diabetic mice where the treatment is expected to foster occupation of inflamed islets by protective memory T cells, treatments in diabetic mice is predicted to promote accumulation of the protective memory T cells in the pancreatic lymph nodes (in addition to other reservoirs of memory T cells), but not in islets (presumably newborn islets lacking inflammatory potential).

**Peptide/HLA-coated nanospheres work by inducing the deletion of naive high-avidity clonotypes.** The inventors contemplate that low avidity autoreactive $CD8^+$ T cells tend to accumulate as memory cells during T1D progression (in small numbers) and that peptide/HLA-coated nanospheres work by inducing the deletion of naive high-avidity clonotypes (owing to TCR triggering without costimulation) and the expansion of small pools of pre-existing memory low-avidity clonotypes (costimulation-independent). In part, this stems from the observation that treatment of mice with an irrelevant peptide (from a tumor antigenic, (TUM/H-2 $K^d$) complex did not induce the peripheral expansion of TUM/$K^d$ tetramer-reactive $CD8^+$ T cells above background (see above). These memory low-avidity autoreactive $CD8^+$ cells then inhibit the activation of their naive high-avidity (presumably less-fitter) counterparts by competing for stimulatory resources (i.e., antigen/MHC on DCs, cytokines, etc.). In fact, there is evidence in other systems that memory cells can compete effectively with naive T cells for homeostatic cues (i.e., IL-15) (Tan *et al.*, 2002). By making stable contacts with autoantigen-loaded DCs in the PLNs, these prevalent memory low-avidity clonotypes would also inhibit the activation of other autoreactive T cell specificities.

**Manifestation of the T cell expansion (and anti-diabetogenic activity) of peptide/HLA-coated nanospheres requires expression of the endogenous target autoantigen in beta cells.** The expression of endogenous target autoantigens are believed to be the source of the stimulus that induces the formation of the memory low avidity autoreactive $CD8^+$ T cell pools that are subsequently expanded by the nanosphere treatment. An IGRP deficiency will be introduced into NOD.β2m$^{null}$.HHD mice. These mice will be treated with IGRP$_{228-236}$ (cross-reactive with mIGRP$_{228-236}$) and IGRP$_{265-273}$ (identical to mIGRP$_{265-273}$)/HLA-A*0201-coated nanospheres (Takaki et *al.*, 2006). NOD.β2m$^{null}$.IGRP$^{null}$.HHD mice will be treated with optimal doses of nanospheres coated with the two IGRP/HLA complexes. The inventors contemplate that the treatment will not induce the expansion/recruitment of the corresponding hIGRP peptide/HLA-reactive $CD8^+$ cells.

If IGRP expression is dispensable for diabetes development (it is known that lack of IGRP expression is not lethal, as rats do not express it) and the mice spontaneously develop diabetes, it is also predicted that the nanosphere treatment will not protect the mice from T1D (there will be no memory IGRP-reactive $CD8^+$ T cells). In contrast, it is believed that treatment with nanospheres coated with complexes of HLA-A*0201 and insulin epitopes will induce expansion of the corresponding memory T cell pools and will be protective, as the mice will continue to express insulin.

It is possible that the nanosphere types to be tested here will not induce significant T cell expansions in all the mice. This will likely depend on whether the corresponding T cell population has previously undergone priming *in vivo* prior to the initiation of treatment. It may be useful/necessary to study additional cohorts of mice treated with combinations of several different nanosphere types. Obviously, it is conceivable that, contrary to our prediction, nanosphere treatment might be able to induce the *de novo* formation of memory low-avidity T cell pools. In this case, however, the inventors contemplate that these cells will not be protective because they will not be able to engage endogenous IGRP/HLA-A*0201 complexes on DCs in treated NOD.β2m$^{null}$.IGRP$^{null}$.HHD mice.

**hIGRP expressing mice.** The inventors have generated several lines of mice expressing a rat insulin promoter-driven human IGRP transgene and have compared the levels of expression of the human transgene in each of these lines to that of the endogenous mIGRP-encoding locus by real-time RT-PCR. Although the levels of expression of the transgene were highly variable from line to line, the levels of expression were consistent among different individuals within individual lines. In one of these lines (#1114) the levels of expression of hIGRP were equivalent to those of mIGRP.

The inventors will introduce this RIP-hIGRP transgene into NOD.β2m$^{null}$.IGRP$^{null}$.HHD mice and hβ2m/HLA-A*1101, HLA-B*0702, or HLA-Cw*0304-transgenic NOD.β2m$^{null}$.IGRP$^{null}$ mice, to identify additional epitopes in hIGRP that are targets of $CD8^+$ T cell responses in the context of these four different HLA alleles. The islet-associated $CD8^+$ T cells of these mice will be screened for reactivity against libraries of HLA-A*0201, HLA-A* 1101, HLA-B*0702 and HLA-Cw*0304-binding hIGRP peptides.

The corresponding peptide/HLA complex-coated nanospheres will then be tested for anti-diabetogenic efficacy in the corresponding hβ2 m/HLA-A*1101, HLA-B*0702, or HLA-Cw*0304-transgenic NOD.β2m$^{null}$.IGRP$^{null}$ mice. The overall objective of this exercise is to expand on the repertoire of peptide/HLA combinations that could be used to treat as many patients as possible.

Optimization of NP therapeutic properties and pMHC conjugation. Ongoing studies using pMHC class I complexes were done to optimize the NP size and density, as well as pMHC valency. This was done using gold NP, as they are more amenable to these types of studies than iron oxide. This has also helped us show that the ability of pMHC-NP to expand autoregulatory T-cell memory is independent of the chemical composition of the NP core (iron oxide vs. gold). Extensive work was done with gold NPs of different diameters and the data suggests NPs of <14 nm in diameter are beneficial along the lines that point to smaller as being the optimal NP size. Directional binding of multiple pMHCs to the NP surface (>50-200/NP) via their carboxytermini and adequate stabilization of the coated NPs to allow concentration to high particle densities (>10^10/uL) without compromising monodispersion of the NPs or stability. By testing these class I nanovaccines in pre-diabetic NOD mice, we have established a workable range of NP dose and pMHC valency that enables significant expansion of cognate autoregulatory CD8+ T-cells *in vivo* (~10-20 fold). We have established that total dose of pMHC is key, and that best results are obtained when giving higher numbers of small NPs coated with fewer pMHCs than fewer numbers of larger NPs coated at higher pMHC valencies (for the same amount of total pMHC). These conditions also reduce tissue accumulation and enable the delivery of therapeutic levels of pMHC at the lowest possible doses of metal.

In one preferred embodiment, dosing regimens based on the following assumptions are used:

Assumptions: 4 nm iron-oxide nanoparticle coated with pMHC antigen in a density sufficient that the total amount of protein per dose is about 1 μg to 25 μg.

For example, Total pMHC per dose (1μg-25 μg) = (1.34 x $10^{13}$ pMHC/dose to 3.33 x $10^{14}$ pMHC/dose)
Total iron per dose (4nm) NPs

$$(1\ \mu g\text{-}15\ \mu g) = (3.73x10^{12}\ \text{Nps/dose to } 5.60x10^{13}\ \text{Nps/dose})$$

It is understood that the amount ofpMHC per dose can range from as low as 0.1 μg to 500 μg.

As an example, in a 60 kg human patient, the amount of pMHC per dose can range from 0.24 to 6.08 mg with the understanding that this corresponds to the 1 μg to 25 μg discussed above. Also as above, this dose can be changed to correspond to 0.1 μg to 500 μg.

The 4 nm iron oxide particles described above provide for enhance efficiacy relative to gold nanopartilces having a diameter of 14 nm suggesting that the smaller particles have improved utility. It is understood the term 'iron oxide" is meant to be encompassing of all iron oxides available or known to the skilled artisan and include by way of example, iron oxyhydroxides.

**Example 3**

**Monospecific Peptide-MHC Class II-Coated Nanospheres**

**Production of T1D-relevant pMHC class II complexes.** In order to test the hypothesis that nanospheres coated with T1D-relevant peptide/I-$A^{g7}$ complexes could also afford T1D protection and cure T1D in NOD mice, several different peptide/I-$A^{g7}$ complexes can be constructed from the following five T1D-relevant and control peptide sequences: BDC2.5 mimotope: AHHPIWARMDA (SEQ ID No. 59); $IGRP_{128-145}$: TAALSYTISRMEESSVTL (SEQ ID No. 60); $IGRP_{4-22}$: LHRSGVLIIHHLQEDYRTY (SEQ ID No. 61); $IGRP_{195-214}$: HTPGVHMASLSVYLKTNVFL (SEQ ID No. 62); and Insulin $B_{9-23}$: SHLVEALYLVAGERG (SEQ ID No. 63). As negative controls, G6P isomerase peptide (LSIALHVGFDH; SEQ ID No. 64)/I-$A^{g7}$ complex (self pMHC control), and two hen egg lysozyme ($HEL_{14-22}$ -RHGLDNYRG; SEQ ID No. 65- and $HEL_{11-25}$ -AMKRHGLDNYRGYSL; SEQ ID No. 66-)/I-$A^{g7}$ complexes (foreign pMHC controls) can be used. Recombinant I-$A^{g7}$ monomers are generated as previously described. Briefly, the final constructs in vector pRMHa3 code for the natural leader sequences, followed by the peptide sequences described above, and the extracytoplasmatic domains of the I-$A^{g7}$ a and the b chains, respectively. Both chains extend into a linker sequence (SSAD), a thrombin cleavage site (LVPRGS) and an acidic or a basic leucine zipper sequence for the a and the b chain, respectively, followed by six consecutive histidine residues. A biotinylation sequence #85 follows the acidic zipper on the a chain. The DNA constructs are co-transfected into *Drosophila melanogaster* SC2 cells along with a puromycine resistance gene to generate stable cell lines. For large-scale preparation (12 liters), recombinant proteins are harvested by tangential flow from $CuSO_4$-induced cell supernatants and purified by metal chelate affinity chromatography, anion exchange chromatography, as well as size exclusion chromatography. Biotinylation of purified molecules is performed using the BirA enzyme. Biotinylation is measured by immunodepletion on streptavidin-agarose beads and subsequent analysis by SDS-PAGE. These complexes can be conjugated to the bioabsorbable biodegradable nanospheres. These monomers can also be used to produce fluorochrome-conjugated tetramers to enumerate cognate autoreactive CD4+ T-cells, both before and after treatment with pMHC-coated nanospheres. Purified biotinylated pMHC complexes can then be coated onto the nanospheres. For the studies proposed herein, we can produce the recombinant pMHC complexes in fly cells, as above, but using a different construct design that increases the total valency of pMHCs that can be coated onto individual nanospheres: dimers of pMHC fused to the IgG2a Fc.

**Optimization of nanosphere therapeutic properties and pMHC conjugation.** Studies using pMHC class I complexes can be done to optimize the nanosphere size and density, as well as pMHC valency. The size, density, charge and monodispersity of the nanospheres can be measured by spectrophotometry, transmission electron microscopy (TEM) and dynamic light scattering. The nanosphere samples can be concentrated, conjugated with 3.4 kD thiol-PEG-$NH_2$-pMHCs, washed and concentrated at high densities (~$10^{14}$/ml) without compromising monodispersion.

It is contemplated that nanospheres of 5-15 nm in diameter are optimal. New synthesis protocols will enable directional binding of multiple pMHCs to the nanosphere surface (up to 200/nanosphere, for example) via their carboxytermini and adequate stabilization of the coated nanosphere to allow concentration to high particle densities (>$10^{10}$/uL) without compromising monodispersion or stability. These class I nanovaccines can be tested in pre-diabetic NOD mice to establish a workable range of nanosphere dose and pMHC valency that enables massive expansion of cognate autoregulatory CD8+ T-cells *in vivo.* It is contemplated that treatment of newly diagnosed diabetic mice with these pMHC-nanospheres will be highly efficient at restoring normoglycemia. It is contemplated that the best results will be obtained when giving higher numbers of small nanospheres coated with fewer pMHCs than fewer numbers of larger nanospheres coated at higher pMHC valencies (for the same amount of total pMHC). It is further contemplated that these conditions and the bioabsorbable and biodegradable properties of the nanospheres will reduce tissue accumulation and enable the delivery of therapeutic levels of pMHC at the lowest possible dose.

**Reversal of hyperglycemia in NOD mice by treatment with T1D-relevant pMHC class II-coated nanospheres.** It is contemplated that pMHC class II-coated biodegradable bioabsorbable nanospheres can reverse hyperglycemia in newly diagnosed diabetic NOD mice, essentially as described for pMHC class I-coated nanospheres. Diabetic NOD mice can be treated twice a wk with 7.5 $\mu$g of NPs. Mice are considered cured when they remain normoglycemic for 4 consecutive weeks. It is contemplated that 2.5mi/I-$A^{g7}$-nanospheres will reverse hyperglycemia in almost all tested mice. Hyperglycemia can be measured, for example, by Intraperitoneal glucose tolerance tests (IPGTTs). It is contemplated that most if not all hyperglycemic mice treated with $IGRP_{128-145}$/I-$A^{g7}$-nanospheres, $B_{9-23}$/I-$A^{g7}$-nanospheres and $IGRP_{4-22}$/I-$A^{g7}$-nanospheres will become normoglycemic. As a control, nanospheres coated with $GPI_{282-292}$/I-$A^{g7}$ (a T1D-irrelevant self antigen) and $HEL_{14-22}$/I-$A^{g7}$ (a foreign antigen can be used. It is contemplated that these experiments will demonstrate that 'monospecific' nanospheres coated with several different T1D-relevant pMHC class II-complexes can be at least as effective at inducing T1D reversal as pMHC class I-coated nanospheres. In some cases, it is believed that boosting might be necessary for consistent and universal long-term maintenance of normoglycemia.

**T1D-relevant pMHC class II-coated nanospheres and the expansion of cognate Tr1-like and memory-phenotype autoregulatory CD4+ T-cells.** It is contemplated that the blood, spleens, pancreatic lymph nodes (PLNs), mesenteric lymph nodes (MLNs) and bone marrow of 50 wk-old diabetic mice that may been rendered normoglycemic by treatment with 2.5mi/I-$A^{g7}$-nanospheres will reveal a significantly increased percentage of 2.5mi/I-$A^{g7}$ tetramer+ CD4+ T-cells in all organs examined except MLNs, as compared to mice studied immediately at diabetes onset or age-matched non-diabetic untreated animals. It is believed that, CD4+ T-cell expansion will be antigen-specific and no expansion in pools of non-cognate autoreactive CD4+ T-cells will be detected. It is contemplated that maintenance of autoregulatory T-cell expansion will be necessary for sustained maintenance of normoglycemia. Phenotypic analyses of these NP-expanded 2.5mi/I-$A^{g7}$ tetramer+ cells vs.2.5mi/I-$A^{g7}$ tetramer-cells may reveal a memory-like phenotype ($CD62^{low}$, $CD44^{high}$ and moderate CD69 upregulation). These cells may be CD25- and FoxP3-. This can be confirmed in NOD mice expressing FoxP3-eGFP, in which Treg cells constitutively express eGFP. Treatment of these mice at 10 wk of age with 2.5mi/I-$A^{g7}$-nanospheres may expand eGFP- 2.5mi tetramer+ CD4+ T-cells. Functional *in vitro* studies of FACS-sorted 2.5mi/I-$A^{g7}$ tetramer+ CD4+ T-cells can determine if they proliferate in response to DCs pulsed with cognate, but not non-cognate, peptide and whether they secrete IL-10 and IFN$\gamma$. These studies can be done using ELISA and luminex technology. It is contemplated that these Tr1-like CD4+ T-cells will express high levels of cell surface TGF$\beta$.

It is contemplated that pMHC class II-nanosphere-expanded tetramer+ cells, but not their tetramer- counterparts, will effectively suppress the proliferation of naive LCMV Gp33-specific CD8+ cells against Gp33 and 2.5mi peptide-pulsed DCs (recognized by the responder and tetramer+ Tr1 CD4+ cells, respectively). It is further believed that addition of an anti-IL10 mAb to the cultures will partially inhibit this suppressive activity, as compared to cultures receiving control rat-IgG. It is believed that experiments in diabetic mice treated with $IGRP_{4-22}$/I-$A^{g7}$-nanospheres and blocking anti-IL-10 or rat-IgG (as a control), as well as experiments in perforin-deficient NOD mice will show that restoration of normoglycemia by pMHC class II-nanospheres is IL-10- and perforin-dependent. It is further contemplated that IFN$\gamma$ is necessary for development of the Tr1 cells that pMHC-nanospheres may expand, but not for their suppressive activity. The cognate autoreactive T-cells that may expand in response to pMHC-nanosphere therapy in IFNy-/- mice may have a Th2 phenotype (producing IL-4 and IL-10 in response to peptide challenge *ex vivo*), whereas those expanding in IL-10-/- mice may produce IFN$\gamma$ and IL-4 but no IL-10. These results would indicate that diabetes reversal is mediated by Tr1-like cells.

**Example 4**

**T-Cell Expansion Using IGRP/Kd-PLGA Nanospheres**

IGRP206-214/Kd-PLGA nanospheres induce the activation of cognate CD8+ T-cells *in vitro.* This assay was performed essentially as described in Tsai et al., Immunity (2010) 32, 568-680. Briefly, purified naive IGRP206-214/Kd-specific T cell receptor (TCR)-transgenic CD8+ T-cells from 8.3-NOD mice were cultured with IGRP206-214/Kd-PLGA nanospheres for 48h. The supernatants were assayed for IFN-gamma. The cells were pulsed with 1 μCi of [3H]-thymidine and harvested. The CPM and IFN-gamma was measured in relation to the number of nanospheres per well. This is depicted in Figure 9. Figure 8 depicts a magnified image of the IGRP/Kd conjugated PLGA particles. The PLGA particles were made according to the following:Concentration: 100 nm (PLGA: 20 mg/mL in 1.5 mL).
DLS size: 154.72 nm
pMHC Conjugation: conjugated 5 mg IGRP/Kd with 2 mL PLGA particles Final volume: 2 mL

**REFERENCES**

U.S. Pat. No. 4,367,110
U.S. Pat. No. 4,452,901
U.S. Pat. No. 4,554,101
U.S. Pat. No. 4,589,330
U.S. Pat. No. 4,818,542
U.S. Pat. No. 6,103,379
U.S. Pat. No. 6,387,498
U.S. Pat. No. 6,651,655
U.S. Pat. No. 6,712,997
U.S. Publication 20050202032
U.S. Publication 20090155292
Aichele et al., Proc. Natl. Acad. Sci. USA, 91:444-448, 1994.
Amrani et al., J. Immunology, 167:655-666, 2001.
Amrani et al., Nature, 406:739-742, 2000.
Anderson et al., Proc. Natl. Acad. Sci. USA, 96:9311-9316, 1999.
Anderton and Wraith, Eur. J. Immunol., 28:1251-1261, 1998.
Arden et al., Diabetes, 48:531-539, 1999.
Atkinson and Maclaren, N. Engl. J. Med., 331:1428-1436, 1994.
Atkinson and Maclaren, Sci. Am., 7:62-67, 1990.
Banga, In: Therapeutic Peptides and Proteins. Formulation, Processing, and Delivery Systems, Technomic Pub. Co., Inc., Lancaster, Pa., 1995.
Barany and Merrifield, In: The Peptides, Gross and Meienhofer (Eds.), Academic Press, NY, 1-284, 1979.
Bielekova et al., Nat. Med., 6:1167-1175, 2000.
Bottazzo et al., N. Engl. J. Med., 313:353-360, 1985.
Brinker and Scherer, In: Sol-gel Science, Academic Press, 1990.
Burke et al., J. Inf. Dis., 170:1110-1119, 1994.
Cao et al., Hum. Immunol., 62:1009, 2001.
Caruso et al., Macromolecules, 32(7):2317-2328, 1999.
Caruso, J. Amer. Chem. Soc., 121(25):6039-6046, 1999.
Castano and Eisenbarth, Annu. Rev. Immunol., 8:647-679, 1990.
Chentoufi and Polychronakos, Diabetes, 51:1383, 2002.
Chou and Fasman, Adv. Enzymol., 47:45-148, 1978a.
Chou and Fasman, Annu. Rev. Biochem., 47:251-276, 1978b.
Chou and Fasman, Biochemistry, 13(2):211-222, 1974a.
Chou and Fasman, Biochemistry, 13(2):222-245, 1974b.
Chou and Fasman, Biophys. J, 26(3):385-399, 1979.
Davies, Advanced Materials, 10:1264-1270, 1998.
DiLorenzo, et al., Proc. Natl. Acad. Sci. USA, 95:12538-12543, 1998.
Doytchinova et al., J. Immunol., 172:4314, 2004.
Epitope Mapping Protocols (1996)
Fennessy et al., Diabetologia, 37:937-945, 1994.
Golman and Shinohara, Trends Chem. Engin., 6:1-6, 2000.
Goulder et al. J. Exp. Med., 192:1819-1832, 2000.
Group, D.P.T.-T.D.S, N. Engl. J. Med., 346:1685-1691, 2002.
Haller et al., N. Engl. J. Med., 353:2086-2087, 2005.
Hamilton-Williams et al., Proc. Natl. Acad. Sci. USA, 98:11533, 2001.

Han et al., J. Clin. Invest., 115:1879 2005.
Han et al., Nat. Med., 11:645-652, 2005.
Hanninen et al., J. Clin. Invest., 90:1901-1910, 1992.
Hanprasopwattana, Langmuir, 12:3173-3179, 1996
Honeyman et al., Mol. Med., 1(5):576-582, 1995.
Iler In: Chemistry of Silica, John Wiley & Sons, 1979.
Imagawa et al., Diabetes, 50:1269-1273, 2001.
Itoh et al., J. Clin. Invest., 92:2313-2322, 1993.
Kappos et al., Nat. Med., 6:1176-1182, 2000.
Karin et al., J. Exp. Med., 180:2227-2237, 1994.
Kent et al., Nature, 435:224-228, 2005.
Keymeulen et al. N. Engl. J. Med., 352(25):2598-608, 2005.
Kreuter, In: Colloidal Drug Delivery Systems, Marcel Dekker, Inc., NY., 219-342, 1994.
Liblau et al., Immunity, 17:1-6, 2002.
Lieberman and DiLorenzo, Tissue Antigens, 62:359-377, 2003.
Lieberman et al., J. Immunol., 173:6727, 2004.
Lieberman et al., Proc. Natl. Acad. Sci. USA, 100:8384-8388, 2003.
Liu and Wraith, Int. Immunol., 7:1255-1263, 1995.
Maree et al., Int. Immunol., 18:1067-1077, 2006.
Martin et al., J. Biol. Chem., 276:25197-25207, 2001.
McKown, et al., Arthritis Rheum., 42:1204-1208, 1999.
Merrifield, Science, 232(4748):341-347, 1986.
Metzler and Wraith, Int. Immunol., 5:1159-1165, 1993.
Miller et al., J. Exp. Med., 149:758-766, 1979.
Moore et al., Diabetes, 53:1459-1466, 2004.
Moriwaki et al., Diabetologia, 42:1332-1340, 1999.
Nakanishi et al., J. Clin. Endocrinol. Metab. 84:3721-3725, 1999.
Nakayama et al., Nature, 435:220-224, 2005.
Nejentsev et al., Diabetes, 46:1888-1895, 1997.
Owerbach, Diabetes, 49:508-512, 2000.
Palmer et al., Science, 222:1337-1339, 1983.
Partch and Brown., J Adhesion, 67:259-276, 1998.
Pascolo et al., J. Exp. Med., 185:2043, 1997.
Pekarek et al., Nature, 367:258, 1994.
Pinkse et al., Proc. Natl. Acad. Sci. USA, 102:18425-18430, 2005.
Pociot and McDermott, Genes Immun., 3:235-249, 2002.
Pozzilli, et al., Diabetologia, 43:1000-1004, 2000.
Robles et al., Clin. Immunol., 102:117-224, 2002.
Santamaria et al., Diabetes, 41:53-61, 1992.
Santamaria et al., J. Immunol., 154:2494, 1995.
Santamaria, Curr. Opin Immunol., 13:663-669, 2001.
Serreze et al., Diabetes, 43:505, 1994.
Serreze et al., Diabetes, 50:1992-2000, 2001.
Sibley et al., Lab. Invest., 53:132-144, 1985.
Sidney et al., Hum. Immunol., 45:79, 1996.
Somoza et al., J. Immunol., 153:1360-1377, 1994.
Stewart and Young, In: Solid Phase Peptide Synthesis, 2d. ed., Pierce Chemical Co., 1984.
Sukhorukov et al., Polymers Adv. Tech., 9(10-11):759-767, 1998.
Tait et al., Hum. Immunol., 42:116-124, 1995.
Takaki et al., J. Immunol., 176:3257-3265, 2006.
Tam et al., J. Am. Chem. Soc., 105:6442, 1983.
Tan et al., J. Exp. Med., 12:1523-1532, 2002.
Tice & Tabibi, In: Treatise on Controlled Drug Delivery, Kydonieus (Ed.), Marcel Dekker, Inc. NY, 315-339, 1992.
Tigges et al., J. Immunol., 156(10):3901-3910, 1996.
Toes et al., Proc. Natl. Acad. Sci. USA, 93:7855-7860, 1996.
Toma et al., Proc. Natl. Acad. Sci. USA, 102:10581-10585, 2005.
Trentham, et al., Science, 261:1727-1730, 1993.
Trudeau et al., J. Clin. Invest., 111:217-223, 2003.

Verdaguer et al., J Immunology, 157:4726-4735, 1996.
Verdaguer et al., J. Exp. Med., 186:1663-1676, 1997.
Weiner, Science, 259:1321-1324, 1993.
Wong et al., Nat. Med., 5:1026-1031, 1999.
Wraith et al., Cell, 59:247-255, 1989.
Yeaman et al., Ann. NY Acad. Sci., 955:174-182, 2002.

## Claims

1. A composition of high particle density nanoparticles comprising antigen-MHC complexes coupled at a ratio of greater than 50 to 200 to a biocompatible bioabsorbable nanosphere having a diameter of 4 nm or from 5 to 15 nm for use in a method for preventing or treating an autoimmune disorder when administered to a subject to expand anti-pathogenic autoreactive T cells.

2. A composition of high particle density nanoparticles comprising antigen-MHC complexes coupled at a ratio of greater than 50 to 200 to a biocompatible bioabsorbable nanosphere having a diameter of 4 nm or from 5 to 15 nm for use in a method for expanding and/or developing populations of anti-pathogenic autoreactive T-cells in a subject when administered to expand said populations.

3. The composition for use of Claim 1 or 2, wherein a plurality of auto-antigen epitopes are contained in said antigen-MHC complexes.

4. The composition for use of Claim 3, wherein said plurality of auto-antigen epitopes are derived from a single auto-antigen or a plurality of auto-antigens.

5. The composition for use of any of Claims 1-4, wherein the expanded population of anti-pathogenic autoreactive T-cells are antigen specific but suppress in a non-antigen-specific manner.

6. The composition for use of any of Claims 1-5, wherein the administration of said biocompatible bioabsorbable nanosphere depletes the population of cognate or non-cognate pathogenic autoreactive T cells.

7. An auto-antigen epitope-MHC-biocompatible bioabsorbable nanosphere complex comprising a biocompatible core and a biodegradable, bioabsorbable coating on the outer surface of said core and high density antigen-MHC complexes coupled at a ratio of greater than 50 to 200 to a biocompatible bioabsorbable nanosphere having a diameter of 4 nm or from 5 to 15 nm, wherein the high density antigen-MHC complexes are coupled to the biocompatible core or the biodegradable, bioabsorbable coating.

8. The auto-antigen epitope-MHC biocompatible bioabsorbable nanosphere complex of Claim 7, wherein said biocompatible core comprises a metal, iron, iron oxide, a polymer, or gold.

9. The auto-antigen epitope-MHC biocompatible bioabsorbable nanosphere complex of Claim 7 or 8, wherein said biodegradable, bioabsorbable coating is selected from dextran, mannitol, and poly(ethylene glycol).

10. The composition for use of any of Claims 1-6, or the complex of any of Claims 7-9, wherein said coupling further comprises a linker and wherein the linker is optionally ethylene glycol.

11. The composition for use of Claim 1, wherein the MHC molecule comprises MHC class II molecules.

12. The composition for use of Claim 1 wherein said autoreactive T cells are CD4+ TR1 cells and optionally wherein the CD4+ TR1 cells are **characterized by** IL-10 and IFN$\gamma$ expression.

13. The composition for use of Claim 1, wherein the antigen-MHC complexes are covalently coupled to the biocompatible bioabsorbable nanosphere, wherein the MHC molecule comprises MHC Class II molecules, and wherein the anti-pathogenic autoreactive T cells are TR1 cells which express IL-10.

**14.** The composition for use of any of Claims 1-6 and 11-13, or the complex of any of Claims 7-9, wherein the MHC is MHC I or MHC II or a combination thereof and optionally wherein said coupling further comprises a linker.

**15.** The composition for use of any of Claims 1-6 or 11-13, or the complex of any one of Claims 7-9, wherein said autoimmune disorder or disease is pre-diabetes, diabetes, type I diabetes, type II diabetes, allergic asthma, multiple sclerosis, primary biliary cirrhosis, neuromyelitis optica, pemphigus vulgaris, irritable bowel disease, Crohn's disease, colitis, rheumatoid arthritis, psoriatic arthritis, systemic lupus erythematosus, Celiac disease, psoriasis, cardiomyopathy, myasthenia gravis, uveitis, ankylosing spondylitis, Grave's disease, inflammatory myopathy, or anti-phospholipid antibody syndrome and optionally wherein said coupling further comprises a linker.

**16.** The complex of any of Claims 7-9, or the composition for use of any of Claims 11-15, wherein a plurality of auto-antigen epitopes are contained in said antigen-MHC complexes that are derived from a single auto-antigen or a plurality of auto-antigens and optionally wherein said coupling further comprises a linker.

**17.** The composition for use of Claims 1, 2 or 11 wherein:

a) the autoimmune disease is type I diabetes, the antigen is derived from an antigen selected from the group consisting of PPI, IGRP, GAD, or pro-insulin, and the MHC component comprises all or part of HLA-DR; or
b) the autoimmune disease is multiple sclerosis, the antigen is derived from an antigen selected from the group consisting of MOG, MBP, or PLP, and the MHC component comprises all or part of HLA-DR.

**18.** A dosage formulation comprising the composition for use of any one of Claims 1, 2, 11 or 17, wherein the amount of antigen-MHC complexes per dose is from 0.024 - 121.6 mg.

## Patentansprüche

**1.** Zusammensetzung von Nanopartikeln mit hoher Teilchendichte, die Antigen-MHC-Komplexe umfasst, die in einem Verhältnis von über 50 bis 200 an ein bioverträgliches bioabsorbierbares Nanokügelchen mit einem Durchmesser von 4 nm oder von 5 bis 15 nm gekoppelt sind, zur Verwendung in einem Verfahren zur Vorbeugung oder Behandlung einer Autoimmunerkrankung, wenn sie einem Subjekt verabreicht wird, um antipathogene autoreaktive T-Zellen zu expandieren.

**2.** Zusammensetzung von Nanopartikeln mit hoher Teilchendichte, die Antigen-MHC-Komplexe umfasst, die in einem Verhältnis von über 50 bis 200 an ein bioverträgliches bioabsorbierbares Nanokügelchen mit einem Durchmesser von 4 nm oder von 5 bis 15 nm gekoppelt sind, zur Verwendung in einem Verfahren zum Expandieren und/oder Entwickeln von Populationen von antipathogenen autoreaktiven T-Zellen in einem Subjekt, wenn sie verabreicht wird, um die Populationen zu expandieren.

**3.** Zusammensetzung zur Verwendung nach Anspruch 1 oder 2, wobei eine Vielzahl von Autoantigenepitopen in den Antigen-MHC-Komplexen enthalten sind.

**4.** Zusammensetzung zur Verwendung nach Anspruch 3, wobei die Vielzahl von Autoantigenepitopen von einem einzigen Autoantigen oder einer Vielzahl von Autoantigenen abgeleitet ist.

**5.** Zusammensetzung zur Verwendung nach einem der Ansprüche 1-4, wobei die expandierte Population von antipathogenen autoreaktiven T-Zellen antigenspezifisch ist, aber in nichtantigenspezifischer Weise unterdrückt.

**6.** Zusammensetzung zur Verwendung nach einem der Ansprüche 1-5, wobei die Verabreichung des bioverträglichen bioabsorbierbaren Nanokügelchens die Population von verwandten oder nicht-verwandten pathogenen autoreaktiven T-Zellen dezimiert.

**7.** Autoantigenepitop-MHC-bioverträgliches bioabsorbierbares Nanokügelchen-Komplex, umfassend einen bioverträglichen Kern und eine biologisch abbaubare, bioabsorbierbare Beschichtung auf der Außenfläche des Kerns und Antigen-MHC-Komplexe hoher Dichte, die in einem Verhältnis von über 50 bis 200 an ein bioverträgliches bioabsorbierbares Nanokügelchen mit einem Durchmesser von 4 nm oder von 5 bis 15 nm gekoppelt sind, wobei die Antigen-MHC-Komplexe hoher Dichte an den bioverträglichen Kern oder die biologisch abbaubare, bioabsorbierbare Beschichtung gekoppelt sind.

**8.** Autoantigenepitop-MHC-bioverträgliches bioabsorbierbares Nanokügelchen-Komplex nach Anspruch 7, wobei der bioverträgliche Kern ein Metall, Eisen, Eisenoxid, ein Polymer oder Gold umfasst.

**9.** Autoantigenepitop-MHC-bioverträgliches bioabsorbierbares Nanokügelchen-Komplex nach Anspruch 7 oder 8, wobei die biologisch abbaubare, bioabsorbierbare Beschichtung aus Dextran, Mannit und Poly(ethylenglykol) ausgewählt ist.

**10.** Zusammensetzung zur Verwendung nach einem der Ansprüche 1-6 oder Komplex nach einem der Ansprüche 7-9, wobei das Koppeln ferner einen Linker umfasst und wobei der Linker wahlweise Ethylenglykol ist.

**11.** Zusammensetzung zur Verwendung nach Anspruch 1, wobei das MHC-Molekül Moleküle der MHC-Klasse II umfasst.

**12.** Zusammensetzung zur Verwendung nach Anspruch 1, wobei die autoreaktiven T-Zellen CD4+-TR1-Zellen sind und wahlweise, wobei die CD4+-TR1-Zellen durch IL-10- und IFNγ-Expression gekennzeichnet sind.

**13.** Zusammensetzung zur Verwendung nach Anspruch 1, wobei die Antigen-MHC-Komplexe kovalent an das bioverträgliche bioabsorbierbare Nanokügelchen gekoppelt sind, wobei das MHC-Molekül Moleküle der MHC-Klasse II umfasst und wobei die antipathogenen autoreaktiven T-Zellen TR1-Zellen sind, die IL-10 exprimieren.

**14.** Zusammensetzung zur Verwendung nach einem der Ansprüche 1-6 und 11-13 oder Komplex nach einem der Ansprüche 7-9, wobei der MHC MHC I oder MHC II oder eine Kombination davon ist und wahlweise, wobei das Koppeln ferner einen Linker umfasst.

**15.** Zusammensetzung nach einem der Ansprüche 1-6 oder 11-13 oder Komplex nach einem der Ansprüche 7-9, wobei die Autoimmunstörung oder -krankheit Prädiabetes, Diabetes, Diabetes Typ I, Diabetes Typ II, allergisches Asthma, multiple Sklerose, primäre biliäre Zirrhose, Neuromyelitis optica, Pemphigus vulgaris, Reizdarmkrankheit, Morbus Crohn, Kolitis, rheumatoide Arthritis, Psoriasisarthritis, systemischer Lupus erythematodes, Zöliakie, Psoriasis, Kardiomyopathie, Myasthenia gravis, Uveitis, ankylosierende Spondylitis, Morbus Basedow, entzündliche Myopathie oder Antiphospholipid-Antikörpersyndrom ist und wahlweise, wobei das Koppeln ferner einen Linker umfasst.

**16.** Komplex nach einem der Ansprüche 7-9 oder Zusammensetzung zur Verwendung nach einem der Ansprüche 11-15, wobei eine Vielzahl von Autoantigenepitopen in den Antigen-MHC-Komplexen enthalten ist, die von einem einzigen Autoantigen oder einer Vielzahl von Autoantigenen abgeleitet ist und wahlweise, wobei das Koppeln ferner einen Linker umfasst.

**17.** Zusammensetzung zur Verwendung nach Ansprüchen 1, 2 oder 11, wobei:

a) die Autoimmunkrankheit Diabetes Typ I ist, das Antigen von einem Antigen abgeleitet ist, das aus der Gruppe bestehend aus PPI, IGRP, GAD oder Proinsulin ausgewählt ist und die MHC-Komponente HLA-DR ganz oder teilweise umfasst; oder
b) die Autoimmunkrankheit multiple Sklerose ist, das Antigen von einem Antigen abgeleitet ist, das aus der Gruppe bestehend aus MOG, MBP oder PLP ausgewählt ist und die MHC-Komponente HLA-DR ganz oder teilweise umfasst.

**18.** Dosierungsformulierung, umfassend die Zusammensetzung zur Verwendung nach einem der Ansprüche 1, 2, 11 oder 17, wobei die Menge an Antigen-MHC-Komplexen pro Dosis 0,024 bis 121,6 mg beträgt.

## Revendications

**1.** Composition de nanoparticules de densité particulaire élevée comprenant des complexes CMH-antigène couplés à un rapport supérieur à 50 à 200 à une nanosphère bioabsorbable biocompatible ayant un diamètre de 4 nm ou de 5 à 15 nm destinée à être utilisée dans un procédé d'empêchement ou de traitement d'un trouble auto-immune lorsqu'elle est administrée à un sujet pour l'expansion de cellules T autoréactives anti-pathogènes.

2. Composition de nanoparticules de densité particulaire élevée comprenant des complexes CMH-antigène couplés à un rapport supérieur à 50 à 200 à une nanosphère bioabsorbable biocompatible ayant un diamètre de 4 nm ou de 5 à 15 nm destinée à être utilisée dans un procédé d'expansion et/ou de développement de populations de cellules T autoréactives anti-pathogènes chez un sujet lorsqu'elle est administrée pour l'expansion desdites populations.

3. Composition destinée à être utilisée selon la revendication 1 ou 2, dans laquelle une pluralité d'épitopes auto-antigènes est contenue dans lesdits complexes CMH-antigène.

4. Composition destinée à être utilisée selon la revendication 3, dans laquelle ladite pluralité d'épitopes auto-antigènes est dérivée d'un auto-antigène unique ou d'une pluralité d'auto-antigènes.

5. Composition destinée à être utilisée selon l'une quelconque des revendications 1 à 4, dans laquelle la population étendue de cellules T autoréactives anti-pathogènes est spécifique d'un antigène mais supprime de manière non spécifique d'un antigène.

6. Composition destinée à être utilisée selon l'une quelconque des revendications 1 à 5, dans laquelle l'administration de ladite nanosphère bioabsorbable biocompatible appauvrit la population de cellules T autoréactives pathogènes apparentées ou non apparentées.

7. Complexe de nanosphères bioabsorbables biocompatibles CMH- épitope auto-antigène comprenant un noyau biocompatible et un revêtement bioabsorbable biodégradable sur la surface extérieure dudit noyau et des complexes CMH-antigène de densité élevée couplés à un rapport supérieur à 50 à 200 à une nanosphère bioabsorbable biocompatible ayant un diamètre de 4 nm ou de 5 à 15 nm, dans lequel les complexes CMH-antigène de densité élevée sont couplés au noyau biocompatible ou au revêtement bioabsorbable biodégradable.

8. Complexe de nanosphères bioabsorbables biocompatibles CMH- épitope auto-antigène selon la revendication 7, dans lequel ledit noyau biocompatible comprend un métal, du fer, de l'oxyde de fer, un polymère, ou de l'or.

9. Complexe de nanosphères bioabsorbables biocompatibles CMH- épitope auto-antigène selon la revendication 7 ou 8, dans lequel ledit revêtement bioabsorbable biodégradable est choisi parmi le dextrane, le mannitol, et le poly(éthylène glycol).

10. Composition destinée à être utilisée selon l'une quelconque des revendications 1 à 6, ou complexe selon l'une quelconque des revendications 7 à 9, dans lequel ledit couplage comprend en outre un lieur et dans lequel le lieur est facultativement de l'éthylène glycol.

11. Composition destinée à être utilisée selon la revendication 1, dans laquelle la molécule CMH comprend des molécules CMH de classe II.

12. Composition destinée à être utilisée selon la revendication 1 dans laquelle lesdites cellules T autoréactives sont des cellules CD4 + TR1 et facultativement dans laquelle les cellules CD4 + TR1 sont **caractérisées par** l'expression d'IL-10 et d'IFNγ.

13. Composition destinée à être utilisée selon la revendication 1, dans laquelle les complexes CMH-antigène sont couplés de manière covalente à la nanosphère bioabsorbable biocompatible, dans laquelle la molécule CMH comprend des molécules CMH de classe II, et dans laquelle les cellules T autoréactives anti-pathogènes sont des cellules TR1 qui expriment IL-10.

14. Composition destinée à être utilisée selon l'une quelconque des revendications 1 à 6 et 11 à 13, ou complexe selon l'une quelconque des revendications 7 à 9, dans lequel le CMH est CMH I ou CMH II ou une combinaison de ceux-ci et facultativement dans lequel ledit couplage comprend en outre un lieur.

15. Composition destinée à être utilisée selon l'une quelconque des revendications 1 à 6 ou 11 à 13, ou complexe selon l'une quelconque des revendications 7 à 9, dans lequel ledit trouble ou ladite maladie auto-immune est le prédiabète, le diabète, le diabète de type I, le diabète de type II, l'asthme allergique, la sclérose en plaques, la cirrhose biliaire primaire, la neuromyélite optique, le pemphigus vulgaire, la maladie du côlon irritable, la maladie de Crohn, la colite, la polyarthrite rhumatoïde, la polyarthrite psoriasique, le lupus érythémateux systémique, la maladie coeliaque, le psoriasis, la cardiomyopathie, la myasthénie grave, l'uvéite, la spondylarthrite ankylosante, la maladie de Grave, la myopathie inflammatoire, ou le syndrome d'anticorps anti-phospholipide et facultativement dans lequel ledit couplage comprend en outre un lieur.

**16.** Complexe selon l'une quelconque des revendications 7 à 9, ou composition destinée à être utilisée selon l'une quelconque des revendications 11 à 15, dans laquelle une pluralité d'épitopes auto-antigènes est contenue dans lesdits complexes CMH-antigène qui sont dérivés d'un auto-antigène unique ou d'une pluralité d'auto-antigènes et facultativement dans laquelle ledit couplage comprend en outre un lieur.

**17.** Composition destinée à être utilisée selon les revendications 1, 2 ou 11 dans laquelle :

a) la maladie auto-immune est le diabète de type I, l'antigène est dérivé d'un antigène choisi dans le groupe constitué de PPI, IGRP, GAD ou pro-insuline, et le composant CMH comprend tout ou partie d'HLA-DR ; ou
b) la maladie auto-immune est la sclérose en plaques, l'antigène est dérivé d'un antigène choisi dans le groupe constitué de MOG, MBP, ou PLP, et le composant CMH comprend tout ou partie d'HLA-DR.

**18.** Formulation posologique comprenant la composition destinée à être utilisée selon l'une quelconque des revendications 1, 2, 11 ou 17, dans laquelle la quantité de complexes CMH-antigène par dose est de 0,024 à 121,6 mg.

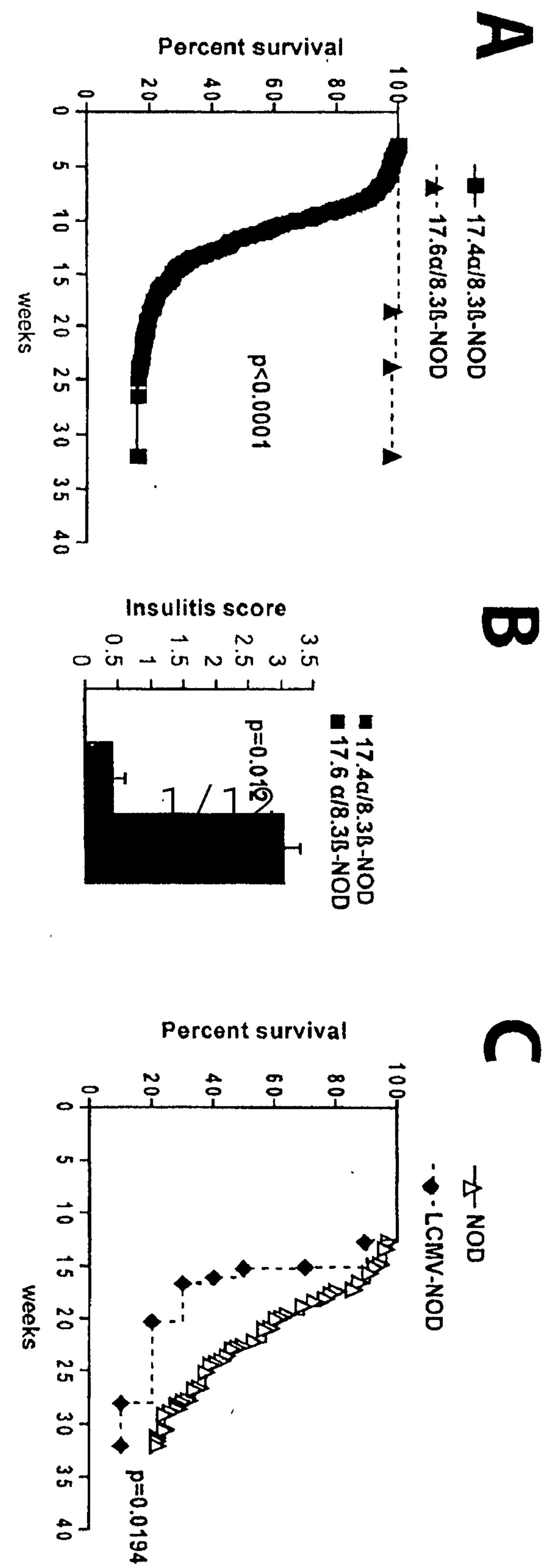
A
Percent survival
weeks
17.4α/8.3β-NOD
17.6α/8.3β-NOD
p<0.0001
B
Insulitis score
17.4α/8.3β-NOD
17.6 α/8.3β-NOD
p=0.012
C
Percent survival
weeks
NOD
LCMV-NOD
p=0.0194

FIGS 1A - 1C

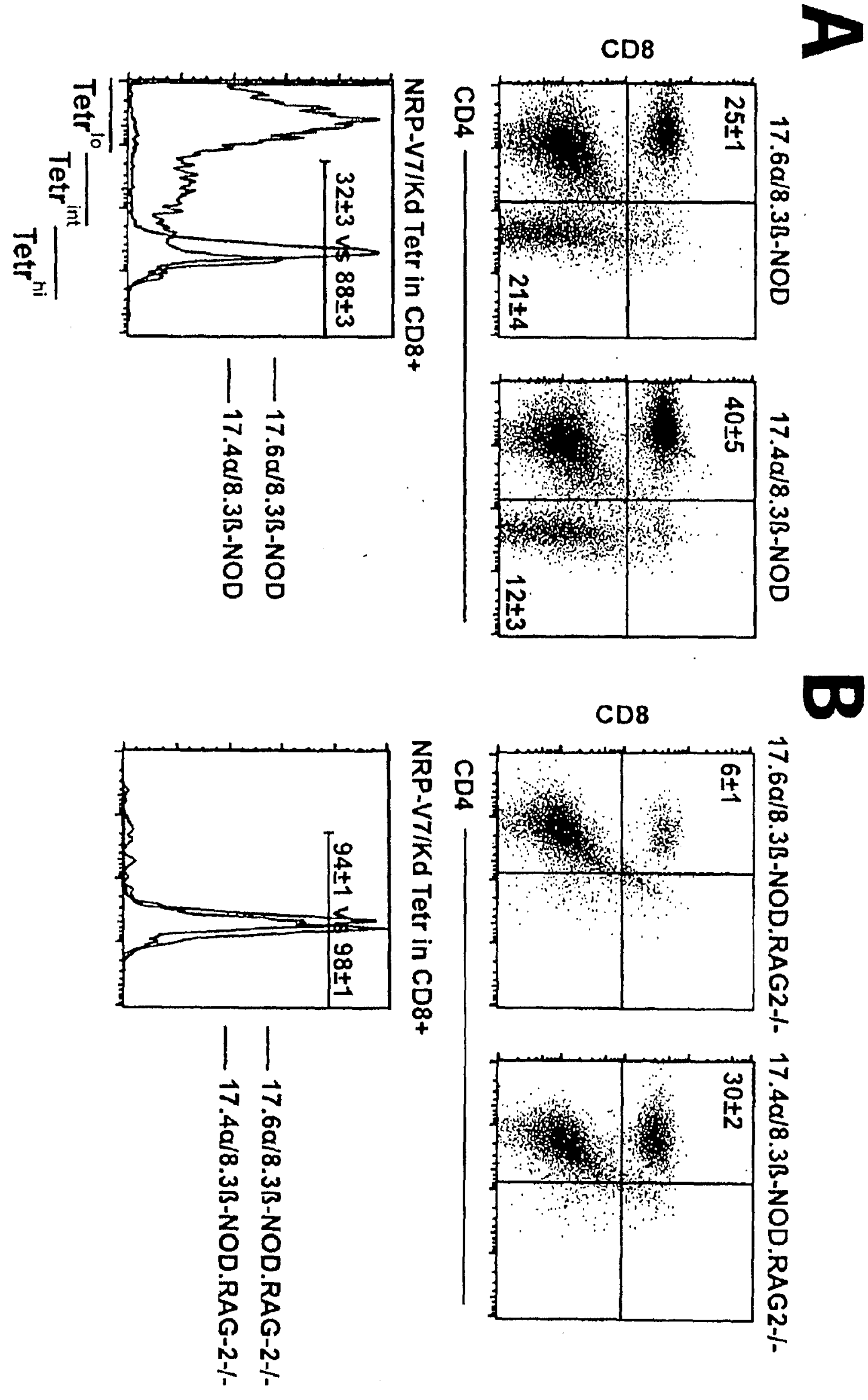
A
17.6α/8.3β-NOD
17.4α/8.3β-NOD
25±1
21±4
40±5
12±3
CD8
CD4
NRP-V7/Kd Tetr in CD8+
32±3 vs 88±3
17.6α/8.3β-NOD
17.4α/8.3β-NOD
Tetr^lo
Tetr^int
Tetr^hi
B
17.6α/8.3β-NOD.RAG2-/-
17.4α/8.3β-NOD.RAG2-/-
6±1
30±2
CD8
CD4
NRP-V7/Kd Tetr in CD8+
94±1 vs 98±1
17.6α/8.3β-NOD.RAG-2-/-
17.4α/8.3β-NOD.RAG-2-/-

FIGS 2A – 2B

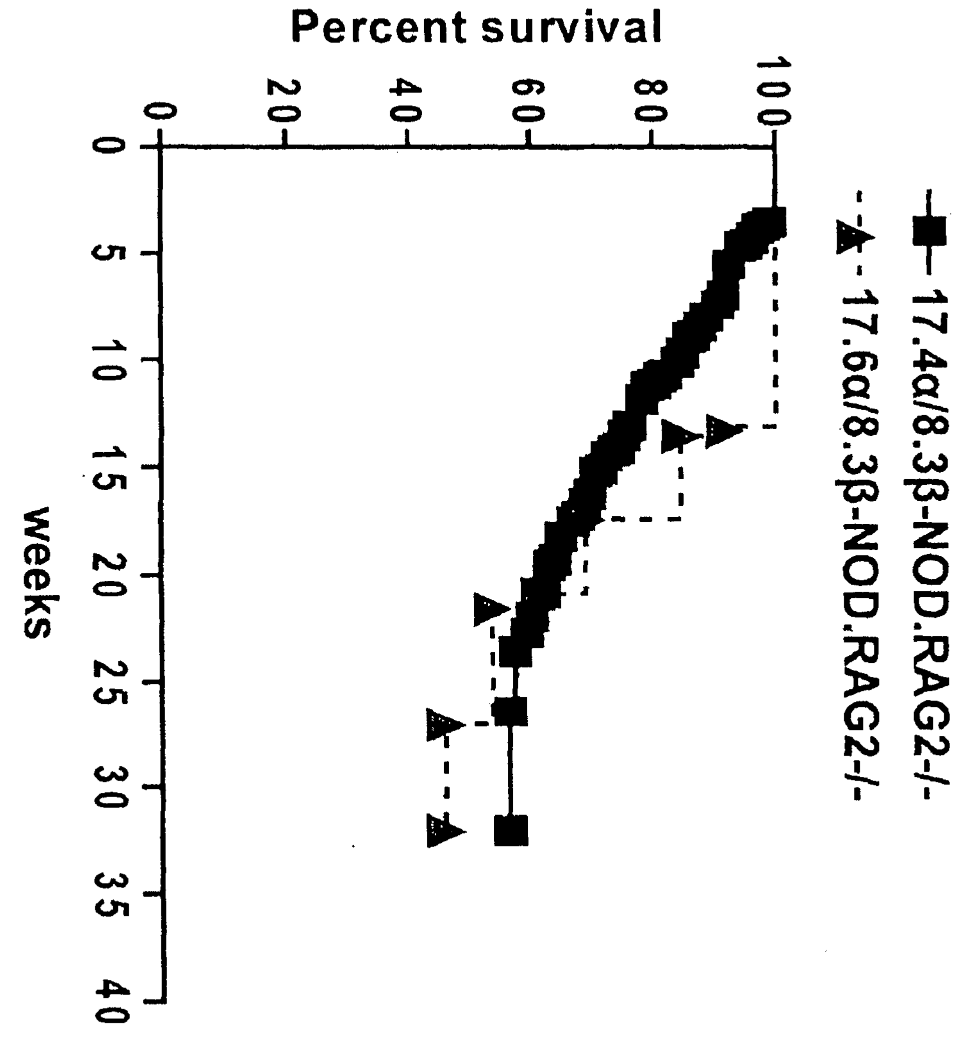
17.4α/8.3β-NOD.RAG2-/-
17.6α/8.3β-NOD.RAG2-/-
Percent survival
100
80
60
40
20
0
0
5
10
15
20
25
30
35
40
weeks

FIG 3

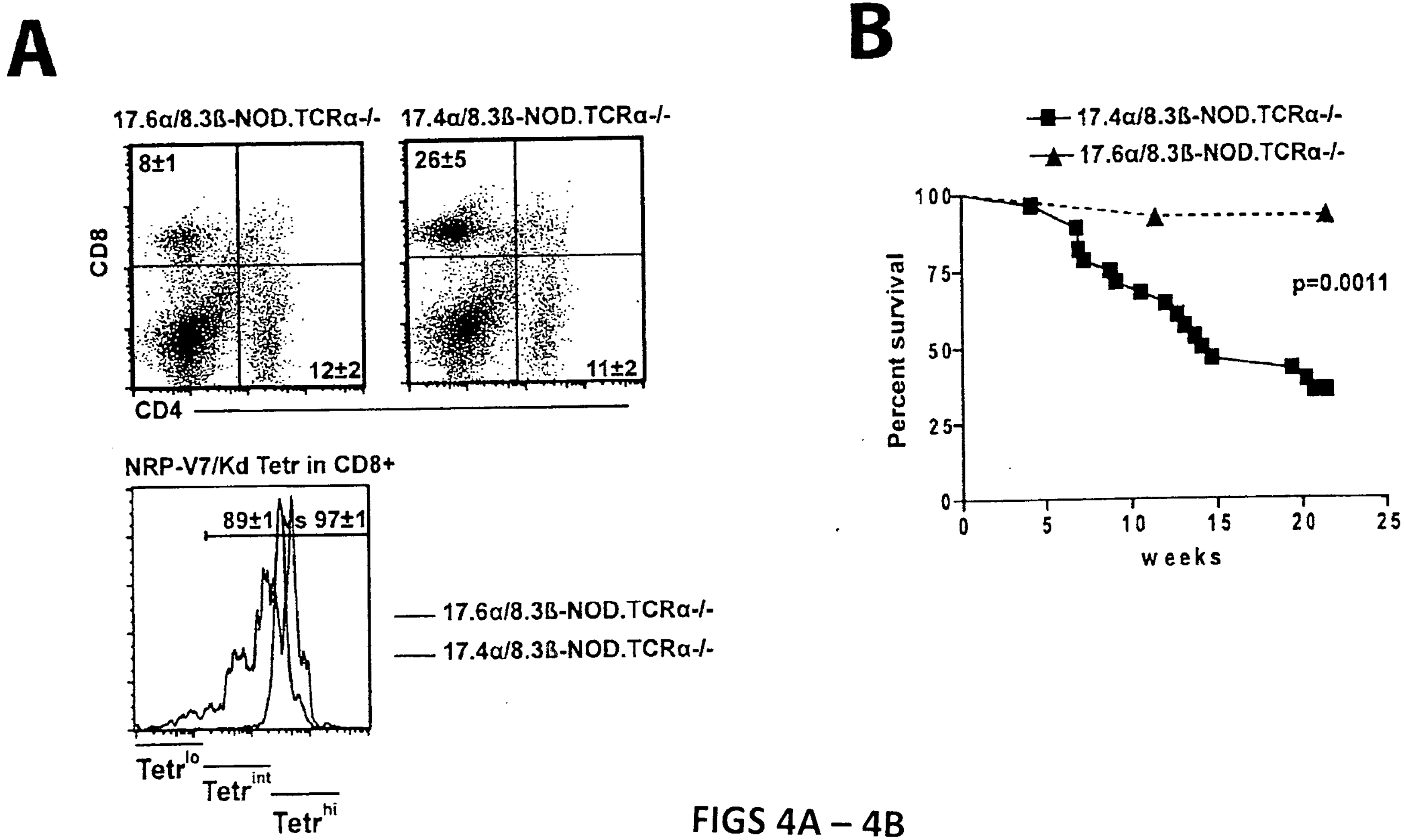
A
17.6α/8.3β-NOD.TCRα-/-
17.4α/8.3β-NOD.TCRα-/-
CD8
8±1
12±2
26±5
11±2
CD4
NRP-V7/Kd Tetr in CD8+
89±1 vs 97±1
17.6α/8.3β-NOD.TCRα-/-
17.4α/8.3β-NOD.TCRα-/-
Tetr lo
Tetr int
Tetr hi
B
17.4α/8.3β-NOD.TCRα-/-
17.6α/8.3β-NOD.TCRα-/-
Percent survival
100
75
50
25
0
p=0.0011
0
5
10
15
20
25
weeks

FIGS 4A – 4B

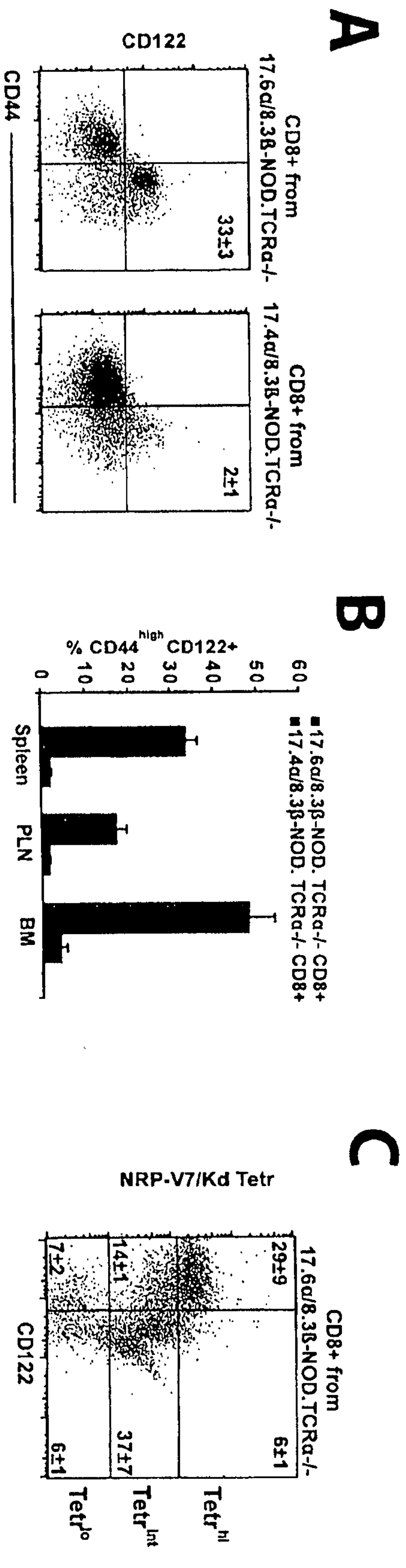
A
CD122
CD8+ from 17.6α/8.3β-NOD.TCRα-/-
33±3
CD8+ from 17.4α/8.3β-NOD.TCRα-/-
2±1
CD44
B
% CD44high CD122+
0
10
20
30
40
50
60
17.6α/8.3β-NOD. TCRα-/- CD8+
17.4α/8.3β-NOD. TCRα-/- CD8+
Spleen
PLN
BM
C
NRP-V7/Kd Tetr
CD8+ from 17.6α/8.3β-NOD.TCRα-/-
29±9
6±1
14±1
37±7
7±2
6±1
CD122
Tetrhi
Tetrint
Tetrlo

FIGS 5A – 5C

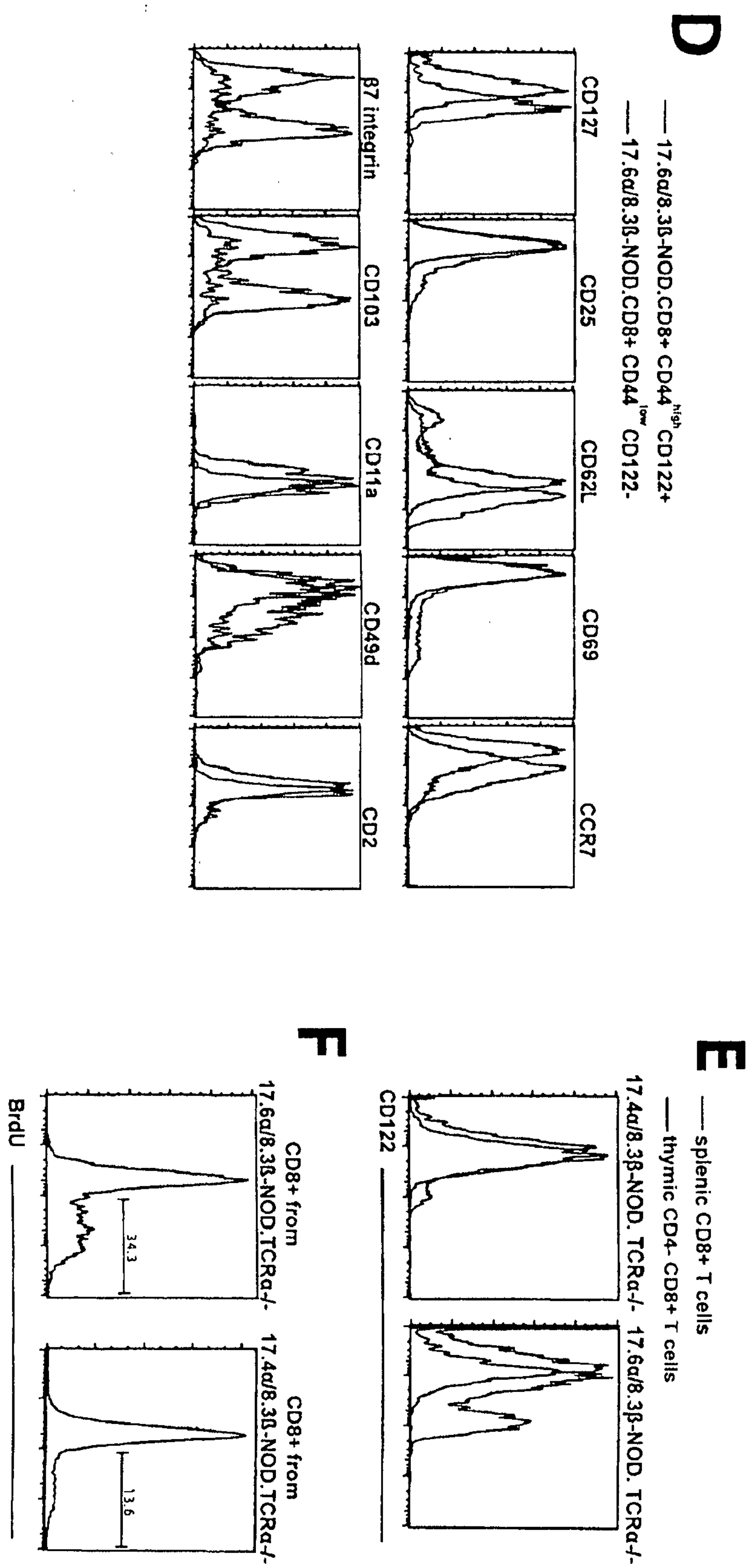
D
17.6α/8.3β-NOD.CD8+ CD44high CD122+
17.6α/8.3β-NOD.CD8+ CD44low CD122-
CD127
CD25
CD62L
CD69
CCR7
β7 integrin
CD103
CD11a
CD49d
CD2
E
splenic CD8+ T cells
thymic CD4- CD8+ T cells
17.4α/8.3β-NOD. TCRα-/-
17.6α/8.3β-NOD. TCRα-/-
CD122
F
CD8+ from
17.6α/8.3β-NOD.TCRα-/-
34.3
CD8+ from
17.4α/8.3β-NOD.TCRα-/-
13.6
BrdU

FIGS 5D – 5F

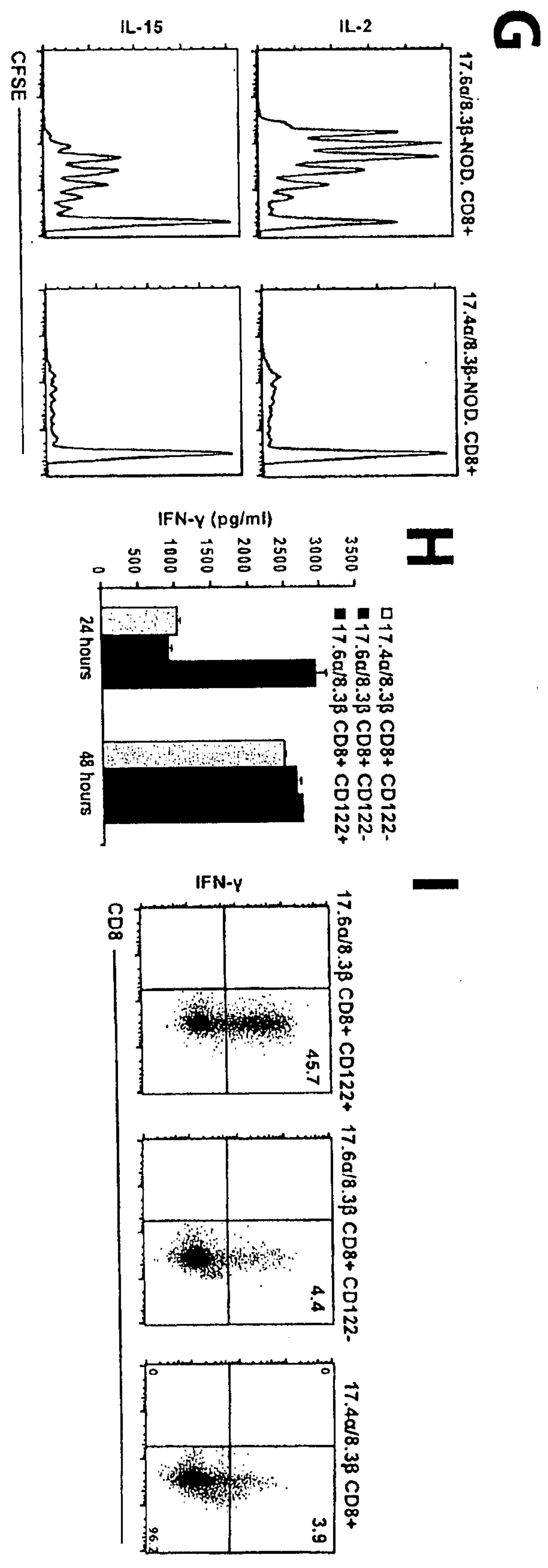
G
17.6α/8.3β-NOD. CD8+
17.4α/8.3β-NOD. CD8+
IL-2
IL-15
CFSE
H
□ 17.4α/8.3β CD8+ CD122-
■ 17.6α/8.3β CD8+ CD122-
■ 17.6α/8.3β CD8+ CD122+
IFN-γ (pg/ml)
0
500
1000
1500
2000
2500
3000
3500
24 hours
48 hours
I
17.6α/8.3β CD8+ CD122+
17.6α/8.3β CD8+ CD122-
17.4α/8.3β CD8+
IFN-γ
CD8
45.7
4.4
3.9
0
0
0
96.2

FIGS 5G – 5I

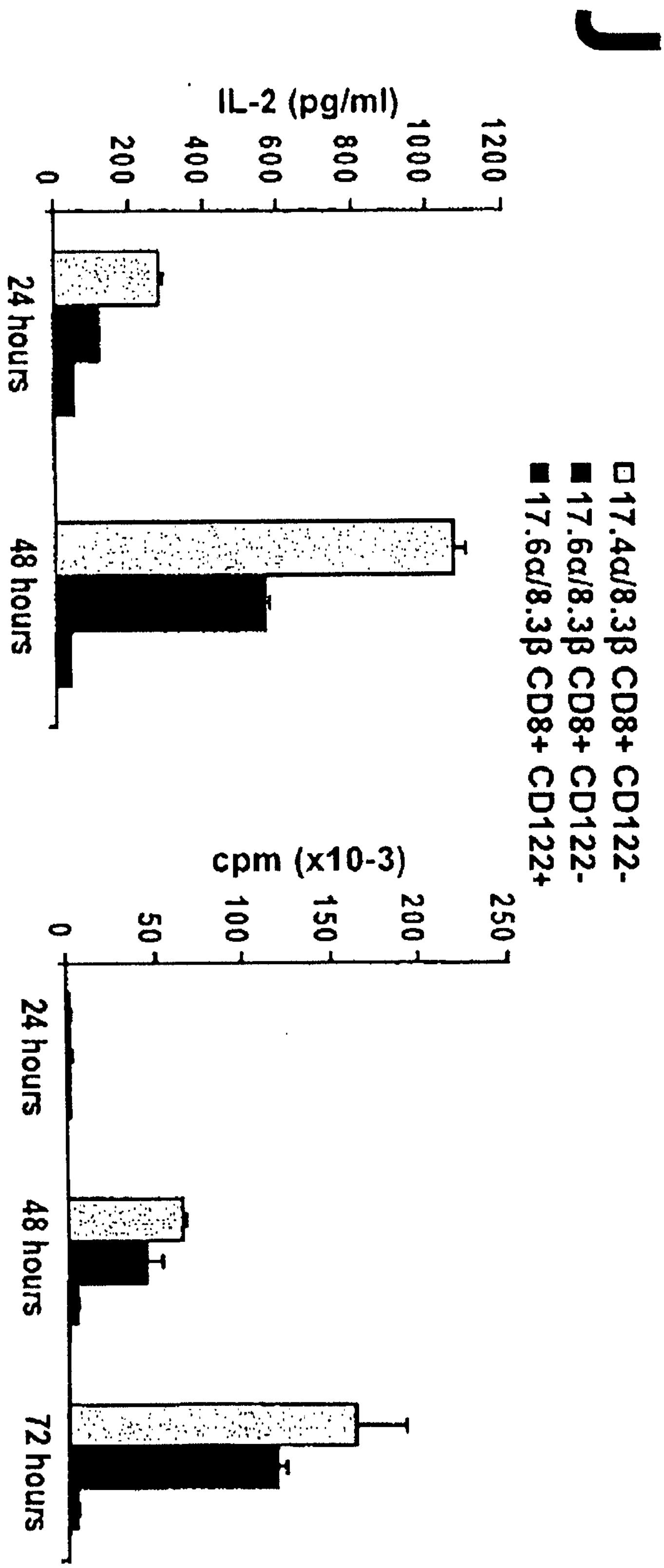
J
IL-2 (pg/ml)
0
200
400
600
800
1000
1200
24 hours
48 hours
17.4α/8.3β CD8+ CD122-
17.6α/8.3β CD8+ CD122-
17.6α/8.3β CD8+ CD122+
cpm (x10-3)
0
50
100
150
200
250
24 hours
48 hours
72 hours

FIG 5J

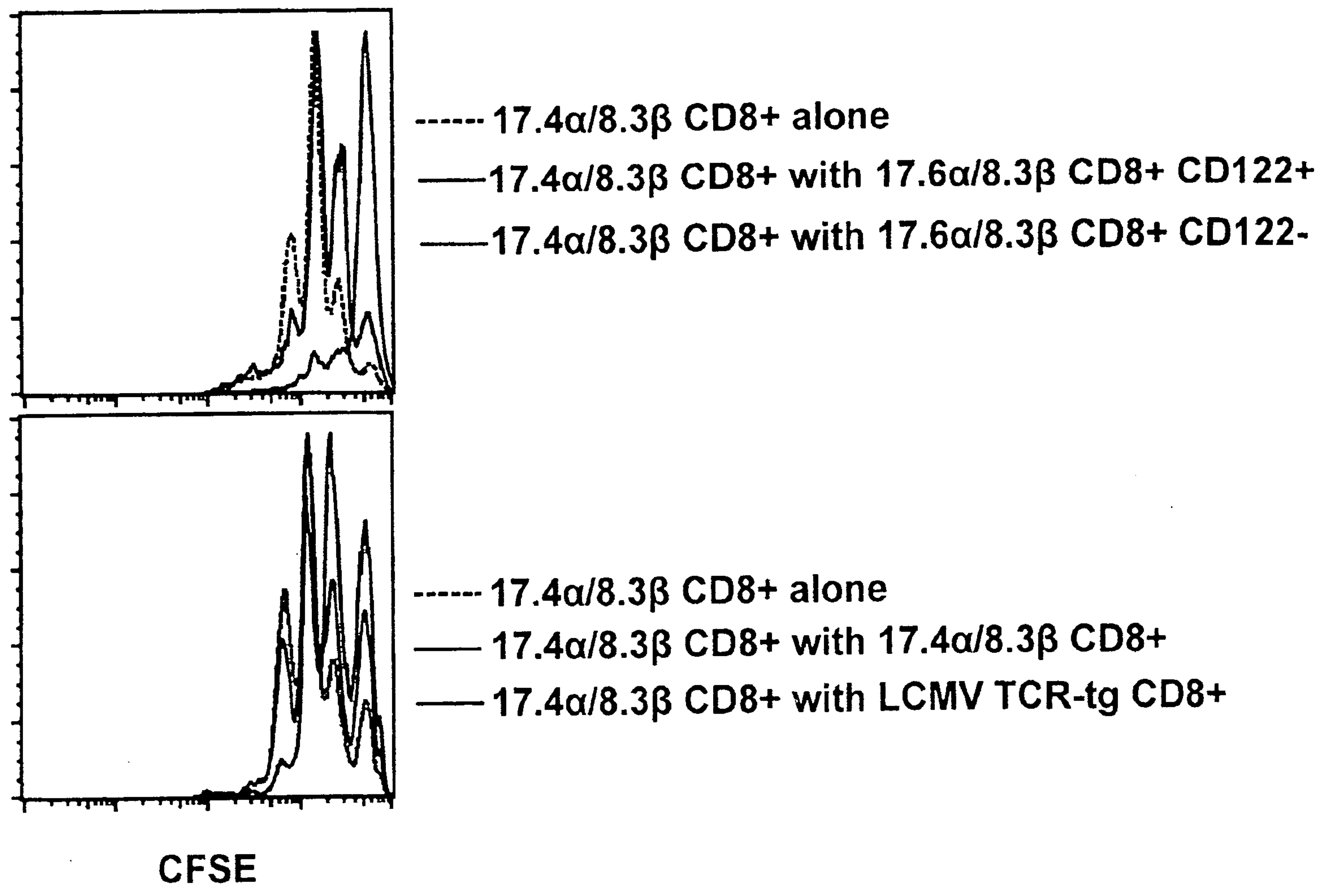
------ 17.4α/8.3β CD8+ alone
—— 17.4α/8.3β CD8+ with 17.6α/8.3β CD8+ CD122+
—— 17.4α/8.3β CD8+ with 17.6α/8.3β CD8+ CD122-
------ 17.4α/8.3β CD8+ alone
—— 17.4α/8.3β CD8+ with 17.4α/8.3β CD8+
—— 17.4α/8.3β CD8+ with LCMV TCR-tg CD8+
CFSE

FIG 6

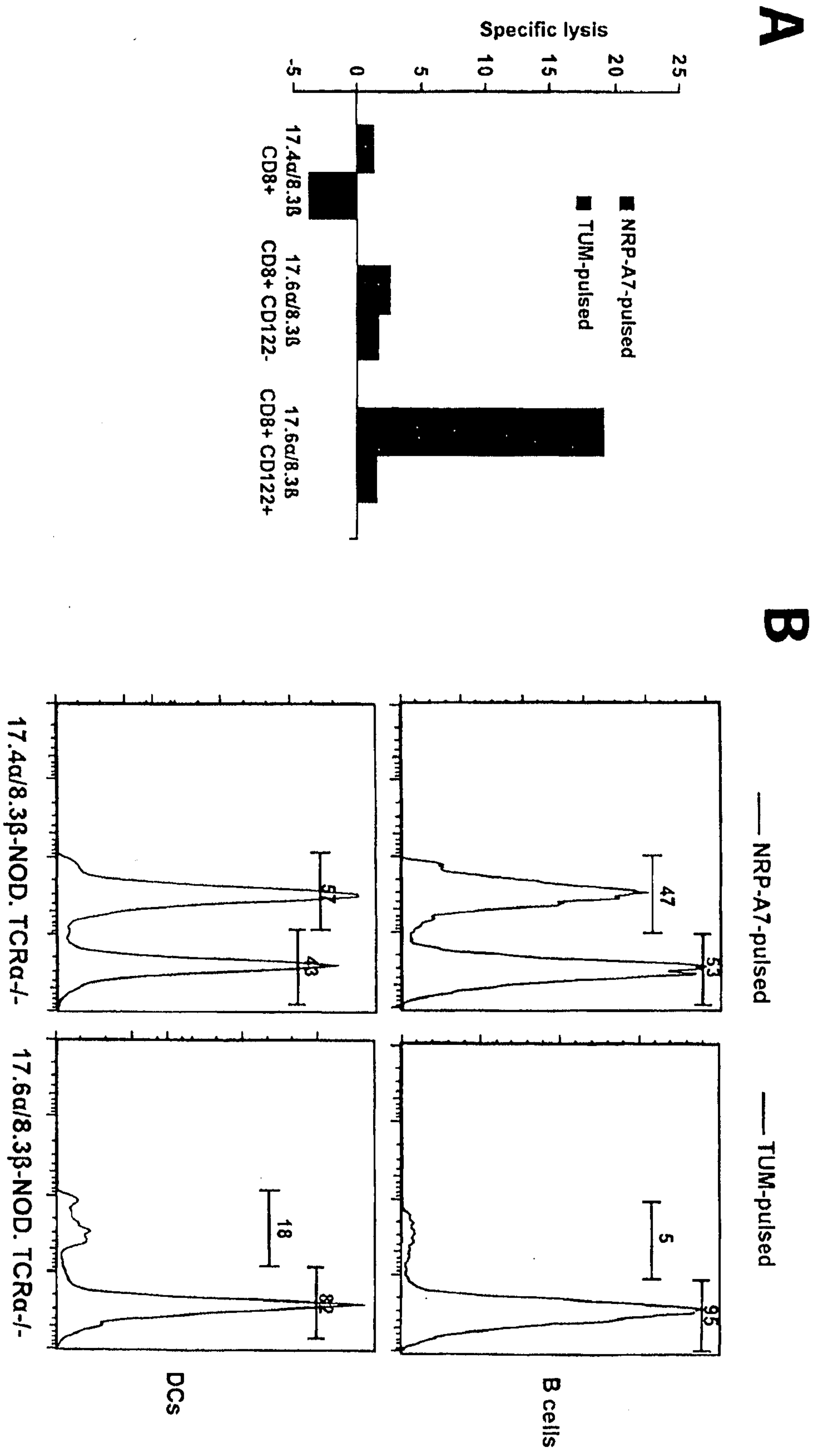
A
Specific lysis
25
20
15
10
5
0
-5
NRP-A7-pulsed
TUM-pulsed
17.4α/8.3β CD8+
17.6α/8.3β CD8+ CD122-
17.6α/8.3β CD8+ CD122+
B
NRP-A7-pulsed
TUM-pulsed
47
53
5
95
57
43
18
82
B cells
DCs
17.4α/8.3β-NOD. TCRα-/-
17.6α/8.3β-NOD. TCRα-/-

FIGS 7A – 7B

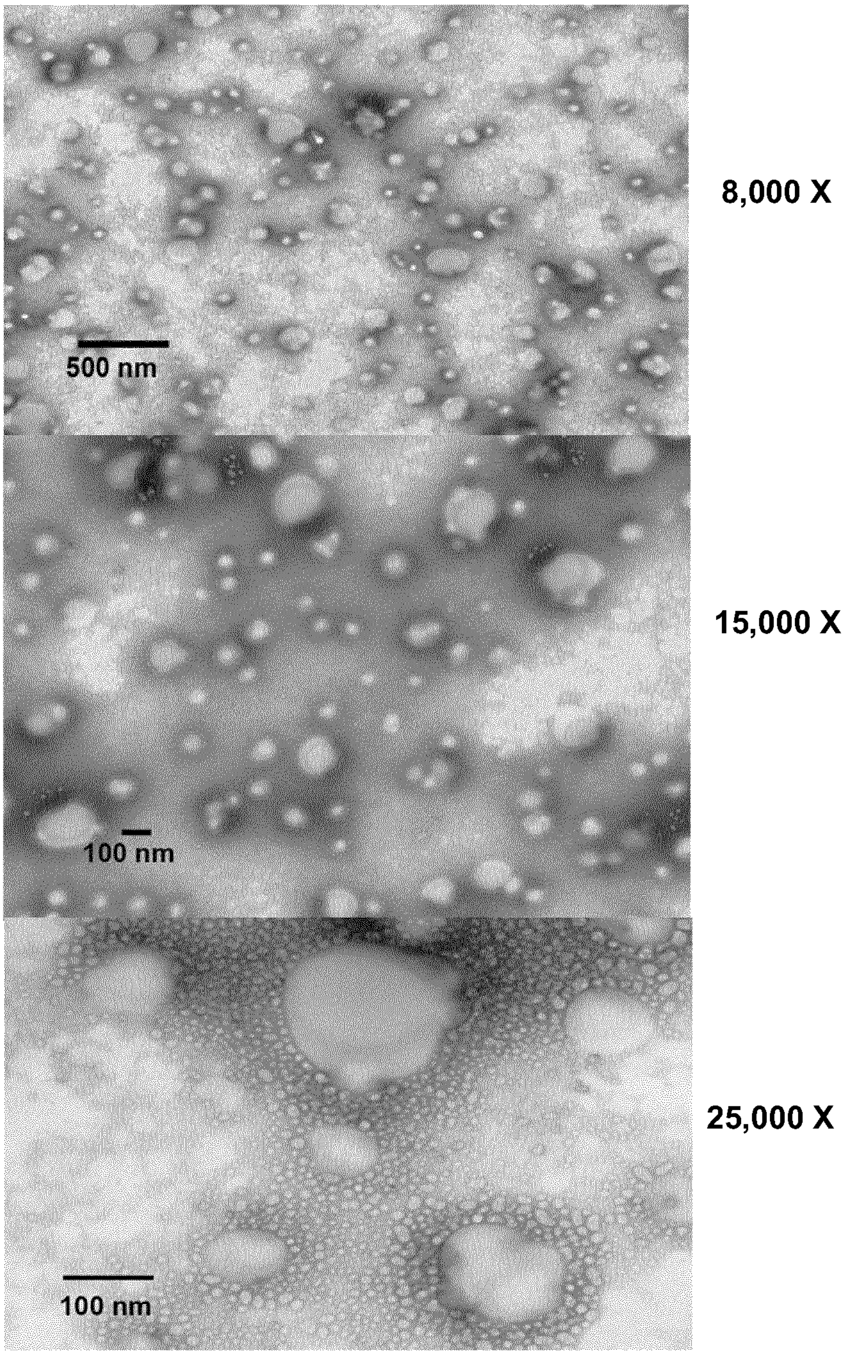


Fig. 8

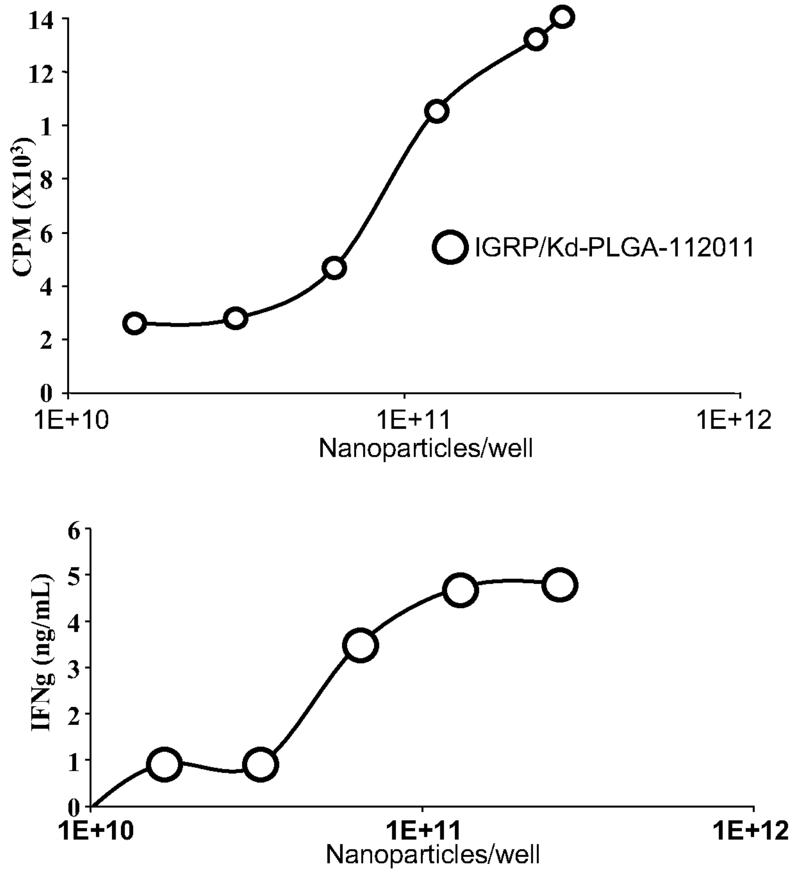
14
12
1
8
6
4
2
0
CPM (X10³)
IGRP/Kd-PLGA-112011
1E+10
1E+11
1E+12
Nanoparticles/well
6
5
4
3
2
1
0
IFNg (ng/mL)
1E+10
1E+11
1E+12
Nanoparticles/well

**Fig. 9**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20050202032 A
- US 20070059775 A
- US 4671958 A
- US 4659839 A
- US 4414148 A
- US 4699784 A
- US 4680338 A
- US 4569789 A
- US 4589071 A
- US 7186814 B
- US 5543391 A
- US 188256 A
- US 6651655 B
- US 4554101 A, Hopp
- US 20090155292 A
- US 6387498 B
- US 6103379 A
- US 4589330 A
- US 4818542 A
- US 4367110 A
- US 4452901 A
- US 6712997 B
- WO 61387873 A


### Non-patent literature cited in the description


- **TSAI et al.** *Immunity,* 2010, vol. 32, 568-680
- **MOORE et al.** *Diabetes,* 2004, vol. 53, 1459-1466
- **GREG T. HERMANSON.** Bioconjugate Techniques. Academic Press, 1996
- **AICHELE et al.** *Proc. Natl. Acad. Sci. USA,* 1994, vol. 91, 444-448
- **AMRANI et al.** *J. Immunology,* 2001, vol. 167, 655-666
- **AMRANI et al.** *Nature,* 2000, vol. 406, 739-742
- **ANDERSON et al.** *Proc. Natl. Acad. Sci. USA,* 1999, vol. 96, 9311-9316
- **ANDERTON ; WRAITH.** *Eur. J. Immunol.,* 1998, vol. 28, 1251-1261
- **ARDEN et al.** *Diabetes,* 1999, vol. 48, 531-539
- **ATKINSON ; MACLAREN.** *N. Engl. J. Med.,* 1994, vol. 331, 1428-1436
- **ATKINSON ; MACLAREN.** *Sci. Am.,* 1990, vol. 7, 62-67
- Therapeutic Peptides and Proteins. **BANGA.** Formulation, Processing, and Delivery Systems. Technomic Pub. Co., Inc, 1995
- **BARANY ; MERRIFIELD.** The Peptides, Gross and Meienhofer. Academic Press, 1979, 1-284
- **BIELEKOVA et al.** *Nat. Med.,* 2000, vol. 6, 1167-1175
- **BOTTAZZO et al.** *N. Engl. J. Med.,* 1985, vol. 313, 353-360
- **BRINKER ; SCHERER.** Sol-gel Science. Academic Press, 1990
- **BURKE et al.** *J. Inf. Dis.,* 1994, vol. 170, 1110-1119
- **CAO et al.** *Hum. Immunol.,* 2001, vol. 62, 1009
- **CARUSO et al.** *Macromolecules,* 1999, vol. 32 (7), 2317-2328
- **CARUSO.** *J. Amer. Chem. Soc.,* 1999, vol. 121 (25), 6039-6046
- **CASTANO ; EISENBARTH.** *Annu. Rev. Immunol.,* 1990, vol. 8, 647-679
- **CHENTOUFI ; POLYCHRONAKOS.** *Diabetes,* 2002, vol. 51, 1383
- **CHOU ; FASMAN.** *Adv. Enzymol.,* 1978, vol. 47, 45-148
- **CHOU ; FASMAN.** *Annu. Rev. Biochem.,* 1978, vol. 47, 251-276
- **CHOU ; FASMAN.** *Biochemistry,* 1974, vol. 13 (2), 211-222
- **CHOU ; FASMAN.** *Biochemistry,* 1974, vol. 13 (2), 222-245
- **CHOU ; FASMAN.** *Biophys. J,* 1979, vol. 26 (3), 385-399
- **DAVIES.** *Advanced Materials,* 1998, vol. 10, 1264-1270
- **DILORENZO et al.** *Proc. Natl. Acad. Sci. USA,* 1998, vol. 95, 12538-12543
- **DOYTCHINOVA et al.** *J. Immunol.,* 2004, vol. 172, 4314
- *Epitope Mapping Protocols,* 1996
- **FENNESSY et al.** *Diabetologia,* 1994, vol. 37, 937-945
- **GOLMAN ; SHINOHARA.** *Trends Chem. Engin.,* 2000, vol. 6, 1-6

- **GOULDER et al.** *J. Exp. Med.,* 2000, vol. 192, 1819-1832
- *N. Engl. J. Med.,* 2002, vol. 346, 1685-1691
- **HALLER et al.** *N. Engl. J. Med.,* 2005, vol. 353, 2086-2087
- **HAMILTON-WILLIAMS et al.** *Proc. Natl. Acad. Sci. USA,* 2001, vol. 98, 11533
- **HAN et al.** *J. Clin. Invest.,* 2005, vol. 115, 1879
- **HAN et al.** *Nat. Med.,* 2005, vol. 11, 645-652
- **HANNINEN et al.** *J. Clin. Invest.,* 1992, vol. 90, 1901-1910
- **HANPRASOPWATTANA.** *Langmuir,* 1996, vol. 12, 3173-3179
- **HONEYMAN et al.** *Mol. Med.,* 1995, vol. 1 (5), 576-582
- **ILER.** Chemistry of Silica. John Wiley & Sons, 1979
- **IMAGAWA et al.** *Diabetes,* 2001, vol. 50, 1269-1273
- **ITOH et al.** *J. Clin. Invest.,* 1993, vol. 92, 2313-2322
- **KAPPOS et al.** *Nat. Med.,* 2000, vol. 6, 1176-1182
- **KARIN et al.** *J. Exp. Med.,* 1994, vol. 180, 2227-2237
- **KENT et al.** *Nature,* 2005, vol. 435, 224-228
- **KEYMEULEN et al.** *N. Engl. J. Med.,* 2005, vol. 352 (25), 2598-608
- **KREUTER.** Colloidal Drug Delivery Systems. Marcel Dekker, Inc, 1994, 219-342
- **LIBLAU et al.** *Immunity,* 2002, vol. 17, 1-6
- **LIEBERMAN ; DILORENZO.** *Tissue Antigens,* 2003, vol. 62, 359-377
- **LIEBERMAN et al.** *J. Immunol.,* 2004, vol. 173, 6727
- **LIEBERMAN et al.** *Proc. Natl. Acad. Sci. USA,* 2003, vol. 100, 8384-8388
- **LIU ; WRAITH.** *Int. Immunol.,* 1995, vol. 7, 1255-1263
- **MAREE et al.** *Int. Immunol.,* 2006, vol. 18, 1067-1077
- **MARTIN et al.** *J. Biol. Chem.,* 2001, vol. 276, 25197-25207
- **MCKOWN et al.** *Arthritis Rheum.,* 1999, vol. 42, 1204-1208
- **MERRIFIELD.** *Science,* 1986, vol. 232 (4748), 341-347
- **METZLER ; WRAITH.** *Int. Immunol.,* 1993, vol. 5, 1159-1165
- **MILLER et al.** *J. Exp. Med.,* 1979, vol. 149, 758-766
- **MORIWAKI et al.** *Diabetologia,* 1999, vol. 42, 1332-1340
- **NAKANISHI et al.** *J. Clin. Endocrinol. Metab.,* 1999, vol. 84, 3721-3725
- **NAKAYAMA et al.** *Nature,* 2005, vol. 435, 220-224
- **NEJENTSEV et al.** *Diabetes,* 1997, vol. 46, 1888-1895
- **OWERBACH.** *Diabetes,* 2000, vol. 49, 508-512
- **PALMER et al.** *Science,* 1983, vol. 222, 1337-1339
- **PARTCH ; BROWN.** *J Adhesion,* 1998, vol. 67, 259-276
- **PASCOLO et al.** *J. Exp. Med.,* 1997, vol. 185, 2043
- **PEKAREK et al.** *Nature,* 1994, vol. 367, 258
- **PINKSE et al.** *Proc. Natl. Acad. Sci. USA,* 2005, vol. 102, 18425-18430
- **POCIOT ; MCDERMOTT.** *Genes Immun.,* 2002, vol. 3, 235-249
- **POZZILLI et al.** *Diabetologia,* 2000, vol. 43, 1000-1004
- **ROBLES et al.** *Clin. Immunol.,* 2002, vol. 102, 117-224
- **SANTAMARIA et al.** *Diabetes,* 1992, vol. 41, 53-61
- **SANTAMARIA et al.** *J. Immunol.,* 1995, vol. 154, 2494
- **SANTAMARIA.** *Curr. Opin Immunol.,* 2001, vol. 13, 663-669
- **SERREZE et al.** *Diabetes,* 1994, vol. 43, 505
- **SERREZE et al.** *Diabetes,* 2001, vol. 50, 1992-2000
- **SIBLEY et al.** *Lab. Invest.,* 1985, vol. 53, 132-144
- **SIDNEY et al.** *Hum. Immunol.,* 1996, vol. 45, 79
- **SOMOZA et al.** *J. Immunol.,* 1994, vol. 153, 1360-1377
- **STEWART ; YOUNG.** Solid Phase Peptide Synthesis. Pierce Chemical Co, 1984
- **SUKHORUKOV et al.** *Polymers Adv. Tech.,* 1998, vol. 9 (10-11), 759-767
- **TAIT et al.** *Hum. Immunol.,* 1995, vol. 42, 116-124
- **TAKAKI et al.** *J. Immunol.,* 2006, vol. 176, 3257-3265
- **TAM et al.** *J. Am. Chem. Soc.,* 1983, vol. 105, 6442
- **TAN et al.** *J. Exp. Med.,* 2002, vol. 12, 1523-1532
- **TICE ; TABIBI.** Treatise on Controlled Drug Delivery, Kydonieus. Marcel Dekker, Inc, 1992, 315-339
- **TIGGES et al.** *J. Immunol.,* 1996, vol. 156 (10), 3901-3910
- **TOES et al.** *Proc. Natl. Acad. Sci. USA,* 1996, vol. 93, 7855-7860
- **TOMA et al.** *Proc. Natl. Acad. Sci. USA,* 2005, vol. 102, 10581-10585
- **TRENTHAM et al.** *Science,* 1993, vol. 261, 1727-1730

- **TRUDEAU et al.** *J. Clin. Invest.,* 2003, vol. 111, 217-223
- **VERDAGUER et al.** *J Immunology,* 1996, vol. 157, 4726-4735
- **VERDAGUER et al.** *J. Exp. Med.,* 1997, vol. 186, 1663-1676
- **WEINER.** *Science,* 1993, vol. 259, 1321-1324
- **WONG et al.** *Nat. Med.,* 1999, vol. 5, 1026-1031
- **WRAITH et al.** *Cell,* 1989, vol. 59, 247-255
- **YEAMAN et al.** *Ann. NY Acad. Sci.,* 2002, vol. 955, 174-182